(19) Europäisches Patentamt
European Patent Office
Office européen des brevets

(11) EP 2 313 520 B1

(12) **EUROPEAN PATENT SPECIFICATION**

(45) Date of publication and mention of the grant of the patent:
**27.08.2014 Bulletin 2014/35**

(21) Application number: **09773073.3**

(22) Date of filing: **03.07.2009**

(51) Int Cl.:
***C12Q 1/68*** *(2006.01)*

(86) International application number:
**PCT/IS2009/000005**

(87) International publication number:
**WO 2010/001419 (07.01.2010 Gazette 2010/01)**

# (54) COPY NUMBER VARIATIONS PREDICTIVE OF RISK OF SCHIZOPHRENIA

KOPIENZAHLVARIATIONEN ALS VORHERSAGE DES SCHIZOPHRENIERISIKOS

VARIATIONS DU NOMBRE DE COPIES PRÉDICTIVES D UN RISQUE DE SCHIZOPHRÉNIE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **04.07.2008 IS 8743**

(43) Date of publication of application:
**27.04.2011 Bulletin 2011/17**

(73) Proprietor: **Decode Genetics EHF**
**101 Reykjavik (IS)**

(72) Inventors:
- **STEFANSSON, Hreinn**
  **IS-210 Gardabaer (IS)**
- **INGASON, Andres**
  **B-1150 Woluwe-Saint-Pierre (BE)**

(74) Representative: **Arnason Faktor**
**Intellectual Property Consulting**
**Gudridarstig 2-4**
**113 Reykjavik (IS)**

(56) References cited:
- **ILLUMINA: "Sentrix HumanHap300 Genotyping BeadChip"[Online] 2006, XP002548936 Retrieved from the Internet: URL:http://www.lerner.ccf.org/ services/gc/ documents/ HUMANHAP300Datasheet.pdf> [retrieved on 2009-10-06]**
- **CHAI J-H ET AL: "Identification of four highly conserved genes between breakpoint hotspots BP1 and BP2 of the Prader-Willi/Angelman syndromes deletion region that have undergone evolutionary transposition mediated by flanking duplicons." AMERICAN JOURNAL OF HUMAN GENETICS OCT 2003, vol. 73, no. 4, October 2003 (2003-10), pages 898-925, XP002548937 ISSN: 0002-9297**
- **STEFANSSON HREINN ET AL: "Large recurrent microdeletions associated with schizophrenia." NATURE 11 SEP 2008, vol. 455, no. 7210, 11 September 2008 (2008-09-11), pages 232-236, XP002548939 ISSN: 1476-4687**
- **KIROV GEORGE ET AL: "Support for the involvement of large copy number variants in the pathogenesis of schizophrenia." HUMAN MOLECULAR GENETICS 15 APR 2009, vol. 18, no. 8, 15 April 2009 (2009-04-15), pages 1497-1503, XP002548938 ISSN: 1460-2083**
- **KARAYIORGOU M ET AL: "Schizophrenia susceptibility associated with interstitial deletions of chromosome 22q11." PROCEEDINGS OF THE NATIONAL ACADEMY OF SCIENCES OF THE UNITED STATES OF AMERICA 15 AUG 1995, vol. 92, no. 17, 15 August 1995 (1995-08-15), pages 7612-7616, XP002548940 ISSN: 0027-8424**
- **FALLIN M DANIELE ET AL: "Genomewide linkage scan for schizophrenia susceptibility loci among Ashkenazi Jewish families shows evidence of linkage on chromosome 10q22." AMERICAN JOURNAL OF HUMAN GENETICS SEP 2003, vol. 73, no. 3, September 2003 (2003-09), pages 601-611, XP002548941 ISSN: 0002-9297**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 313 520 B1

- GEJMAN P V ET AL: "Linkage analysis of schizophrenia to chromosome 15." AMERICAN JOURNAL OF MEDICAL GENETICS 8 DEC 2001, vol. 105, no. 8, 8 December 2001 (2001-12-08), pages 789-793, XP002548942 ISSN: 0148-7299
- CARTER NIGEL P: "Methods and strategies for analyzing copy number variation using DNA microarrays." NATURE GENETICS JUL 2007, vol. 39, no. 7 Suppl, July 2007 (2007-07), pages S16-S21, XP002548943 ISSN: 1061-4036
- WALSH TOM ET AL: "Rare structural variants disrupt multiple genes in neurodevelopmental pathways in schizophrenia." SCIENCE (NEW YORK, N.Y.) 25 APR 2008, vol. 320, no. 5875, 25 April 2008 (2008-04-25), pages 539-543, XP002548952 ISSN: 1095-9203

SEQUENCE LISTING

<110> deCODE genetics ehf

<120> COPY NUMBER VARIATIONS PREDICTIVE OF RISK OF SCHIZOPHRENIA

<130> 2558-PC00

<160> 23

<210> 1
<211> 599
<212> DNA
<213> Homo sapiens

<400> 1

```
tgtagcccag cagagaaggc tggccttcaa gatggagaca gagttcttag gatcaatggt      60
gtctttgtgg acaaagaaga acatatgcag gtgaatgaga catttggggc ttttcttcca     120
ggatctcttc agcccccaca tcttctctgc tgattataat tttggggtt ggtagagttt      180
ggttctttcc tggctgccac tggcaaggca ggacaccttt attgctgtac cttgtgttgg     240
ccaatcaagt cccctatgag gaaccaagaa ctgtacacat tactcttgga tttgaatagy     300
gttaggtcta acatgaattg catgttcact atataccaaa aatggtgcta ggtatttttt     360
tttttttttt tttttgaga tggagtttcg ctctttcacc caggctagag tgcagtggtg      420
cgatctcggt tcactgcaag ctctacctcc cgggttcatg ccattctcct gcctcagcct     480
cccaagtagc tgggactata ggcacccgcc accacaccca gctttttttt tttttttgta     540
tttttagtag agatggggtt tcaccgtgtt agccaggatg gtctcgatct cctgacctc      599
```

<210> 2
<211> 599
<212> DNA
<213> Homo sapiens

<400> 2

```
aatattggct gatttaggag gtgaagggag gcaattccct ggagggaggt gtcaggattt     60
gggacaagag cagcatctag ttaccatcca cagagacccc aaggacagga atccactggt    120
agccggttgg aggggatccc atgaaaacaa gatgaaacgc gtgcattagt actggaacca    180
agatcaggag atgaaaaact acactgtcct aggggatgaa ggaattaggg aatcctggaa    240
gtaaaatttt tcatataggt catttcttcc aaagagacat agggcaatgg cccaatgacr    300
tgaagaaaag aaaactcagg gtctaggatt gaggggaggc agccttttta gtggagacct    360
gtgacctgga ggcccagggt catcctgaca ggggagcggt cttgctggtc gctgggtccg    420
ggactccaat tgcacacagc cagtggcatg gagggtctgt gaccacgatt gggcaatttc    480
ccccattctg cttatggagc aatagagagg aacctcactg gaattataca gaaaggttcc    540
agtgagactt gaactctgat cactgtattc agagtccaaa gtggtcacca ttacaccat     599
```

<210> 3
<211> 599
<212> DNA
<213> Homo sapiens

<400> 3

```
cattgtcttt ttggtaagta aacggtgacg tgagaaattt tgatggggtt agctcagtca     60
ttggtgatta tgtctcagtt tagggtagat ggtttgagac ctaacttaga agtaacactg    120
ttttattcgg atggcccatc ctcttccgaa tgttaattta ctctgttctt catattcaga    180
tttccatgcc taatttctag ctcctacgtc tctattgcgg gacctaaaag tcgctgtttc    240
actaagcttt ggaacccgac tccaggaaat ctcattcttt cgtcccaaaa tttcaaccgk    300
aaagagaaat ccgcgcggcg gcctcttcaa gcgcccgggc cgggagcgcg ggttctgacc    360

ttcggtcgcc gggccggtct cccgcagcac cacgggtaag aggagcctga gcagctcgga    420
gggatgagtg cgggacggtg ggggctcccc ctcttcctgc cagtaccttg ccccaaggag    480
aagatgcctt aggacgcgac agatgaaaaa tcttttcttc tgcttggcgg aacactttgg    540
atgcggctta tggtgggtct tgagagcaag ctaagatgtc ccgccagacg ctcaggacc     599
```

<210> 4
<211> 599
<212> DNA
<213> Homo sapiens

<400> 4

```
cacccccctc ccaaccttgc ggacatcgcc tccggtcgcc tcttcgtaag gcctaagaag     60
cacgttagct gcgaacggag gtgaggaggc tcagctgacc gcctgtgtca gctgacaacg    120
tgtgacacgc acaaacgcca ggacttggct tggcctctct cttagttatt tgcagctctg    180
cccaatcggc gcctccggga cggcggagac ggtgggcttc ttgggtgcag ctccacgaag    240
gctggcatcc ctgcacagcg gtgaacacct gagggagacg ctcagtctct ctctaaagcm    300
acttctgcgg atgacacgga gataaataag agcagtgtgt catgagaggc cgtccaccag    360
gacttgccct cctttgccag ggtttgcacc tagcagagag actgttctgc ctctggccct    420
tggagcaggc tggctgacag cggagtaaag aaaaattact gcgggtgtgc agtcagtgca    480
aaacaattct ctgaccgata attgaacccg ggatgcggtg gtgaaagagc tgaatcatag    540
ccactagacc agcacggggg cacgggaggg tctttctcaa ccttcttgcc atataagtg     599
```

<210> 5
<211> 599

<212> DNA
<213> Homo sapiens

<400> 5

```
caagttcagc aaacttatat gtgtagttaa gatgctttac aacaaaatat tgcattttgt      60
tatagcaacg aagtattgag cagcttccaa gtcactacct tatgtgtatt attcatacta     120
gtaatactgg taaacattag cgatataatt ttcactacca aattttatta tatttttctt     180
agccaataca tggagcaaat ttttataaag tatttctttt caaatcattg aattcctcct     240
ccccaacctt taaatcccct agtcgccaag aatcttgggt tccacaccac cactttttgy     300
ttctgttatt aaagttgttg ccctgtgagc agtgggacac tacacccatc agctccaagg     360
acacatcatg gtcatttaca ttatctagta tcctgtggca tgattttagt atttcattca     420
agcactgaca acttttcgtg tcgattcaga ttttataaga tttgattaca gtgagtttat     480
aaaatatttc agttatatat gcaatagaaa tgaagtatcc tacttttgaa ggtaagtcta     540
aggcattcac agcaataaaa aagaagtact ttagtacttc tggacttcag tcaagggaa      599
```

<210> 6
<211> 599
<212> DNA
<213> Homo sapiens

<400> 6

```
aactccctac tcgctgccct acccgttcgt ctttgctcct ccttttcttt gctcttccgc      60
ttctctcctc cgcctcctca acttcttttc tagagccctc tcctcttttt cctgaccttc     120
ctaaaatggt tgtattttac agccctttgc ggctgatgaa ggcttcaaaa cctaaaaagc     180
aaacagatgc tccctaacac ctagctgaga atattttaac tcactacaaa ggcctttcaa     240
aaccccattc aaaatttccc accaacaaga agagaaccag tatttccctc tggagccgtm     300
ctaaggctgt tctccctgga gcagtgtcgg agcgattttg gactcttctc agagctgctc     360
agcttgtctg ccttcgccca gttgagaagc ccatcgtgga ttcgaagtat gtggtcacta     420
cagacactag aatccccaga ttcctctttt cttttttttt tctttttttt gagaccgagt     480
gcaatggcgt gatctcggct caccgcaacc tccgtcttcc aggttcaagc aattctcctg     540
cctcagcctc ccgagtagct gagattacag gcatgcacct ccacgccccgg ctaattttg     599
```

<210> 7
<211> 599
<212> DNA
<213> Homo sapiens

<400> 7

```
aattgggtag caagcatact catttctcag atcagtacac ctacttacgt tcctgctgtg      60
gtgagcaaat tttatactca atgtagtatc aggtattgat gaatctggtc acagctagaa     120
gaatttttta tatagggcct tgcatagctg tatgttgtgg ctggttacct gttaccaagg     180
attaagctgt acaggttttg gttacagtta cggaaaaata tgtcccttca ctacttatga     240
agccttggga tctcttttct gtatcacaac taaagtactg ctagatctgt gtgtgtgctr     300
ttagaatgca agcctaagtt tccaagttgg caagatttcc caacaaaaaa aaagatatag     360
aaaaaagagg ccacatctct gattgccagt ctaaaatttg gctacactca gaagtagctt     420
cacatattgc ttactaatgt agatgtttgg gggaagaagt agtgcattgc caaatttcag     480
aaaaagtaag tttttaacat taacaagctg agatttgagt ttcaaatata tgccacactt     540
catatagttt taatgtttcc aatttgtaac ttcatcacat actctctccc tcttggttt      599
```

<210> 8
<211> 599
<212> DNA
<213> Homo sapiens

<400> 8

```
tttggaaaat atgaacaagc tcattccaaa acttatctgg aaaagcatat aggtcccaga     60
acagctaaaa caatcttaac aaagaagaat aaaaggagag gactcactct gtccaatatt    120
aagccttatt atgatcaagt agtcttaatt acagtaatca acacaatgtt gtattgataa    180
agggacagac acacagatca atggaaaagt ttagagaacc cataagtagc cccacacatg    240
tatgtccaaa tgatttttga caagacacaa aagtaattca acgcaggaaa gatagcctty    300
tcaacaagta acaccagagc aattaaatat ccacaggcaa aaaccaaaac caaaagaaaa    360
cctctaacta aaccttatat tttatatgga aattaactca aaatggatca caaacttaaa    420
tataaacata taaccatgga atactatgca gccataaaaa atgatgagtt catgtcctct    480
gtagggacat tgatgaagct ggaaaccatc attctcagca aactatcgca aggacaaaaa    540
accaaacacc acatattctc actcataggt aggaattcaa caatgagaac acatggaca     599
```

<210> 9
<211> 599
<212> DNA
<213> Homo sapiens

<400> 9

```
tacgaaagag aatatcactg tcagccacca gcagtgcctt ttcagagaag agcaatgggg     60
aagaaattga gcagacaaag ccagaatccc cattagcaaa cagaaagagg gagctcagga    120
taaccacata cattaataat tcttgccccg caaagggaag ctctgtaaaa taagttgtat    180
tacatctgta catagcagta ctttaaatat aactctagct taagtatttt aagcatctcc    240
gtgatatgaa cctaagggaa taaactcaat aaatcaatat ttataagctc tgttcacttr    300
tgtcttgtgg tttcagccac tgatttcaga atatgcatga aaaatatatt tcttctgaat    360
atttgatttc atgatcccaa gtagacacat ctctgtattg ggtttcaaca agtccacaaa    420
agttaaatac ctacttttag ccagctttga ttttcagaag tttaattctg acatttagtg    480
atatacaata tgtaaaacaa cctggcacta tatctgttat atcataagta cttggcaaat    540
atttcagttt actctttctc ataattgaat aatggctcaa tagtaaaact ctgtaggga     599
```

<210> 10
<211> 599
<212> DNA
<213> Homo sapiens

<400> 10

```
tgcctaggac ttggtcaagg ccacagccaa gtatgggcag ggcaggctct tggccttgga     60

gctctgtgtc cagtgctcgc tccccacagt gccccccaac tcacccacag cagctgactc    120
agccccaacc tgcctctaat aaccacacac aaaagcagca agaaatgacc catactatct    180
tctgggcagg acactgcatc ctgcaggagg gacctttagg ctcattcctc catctgcgaa    240
gctgggatcc caggagactg ggaggtgat tggacttacc ctgtctgcct tcttgtgccr     300
tgtggacaca gcagagagag cccgctgtaa ctctcctgca aagtgccagg aatgatgcaa    360
gcggccggcg agatccttgg actctcctgg aatgagagag gttgagacac agcccaaagg    420
actcccccta aaggcctgtg aaagtgccag gttgaaggat gatggggtgc ccaggttccc    480
atcttcaaat ttcctggcag catcctggct gtaatagagc gctgtctcca gttcagtttt    540
ctgacacgtg agaattcgta ttgtatgatc ctgggccttt gggagaaaag acaagcaag     599
```

<210> 11
<211> 599
<212> DNA
<213> Homo sapiens

<400> 11

```
gacctcccaa agtgctggga ttacaggcgt gagccatctt gcccggccgg catttcagtt   60
ttattaaact gtctactgca tgccagttct tagtagcaca gtctcttttt ttgttttaga  120
aactcaaaac cttctaagtg atcacctggt taaataatgc aatcccataa atgacccctc  180
tcttctgatc catgcctggt gtccatttaa ttaaaccaga ttagaaaaag ggattattgc  240
tggctcaggg aggctgctgc cacagacaat gcctcacttc ggattgtgca gatataatay  300
gattgctcca agccagcctt catgtttaca cctgcctgga agctctcagg ccctggagta  360
acctcaggac actcctggcc ctgtctgcgg gtaaccagtt cttctctcag atgtgcggtc  420
tgtgctgttc ctccccatct gccagtacaa gtaaggtgtg gcacctgggc ccttctccca  480
ctcacattgc ctgtgtcctc tctctaaaag tcaccttaag accatgcggg taaagaggag  540
tgacttaaag gagctcaaat tcatcttact tttttccagt tgggaaattg gcaaaaagt    599
```

<210> 12
<211> 599
<212> DNA
<213> Homo sapiens

<400> 12

```
ctttagataa tttacctcta attagaaatt cagtcattca acaaatatct attgggtacc   60
gtgccaaggt aaggctctat gccaagtgct aaggggata caaaaatata aaagacacaa   120
tcctattttc agagagctga cattctggtt gggaagatga gacaaacatt tgataaacaa  180
caaaatattt cacaattcaa aagaggcagg acataactac agacaaaacc ctggacagaa  240
agaatttttt tttttgtaa atagtagttt agagaataca gcaagcactt tacttgatay   300
agttggcact gggtttacag aagaggtata aggtgggttg aggtcttgaa ggatggctgg  360
aactttatgg ttatatcaga gaagactcca ttcaaggaag ccataatagc atgaattgga  420
aagtgcacaa tttgagaaat gtgaagaaac catgtagttg gatccatgag cttcggacag  480
ccagatactg catcttgaga cttttaatta aaaattcaac catcatttct atacctaact  540
tctgcaaaac ttctatatgt aatatttctt aaaaccttta ctaattaagt aaccagcat    599
```

<210> 13
<211> 599
<212> DNA
<213> Homo sapiens

<400> 13

```
taacataaaa tttactggct ttctcatttt tgatttctct aaagtcttac tatttaaagc   60
aaaaataata acagattttg tgtctaaagc atatgtaaaa ttgtagtgta taaaaatagt  120
acataggtca gagggaggat tggagatata tatagatata tagagagaga tatatacaca  180
cacatacaca aacacattct tataatatgt aattttaatt ttatttttcc ctttattctt  240
atcattgtat atgtaatgtt tttgcagccc atttttgcc acttactatg tgttgaaggr   300
catgaaccaa tcacaatatt tgtaatgact tcatgataat ccctctgtgt gtcagtgaaa  360
atgtattcac agataaaagg ctcctgacta actaatttaa gcagagaagg actttgcaca  420

aggcattaaa ttgcttaatc catgacagga gtgagacagc tggatttaat gtctagaaat  480
gacacccaaa gacagcctgc accactaaaa gcccaggaga gttgcttctt tggacttagc  540
attaggccac ttgtattagt cacagttctc ctgagagtat tacacacatc tctctgtct    599
```

<210> 14
<211> 599
<212> DNA
<213> Homo sapiens

<400> 14

```
gagaggcaca cttatctggg atgcagtctg gggagtcctg gcgaggcagc ttccacctgc      60
tggaggaggg gccggggcgg agctaagatg cggaggaggg tgacgcacta gctctccagt     120
tcgcccgttc ctggcctgac ccccaccaag gcccataccg cagtaggctc ctcgggctgc     180
ccctcggtga gtacagtttt gatgtcggct cggccgcctg ccgccaaccc gagatttggt     240
attgccagtt gtgggagggc gtcctgctaa aatccttgag gtggaggctg gggtcagacr     300
aaggatgcgt aggggattag aatgtttggc tatcagtaag gggaagggag tactgaggga     360
ggagattgtg tagttcatca aatcagagcg gcgtttgctg ggatgacatc ctgcattcag     420
agtggacaag ggaaagatgg agatggagag cctcgtgtct gcctccagcc ttttccatca     480
gaattgcaga ttttgctgtt aaacagctac tctcagctct ttggagagca aggttttata     540
tctagtggct agaaaaggcc ttttctttgc agaaaaagaa attggagggt ataaaatt       599
```

<210> 15
<211> 599
<212> DNA
<213> Homo sapiens

<400> 15

```
tgtgcagaac aattgcttag ggtcggcatc tatggtaagt aagagaacat caaggctgtt      60
tcaccaaagg gcaggattta cgtgatgtac gtgctcttac acaagaaaca ttagataaaa     120
ccagagatct tagaggcttc ccccaaaccg gagttagtga gaagtcaata tggcagatta     180
gcatccaata tagagttgct ttggcctcca cagggtgctt tatagaaaat gcagtaaggt     240
agagcataaa gagatctcat gggaaattct gtaggcatga gttctaatcc tgatctgtar     300
ccaactagga taataataac ttaggcttca agcatgactg gaccctgaga cccaaacgct     360
gtcaccagta ctgactcttt ctctgtccct cacagcactg ttctttcttg tgttttctgc     420
ctcaggcatc tctcttcatg tgggggccct tgtcttctcc actccagact ttgatctgtg     480
cagcttaaca aacctccaga aagaaagcac ttccttccct gtggcactgg aaaaggtctc     540
agggctcaca tgcctttgtt ctgatggacc caggcttgag gcatgagttc accccacaa       599
```

<210> 16
<211> 599
<212> DNA
<213> Homo sapiens

<400> 16

```
gttagggtca tcaacatata ggagacctgc tgttgcttcc acttcctgct tgaactcttc      60
acgcaactgg agcgtacagc acagcactgg cagtttgctt tctatgcagg ctacgagctg     120
atccacatcc tctccccgag ttcctaaaac tgaagatgtt cacaatgtca gagaaatgct     180
cacgagatgg ccaatgacaa gagggcaggg ctccctcttc cactactggg aaggtcaaaa     240
gctcgaggta ctccagttac cgggagccat ggagatttaa acaaaaaaag gccctcaacm     300
cagatatgga agaattaggg ttagaaacag acacagctgg ctgtagcagg tgtggagtgt     360
ttctctcaca gatgtggccc acaatgacat actccagtgt tagaaatcac tcttcccaca     420
gcaagcctac ttcaggcagg ctggagacat cacaccatag gttcagcaca aaaaggcaac     480
cacagattct gtacattcct atgactgaga gaaagggaag tactgtggtg ggctgctgtc     540
cccacacagg aaggacaggg agatggtgac agcgccgggc ctcagggctc actggctta       599
```

<210> 17
<211> 599
<212> DNA
<213> Homo sapiens

<400> 17

```
tcatctcagt gtgacctagt tcagcttccc catctcttag gcagatacca ttgggttcag     60
aaaggttcaa gggcactttt tctcctctta gcttagtgct ttctcaaata gtccatgact    120
tatgacctta tgacgatgcc gtgtggtgag tctgaaatca gatgaagatt tttatcctga    180
catgatgaac acaagcattg cataacctga ctctggttac ccaggatcta tcctctgctt    240
gtggtcacca ctgctaggat tttggaagaa ccctctaaaa actaagctct ctgaggaccr    300
gcatttttgt ttggttcact attgtgatgc caatgattag aagaatgcct gggcccatag    360
gagggactca tgttcaatgc agaggtggat aaatgagaaa gagactgttt gccgcagaat    420
actaagagat tttattgttc ttgtcatagg aaccaggcaa tattccagac acacatgaca    480
tcagtaacat ccatatgtct gtgatggggt aggagagaag actacttttc aggtgggaca    540
ttttgattgg agagggtctc tgattcagag agtgaggtga gctattttct tcttgggag     599
```

<210> 18
<211> 599
<212> DNA
<213> Homo sapiens

<400> 18

```
tgtttgaacc cagcaggtgg aggttgcagt gagccgagat cggccactgc actccagcct     60
gggcgacagt gcaagactcc atctcaaaaa aaaaaaaaag aaagaaagga ctcttgagat    120
cttctcagag ctctttgaga ggaaagattg gattattgca cttctttggc acaaagtagt    180
ctttgggaat gggaagcagt gaaggaaggg tcaaggggaa aatagtttct ttcagaattg    240
caaggtggat tgttttgggt attgtctccc aattcttata tgttaggttg taaaaattak    300
ttctgataca gagcaactgt cttcccccca actttttgga gaagcagtca ctgaggtgat    360
gggagagtga agaattgagt aggctagagc acagaggttt agctctcaag ttgatgtctt    420
ttatttaatc cagtcagtta tcttttgtag gttacattgg cgaatttgaa atcattgatg    480
accacagagc tgggaaaatt gttgtgaacc tcacaggcag gctaaacaag gtaagaacga    540
gtgatctaca catttcaaag ctttaagaat tttttactgt ggcgttaaat gttgtaatt     599
```

<210> 19
<211> 599
<212> DNA
<213> Homo sapiens

<400> 19

```
ctcccgggtt cgagcgattc tcctgcctca gtctcctgag tagctgggac tacaggcatg     60
tgccacctcg cccggctaat ttttgtattt ttagtagaga tggggtttca ccatgttggc    120
caggctgctc tcaaactccc gacatcaagt gatccacctg ccttggcctc ccaaagtgct    180
gggattacag gcatgagcca ccgcacccag ccttggagct ggcttttaaa aatgaagaga    240
gtgcatcagc cagcaaggcg gggaagagca ttccaaaaag agggacgggc agtagtgagr    300
gcaggcagag ggagtctgtg ggcgtgcggg agatgagggg tgggctgaga tgcagggagt    360
ggttcgaggt gaagctggag tggtcgaggg tagtctttgg gaagcccttt tatggagtct    420
ggactctagc aatggggagc catcgaaggg ttttgagtga gggcggaaaa gattagatta    480
tgtgcagaag gactagtttg gttgagtggg atggattaga gaggctggag gaagggaggc    540
cttgaggagg ctgaggacag tgtccccagg gagggcggcc agtatgggat ggagagatg     599
```

<210> 20
<211> 599
<212> DNA
<213> Homo sapiens

<400> 20

```
cttgaccagc tttggaagca ggcggtctaa tagttcccat ttctcaggag acaacaggct     60
cagaaaggtt aagtgatttt ctgtggttac acagccagta aatgatggcg ctggggctgc    120
```

```
agcccagacc tctctggccc tgaagccttg gcctttcctc cttcctgctt ggcacttctc 180
ccctggtctg tgagctcctt ggggcaggag catttctggt cggctgctgt aaccgtggtg 240
cccagcatga gccccaggga gcacttgatg aattaactgg cctgagttca gggcaccaar 300
ggatgagcag acaggatgag gcttttggaa aaaataaaca tctccagctg ctttcaaagg 360
atgcatgtaa taaacaaggt cccagcagaa tgaaatggag gagctgaagg tggagactgg 420
ctgggaggaa ggaaagaatt ggttgagtcg gagtggtccc actctggatt caaggccacg 480
tttgctgagc acttacccag tctttggctg ggccctgagt cctcagagat gagtccacca 540
ccattcctgc cccccaggcg gctcccagtt aggaagaggg cgtcagacag tcacccagc 599
```

<210> 21
<211> 599
<212> DNA
<213> Homo sapiens

<400> 21

```
acacagtggt ccatgagccc tagacggcca acctcccagg cttgtgtgtg agaagaggta 60
tgctctggag gggaagctgc cactttcagc tcaccctgac tactgagtcg tctccagggg 120
cggctgagtt cttacatcag acccttggaa tcattggtag tttgggggtg ttggcttcac 180
ctgccagttc ctccacccac atgcaggctg taggcacctc agagagggct gctacccttg 240
gaatctcaga gtgtgaaaga gaagaggccc ttgcgggagg ggaggacccc cgggaaagcr 300
cacccacctc cttgctgtga gaggggactc cggagtcttc tctttgctgt ttcatgggag 360
gagagtcaca atgagtgaaa tgacaagtgt tttctatgtt taaattttta tattccaccc 420
cttacttggc aaccggaacc cccaggctac gtttttttaca catatagaat tccaagtcca 480
gagttgaaat ttgagagcag aggcgtcacc cgaatccctt ggggggtcagc tcattaaaaa 540
ttcaggttcc caggtccctc ccggaatcca gagtccagca gagctccctg ctctccctc 599
```

<210> 22.
<211> 599
<212> DNA
<213> Homo sapiens

<400> 22

```
gaacatcaga aggaacaaac tgcggacacg cggcctttaa gaactgttaa cactcaccgc 60
gagggtccgc ggcttcattc ttgaaatcag tgagaccaag aacccaccaa ttccagacac 120
actagcaggg agaagcaggc cctaagccca gtaagcacac aggtaaacaa gtgagtaagt 180
aggattgctc cagaaagtgg caactgcaga gaagagaaac agcccatagg ggtgagagcg 240
ggtagctgac ggagcgtggc tattgtggac tggccttcag gggcctctg aagagttgay 300
atttgagcga cttgaacgat gtattccagt tggaggcagt cacctaaggc ctaaagtggg 360
acatggcatt gtgcatgttt tttgctttgt tttgttttac atttttcttt tctttctttt 420
tttttttttt ttgagacagc gtctcactct gtcacccagg ctggagggca gtggcataat 480
cacggctcac tgcagcctca aattccccag gctcaggtga tcctctcacc tcagcctccc 540
aagtagctga gatcacaggc acatggcacc acacctggct aatttttgta tttttgta 599
```

<210> 23
<211> 599
<212> DNA
<213> Homo sapiens

<400> 23

```
ggaggccaga agcagcggct gagcctggcc cgggctgtat acagaaaggc agctgtgtac      60
ctgctggatg accccctggc ggccctggat gcccacgttg gccagcatgt cttcaaccag      120
gtcattgggc ctggtggact actccaggga acagtaagtt tgggaacatg tgtcagacag      180
tacagggcaa aggcagagga agcacttagc atccagtcct aacccaagtt tatctcacct      240
ccccttcca cttgagtcca atttcctctt tgtattggtc agcttttgct gtgacaatgy      300
tatgtaacaa acaaccccca gatcccagca gcttacaaca gcaggtgttt cttccttatg      360
gatctgtgat ttaactacta cagctttgcc ttggatgatt ggccagggtc agatgtactc      420
cttgtcttct tgttttgaga cacaggctaa gggagtcccc tctgtttctc tatttggata      480

tgctgtttac aagaaaggcg gcaggagcac aagaagggga gctgtcccca ctctgagtcc      540
agggacaata cccccacccc aacccccagc tcaggaggct ggccaagcac atgtgtgta       599
```

**Description**

## INTRODUCTION

Genetic risk is conferred by subtle differences in individual genomes within a population. Genes differ between individuals due to genomic variability, most frequently due to single nucleotide polymorphisms (SNPs). SNPs are located on average every 500-1000 base pairs in the human genome. Additional genetic polymorphisms in the human genome are caused by duplications, insertion, deletion, translocation or inversion of either short or long stretches of DNA. Genetic variability among individuals thus in general occurs on many other scales, ranging from single nucleotide changes to gross, microscopically visible, alterations in chromosomal structure and function. Recently, an abundance of submicroscopic copy number variations (CNVs) of DNA segments ranging from a few kilobases to megabases in size have been discovered (summarized in Redon, R. et al. Nature 444:444-54 (2006) and Estivill, X. & Armengol, L. PloS Genetics 3:e190 (2007)). These CNVs include deletions, insertions, duplications and complex multi-site variants. To date, known CNVs account for over 15% of the assembled human genome (Estivill, X. Armengol, L. PloS Genetics 3:e190 (2007)), although most of these variants are so rare that they cover only a small percentage of the human genome of any particular individual.

As genetic polymorphisms conferring risk of common diseases are uncovered, genetic testing for such risk factors is becoming important for clinical medicine. Examples are Apolipoprotein E testing to identify genetic carriers of the ApoE4 polymorphism in dementia patients for the differential diagnosis of Alzheimer's disease, and of Factor V Leiden testing for predisposition to deep venous thrombosis. More importantly, in the treatment of cancer, diagnosis of genetic variants in tumor cells is used for the selection of the most appropriate treatment regime for the individual patient. In breast cancer, genetic variation in estrogen receptor expression or heregulin type 2 (Her2) receptor tyrosine kinase expression determine if anti-estrogenic drugs (tamoxifen) or anti-Her2 antibody (Herceptin) will be incorporated into the treatment plan. In chronic myeloid leukemia (CML) diagnosis of the Philadelphia chromosome genetic translocation fusing the genes encoding the Bcr and Abl receptor tyrosine kinases indicates that Gleevec (STI571), a specific inhibitor of the Bcr-Abl kinase should be used for treatment of the cancer. For CML patients with such a genetic alteration, inhibition of the Bcr-Abl kinase leads to rapid elimination of the tumor cells and remission from leukemia.

Schizophrenia is a heritable, highly debilitating psychotic disorder that affects 0.5 to 1% of the general population. The illness is characterized by a variety of positive and negative signs and symptoms, as well as cognitive dysfunction that typically commence in early adulthood and often continue throughout life. The broad phenotypic presentation and a lack of complete disease concordance in monozygotic twins (~50-60%) imply that a multitude of environmental and/or genetic factors might contribute to disease manifestation (Coyle et al., Ann. NY Acad. Sci. 1003: 318-27, 2003). Twin and adoption studies suggest that both genetic and environmental factors influence susceptibility (see, *e.g.*, Tsuang, M.T. et al., Schizophr. Res. 4(2): 157-71 (1991); Tienari, P.J. and Wynne, L.C., Ann. Med. 26(4):233-7 (1994); Franzek, E. and Beckmann, H., Am. J. Psychiatry 155(1):76-83 (1998); Tsuang, M. T., J. Biomed. Sci. 5(1):28-30 (1998)).

Among first-degree relatives, the genetic risk for schizophrenia has been reported to vary from 6% in parents, to 10% in siblings, and to 13% in children of schizophrenic individuals; if one of the parents is also schizophrenic, the risk to siblings increases to 17%, and children of two schizophrenics have a risk of 46% of developing the illness (McGue, M. and Gottesmann, I.I., Eur. Arch. Psychiatry Clin. Neurosci 240:174-181 (1991); see also, *e.g.*, Lim, L.C. and Sim, L.P., Singapore Med. J. 33(6):645-7 (1992)). The mode of transmission, however, remains uncertain.

A large number of chromosomal regions have been implicated to be involved in the pathogenesis of schizophrenia, through linkage studies. Reports of suggestive linkage to several loci have been published, including loci on chromosomes 3, 5, 6, 8, 10, 13, 20, 22 and the X chromosome (see, *e.g.*, for chromosomes 3p and 8p, Pulver, A.E., et al., Am J Med Genet 60(4):252-60 (1995); for chromosomes 5q, 6p and 8p, Kendler, K.S. et al., Am J Med Genet 88(1):29-33 (1999); for chromosomes 5q, 6p, 8p, 20p and 22q, Hovatta, I. et al., Mol Psychiatry 3(5):452-7 (1998); for chromosome 6p, Schwab, S.G. et al., Nat Genet 11(3):325-7 (1995), Brzustowicz, L.M. et al., Am J Hum Genet 61(6):1388-96 (1997) and Cao, Q. et al., Genomics 43(1):1-8 (1997); for chromosomes 6 and 8, Straub, R.E. et al., Cold Spring Harbor Symp Quant Biol 61I:823-33 (1996); for chromosome 8, Kendler, KS. et al., Am J Psychiatry 153(12):1534-40 (1996); for chromosome 10, Straub, R.E. et al., Am J Med Genet 81(4):296-301 (1998) and Schwab, S.G. et al., Am J Med Genet 81(4):302-307 (1998); for chromosome 13, Lin, M.W. et al., Psyciatr Genet 5(3):117-26 (1995); Lin, M.W. et al., Hum Genet 99(3):417-420 (1997) and Blouin, J.L. et al., Nat Genet 20(1):70-73 (1993) (8 and 13); for chromosome 22, Gill, M. et al., Am J Med Genet 67(1):40-45 (1996) and Bassett, A.S. et al., Am J Med Genet 81(4):328-37 (1998); and for the X chromosome, Milunsky, J. et al., Clin Genet 55(6):455-60 (1999)). However, many of these studies remain to be validated, and evidence for individual underlying genetic variants has not emberged.

Several of the genes recently described as susceptibility candidates for schizophrenia are believed to affect neuroplasticity, as well as glutamatergic neurotransmission (Harrison & Owen, Lancet 361: 417-9, 2003). With the discovery of a number of schizophrenia susceptibility genes, a molecular hypothesis has begun to emerge.

In a genome wide scan of schizophrenia families carried out in Iceland, a susceptibility gene was mapped to chromosome 8p21. Haplotype analysis identified Neuregulin 1 (NRG1) as a gene conferring susceptibility to schizophrenia (Stefansson et al., Am. J. Hum. Genet., 72: 83-7, 2003). NRG1 as a schizophrenia disease gene has been replicated in multiple populations (Stefanson et al., Am. J. Hum. Genet. 72: 83-7, 2003; Williams et al., Mol. Psychiatry 8:485-7, 2003; Yang et al., Mol. Psychiatry 8:706-9, 2003). NRG1 is a polypeptide growth factor implicated in the modulation of neurotransmission in developing and adult synapses. Early studies focused on the neuromuscular junction, where NRG1 was identified as acetylcholine receptor-inducing activity (ARIA) factor (Jessell et al., Proc. Natl. Acad. Sci., 76: 5397-5401, 1979; Falls et al., J. Neurocytol., 32: 619-647, 2003).

Dopamine receptor antagonists, primarily D2 receptor selective antagonists, are used clinically for the control of the positive signs of schizophrenia, suggesting that the misregulation of dopamine neurotransmission contributes to disease pathophysiology (Freedman, N. Engl. J. Med., 349: 1738-1749, 2003). However, the dissociative anesthetics that block the NMDA receptor, such as phencyclidine (PCP) and ketamine, produce a schizophrenia-like disorder. Hence, a role for NMDA receptor hypofunction in the disease has also been suggested. (Reviewed in Konradi & Heckers Pharmacology & Therapeutics, 97: 153-197, 2003) Unlike manipulation of dopamine, for example by chronic exposure to amphetamines creating positive symptoms, exposure to dissociative anesthetics acutely reproduces the negative and cognitive signs of schizophrenia. NMDA receptors are ion channels that function as coincidence detectors. They are simultaneously gated by voltage, as well as by two ligands, glutamate and glycine. Serine/threonine and tyrosine phosphorylation also strongly regulate NMDA receptor function (Yu et al., Science, 275: 674-678, 1997; Wang et al., Nature, 369: 233-235, 1994; Slater et al., Nat. Rev. Neurosci., 5: 317-328, 2004). Subtle misregulation of either membrane potential, ligand binding or tyrosine phosphorylation may therefore have profound effects on the probability and duration of NMDA channel opening, thus influencing behavior modulated by the NMDA receptor (Moghaddam, Neuron, 40: 881-884, 2003).

Despite these advances towards an understanding of the etiology of schizophrenia, there are many questions still unanswered, and a large fraction of the genetic contribution to the disease remains unaccounted for. Identification of underlying genetic variants will aid in the identification of those individuals who are at particular risk of developing the disease, and will be useful in a diagnostic setting and for disease management. There is also a great need to identify new treatments for schizophrenia, and the identification of novel genetic risk factors may assist in the development of potential therapeutics and anti-schizophrenic agents, as well as accurate and informative *in vitro* and *in vivo* assays for predicting and elucidating the effectiveness of potential treatments.

## SUMMARY OF THE INVENTION

The present inventors have discovered that certain copy number variations are present in increased frequency in individuals diagnosed with schizophrenia than in the general population. These copy number variations are therefore predictive of schizophrenia in carriers. The copy number variations are useful in various methods and kits that are useful for risk management of schizophrenia and related conditions, as described further herein.

In a first aspect, the invention provides a method of determining a susceptibility to a schizophrenia condition in a human individual, the method comprising:

obtaining nucleic acid sequence information about a human individual identifying the chromosome 15q11.2 deletion in the genome of the individual, wherein the presence and absence of the chromosome 15q11.2 deletion are associated with different susceptibilities to the condition in humans, and

determining by use of a computer a susceptibility to the condition for the individual from the nucleic acid sequence data.

Obtaining sequence information can in general be done by any method known to the skilled person, including use of polymorphic markes, nucleotide probes and other methods as further described herein.

In some embodiments, determination of a susceptibility comprises comparing the nucleic acid sequence information to a database containing correlation data between copy number variation polymorphisms and susceptibility to the condition. The database can for example comprise a look-up table comprising information about sequence in individuals. Correlation data can for example be a risk measure of schizophrenia for individuals who carry particular copy numer variations in the genome. Such risk measures can for example be represented by an odds ratio (OR), a risk ratio (RR), or an increased in percentage. Other suitable measures known to the skilled person may also be used for this purpose, and are within scope of the invention.

In certain embodiments, obtaining nucleic acid sequence information comprises obtaining a biological sample from the human individual and analyzing at least one polymorphic marker within the chromosome 15q11.2 deletion in a nucleic acid in the sample.

Thus the polymorphic marker is in such embodiments a physical representative of the copy number variation, since the presence or absence of the segment defining the copy number variation translates directly into the presence of at least one particular allele of the polymorphism. In preferred embodiment, the polymorphism is a SNP, wherein a particular allele of the SNP is representative of the copy number variation.

In preferred embodiments, analyzing the at least one polymorphic marker comprises obtaining dosage measurement data for the at least one polymorphic marker representative of the at least one copy number variation. Dosage data is data that is indicative of the quantitative amount of particular alleles of polymorphic markers, such as data indicative of the amount of a particular allele of a SNP. Such dosage data is in certain embodiments data comprising a fluorescence signal from a nucleotide probe indicative of a particular allel of a SNP that is representative of the copy number variation.

In certain embodiments, obtaining nucleic acid sequence information comprises obtaining a nucleic acid sample from the individual and identifying at least one copy number variation using a nucleic acid probe selective for a nucleic acid segment that comprises the copy number variation. In such embodiments, the nucleotide probe is representative of a particular genomic segment, such as the CNV itself. The nucleotide probe may or may not be representative of a polymorphic marker such as a SNP. In certain embodiments, the nucleic acid probe comprises a label, and wherein identifying at least one copy number variation comprises allowing the nucleic acid probe to hybridize to the nucleic acid segment, such that when bound to the nucleic acid segment, the label is representative of the number of copies of the segment in the individual.

The sequence data representative of at least one copy number variation can in certain embodiments by obtained from a preexisting record. Such preexisting record can be any table, such as a look-up table, database or other storage media or record containing such sequence data.

The method of determining a suscepbility to a schizophrenia condition can include a further step comprising reporting the susceptibility to at least one entity selected from the group consisting of the individual, a guardian of the individual, a representative of the individual, a genetic service provider, a physician, a medical organization, and a medical insurer. Other single entities, including any one of the above-mentioned entities may be targeted by such reporting in particular embodiments, as can any combination of the above-mentioned entitites.

The markers and CNVs described herein can be combined with other risk factors for schizophrenia. Thus, certain embodiments combine assessment of particular CNVs, as described herein, with assessment of at least one additional genetic risk variant for schizophrenia, so as to determine overall risk in the individual. In certain embodiments, such additional risk factors are selected from SNPs, microsatellites or insertion/deletion polymorphisms.

The invention also provides computer-implemented aspects. In one such aspect, a computer-readable medium is provided, the medium having computer executable instructions for determining susceptibility to a schizophrenia condition in a human individual, the computer readable medium comprising:

data indicative of the chromosome 15q11.2 deletion;

a routine stored on the computer readable medium and adapted to be executed by a processor to determine based on the data risk of developing a schizophrenia condition.

In some embodiments, the computer readable medium contains data indicative of at least one polymorphic marker that is indicative of the at least one copy number variation. In some other embodiments, the at least one polymorphic marker is in linkage disequilibrium with the at least one copy number variation. In particular embodiments, data indicative of at least one haplotype comprising two or more polymorphic markers are included.

Another aspect relates to an apparatus for determining a genetic indicator for a schizophrenia condition in a human individual, comprising (i) a processor; (ii) a computer readable memory having computer executable instructions adapted to be executed on the processor to analyze information about the chromosome 15q11.2 deletion, and (iii) generate an output based on the information about the chromosome 15q11.2 deletion, wherein the output comprises a risk measure of the at least one copy number variation as a genetic indicator the schizophrenia condition for the human individual.

In certain embodiments, the computer readable memory further comprises data for at least one polymorphic marker in a plurality of individuals diagnosed with the schizophrenia condition, and data for the at least one polymorphic marker in a plurality of reference individuals, wherein the data is representative of at least one copy number variation, and wherein a risk measure is based on a comparison of marker data for the at least one marker for the human individual to marker data for the plurality of individuals with the schizophrenia condition. In some embodiments, the data for the at least one polymorphic marker is dosage data for the at least one marker. In some embodiments, the computer readable memory further comprises data indicative of the risk of developing the schizophrenia condition associated with at least one copy number variation, and wherein a risk measure for the human individual is based on a comparison of status of the at least one copy number variation for the human individual to the risk associated with the at least one copy number variation. In some embodiments, the computer readable memory further comprises data indicative_of the frequency of at least one copy number variation in a plurality of individuals diagnosed with the schizophrenia condition, and data indicative of the frequency of at the least one copy number variation in a plurality of reference individuals, and wherein risk of developing the schizophrenia condition is based on a comparison of the frequency of the at least one copy number variation in individuals diagnosed with the schizophrenia condition and reference individuals. In preferred embodiments, the risk measure is characterized by an Odds Ratio (OR) or a Relative Risk (RR).

In certain embodiments of the invention, the schizophrenia condition is schizophrenia.

It should be understood that all combinations of features described herein are contemplated, even if the combination of feature is not specifically found in the same sentence or paragraph herein.

## BRIEF DESCRIPTION OF THE DRAWINGS

FIG 1 shows the genomic architecture of the 1q21.1, 15q11.2 and 15q13.3 deletions. (A) DosageMiner output showing the shorter form of the 1q21 deletion (horizontal line with vertical lines representing the start and end of the deletion). Ninety-nine SNPs on the HumanHap300 chip are affected by the deletion which spans 1.38 Mb. (B) DosageMiner output showing the 15q11.2 deletion. 54 SNPs on the HumanHap300 chip are affected by the deletion which spans 580 kb. C) DosageMiner output showing the 15q13.3 deletion. One-hundred-sixty-six SNPs on the HumanHap300 chip are affected by the deletion which spans 1.57 Mb. Genes affected by the deletions are shown (Coordinates are based on Build 36 of the human geneome and positions of genes derived from the UCSC genome browser). LCRs flank all three deletions.

FIG 2 shows the genomic architecture of the 1q21.1 deletions. Many large low copy repeats (LCR) with high homology are found at the 1q21.1 locus. LCRs (large arrows) on the picture may mediate NAHR accounting for the larger form of the 1q21.1 deletion. There are though many smaller repeats, 1,000-10,000 bp (not shown on the figure) that potentially could mediate the formation of the deletion. A smaller repeat marked by arrowheads in the figure may assist NAHR accounting for the smaller form of the deletion. Again, for this form of the deletion there are other smaller LCR that potentially could assist with the formation of the deletion (not shown on the figure). Segments that contain SNPs on the Illumina HumanHap300 chip are also indicated. Note that there are no SNPs on 1q on the Illumina HumanHap300 chip centromeric to the larger form of the 1q21.1 deletion. Thus, exact size of the larger form of the deletion is not precisely known, the minimum size is 2.19 Mb. Markers on the p-arm are not deleted in the four cases or the control with the larger form of the 1q21.1 deletion.

**FIG 3** shows (A) DosageMiner output showing the shorter form of the 1q21 deletion (horizontal line with vertical lines representing the start and end of the deletion). Ninety-nine SNPs on the HumanHap300 chip are affected by the deletion which spans 1.38 Mb. B) DosageMiner output showing the larger form of the 1q21.1 deletion. C) Affected genes by both deletions (within shorter form of the deletion; coordinates are based on Build 36 of the human genome and positions of genes derived from the UCSC genome browser). LCRs flank both deletions (see FIG 2); (D) Analysis of the 1q21.1 deletion with fluorescence in situ hybridization (FISH). Two BAC probes, RP11-431G14 (cover the PRK gene on chromosome 1q21) labeled with biotin and an anchor BAC, RP11-458I7 labeled with digoxigenin were used as probes for FISH analysis. A cell from a normal control (left) in interphase shows normal FISH signals, one biotin probe and one digoxigenin probe per chromosome. A cell from a schizophrenia patient (center) with the 1q21.1 deletion shows aberrant FISH signal, the biotin signal is missing for one of the chromosomes. A cell from a schizophrenia patient with the 1q21.1 region duplicated (right), two biotin signals are seen for one of the two chromosomes.

**FIG 4** shows (A) LCRs flanking the deletion at 15q11.2. Several LCR at this locus can mediate the formation of the deletion. The grey horizontal bar shows the minimum size of the deletion, and vertical arrows point to the regions with longest homologous sequences on both sides of the deletion, harbouring possible breakpoints. Coordinates are in line with Build 36 of the human genome.

**FIG 5** shows LCRs flanking the deletion at 15a11.2. It is not clear which LCR might mediating the formation of the recurrent deletion. Grey horizontal bar shows the minimum size of the deletion, and vertical arrows point to the only high homology sequence with same orientation on both sides of the deletion in the UCSC human genome reference sequence. Coordinates are in line with Build 36 of the human genome.

**FIG 6** shows a genome browser view showing the positions of the 16p13.1 CNV relative to genes in the region, and the duplications and deletions found in the present study. Known segmental duplications of >1000 bp is also shown. The region is divided into three intervals called 1,2 and 3. Trace 1 shows deletion interval of interval 2, trace 2 shows duplication of interval 2,trace 3 shows deletion of intervals 1 and 2, trace 4 shows duplication of intervals 1 and 2, trace 5 shows deletion of intervals 2 and 3, and trace 6 shows duplication of intervals 2 and 3.

**FIG 7** shows a genome browser view of the chr 5q35.2 duplicated region. The figure is taken from the UCSC Browser, genome Build 36, and shows the position and orientation of genes in the region, as well.as position of SNP markers on the Illumina HumanHap300 chip in this region.

**FIG 8** shows an exemplary computer environment on which the methods and apparatus as described and claimed herein can be implemented.

## DETAILED DESCRIPTION

*Definitions*

Unless otherwise indicated, nucleic acid sequences are written left to right in a 5' to 3' orientation. Numeric ranges recited within the specification are inclusive of the numbers defining the range and include each integer or any non-integer fraction within the defined range. Unless defined otherwise, all technical and scientific terms used herein have the same meaning as commonly understood by the ordinary person skilled in the art to which the invention pertains.

The following terms shall, in the present context, have the meaning as indicated:

A "polymorphic marker", sometime referred to as a "marker", as described herein, refers to a genomic polymorphic site. Each polymorphic marker has at least two sequence variations characteristic of particular alleles at the polymorphic site. Thus, genetic association to a polymorphic marker implies that there is association to at least one specific allele of that particular polymorphic marker. The marker can comprise any allele of any variant type found in the genome, including SNPs, mini- or microsatellites, translocations and copy number variations (insertions, deletions, duplications). Polymorphic markers can be of any measurable frequency in the population. For mapping of disease genes, polymorphic markers with population frequency higher than 5-10% are in general most useful. However, polymorphic markers may also have lower population frequencies, such as 1-5% frequency, or even lower frequency, in particular copy number variations (CNVs). The term shall, in the present context, be taken to include polymorphic markers with any population frequency.

An "allele" refers to the nucleotide sequence of a given locus (position) on a chromosome. A polymorphic marker allele thus refers to the composition (i.e., sequence) of the marker on a chromosome. Genomic DNA from an individual contains two alleles (e.g., allele-specific sequences) for any given polymorphic marker, representative of each copy of the marker on each chromosome. Sequence codes for nucleotides used herein are: A = 1, C = 2, G = 3, T = 4. For microsatellite alleles, the CEPH sample (Centre d'Etudes du Polymorphisme Humain, genomics repository, CEPH sample 1347-02) is used as a reference, the shorter allele of each microsatellite in this sample is set as 0 and all other alleles in other samples are numbered in relation to this reference. Thus, e.g., allele 1 is 1 bp longer than the shorter allele in the CEPH sample, allele 2 is 2 bp longer than the shorter allele in the CEPH sample, allele 3 is 3 bp longer than the lower allele in the CEPH sample, etc., and allele -1 is 1 bp shorter than the shorter allele in the CEPH sample, allele -2 is 2 bp shorter than the shorter allele in the CEPH sample, etc.

Sequence conucleotide ambiguity as described herein is as proposed by IUPAC-IUB. These codes are compatible with the codes used by the EMBL, GenBank, and PIR databases.

| IUB code | Meaning |
|---|---|
| A | Adenosine |
| C | Cytidine |
| G | Guanine |
| T | Thymidine |
| R | G or A |
| Y | T or C |
| K | G or T |
| M | A or C |
| S | G or C |
| W | A or T |

(continued)

| IUB code | Meaning |
|---|---|
| B | C G or T |
| D | A G or T |
| H | A C or T |
| V | A C or G |
| N | A C G or T (Any base) |

A nucleotide position at which more than one sequence is possible in a population (either a natural population or a synthetic population, *e.g.*, a library of synthetic molecules) is referred to herein as a "polymorphic site".

A "Single Nucleotide Polymorphism" or "SNP" is a DNA sequence variation occurring when a single nucleotide at a specific location in the genome differs between members of a species or between paired chromosomes in an individual. Most SNP polymorphisms have two alleles. Each individual is in this instance either homozygous for one allele of the polymorphism (i.e. both chromosomal copies of the individual have the same nucleotide at the SNP location), or the individual is heterozygous (i.e. the two sister chromosomes of the individual contain different nucleotides). The SNP nomenclature as reported herein refers to the official Reference SNP (rs) ID identification tag as assigned to each unique SNP by the National Center for Biotechnological Information (NCBI).

A "variant", as described herein, refers to a segment of DNA that differs from the reference DNA. A "marker" or a "polymorphic marker", as defined herein, is a variant. Alleles that differ from the reference are referred to as "variant" alleles.

A "microsatellite" is a polymorphic marker that has multiple small repeats of bases that are 2-8 nucleotides in length (such as CA repeats) at a particular site, in which the number of repeat lengths varies in the general population. An "indel" is a common form of polymorphism comprising a small insertion or deletion that is typically only a few nucleotides long.

A "haplotype," as described herein, refers to a segment of genomic DNA that is characterized by a specific combination of alleles arranged along the segment. For diploid organisms such as humans, a haplotype comprises one member of the pair of alleles for each polymorphic marker or locus along the segment. In a certain embodiment, the haplotype can comprise two or more alleles, three or more alleles, four or more alleles, or five or more alleles. Haplotypes are described herein in the context of the marker name and the allele of the marker in that haplotype, *e.g.*, "2 rs2283508" refers to the 2 allele of marker rs2283508 being in the haplotype, and is equivalent to "rs2283508 allele 2". Furthermore, allelic codes in haplotypes are as for individual markers, i.e. 1= A, 2 = C, 3 = G and 4 = T.

The term "susceptibility", as described herein, refers to the proneness of an individual towards the development of a certain state (*e.g.*, a certain trait, phenotype or disease), or towards being less able to resist a particular state than the average individual. The term encompasses both increased susceptibility and decreased susceptibility. Thus, particular alleles at polymorphic markers and/or haplotypes of the invention as described herein may be characteristic of increased susceptibility (i.e., increased risk) of schizophrenia, as characterized by a relative risk (RR) or odds ratio (OR) of greater than one for the particular allele or haplotype. Alternatively, the markers and/or haplotypes of the invention are characteristic of decreased susceptibility (i.e., decreased risk) of schizophrenia, as characterized by a relative risk of less than one.

The term "and/or" shall in the present context be understood to indicate that either or both of the items connected by it are involved. In other words, the term herein shall be taken to mean "one or the other or both".

The term "look-up table", as described herein, is a table that correlates one form of data to another form, or one or more forms of data to a predicted outcome to which the data is relevant, such as phenotype or trait. For example, a look-up table can comprise a correlation between allelic data for at least one polymorphic marker and a particular trait or phenotype, such as a particular disease diagnosis, that an individual who comprises the particular allelic data is likely to display, or is more likely to display than individuals who do not comprise the particular allelic data. Look-up tables can be multidimensional, *i.e.* they can contain information about multiple alleles for single markers simultaneously, or the can contain information about multiple markers, and they may also comprise other factors, such as particulars about diseases diagnoses, racial information, biomarkers, biochemical measurements, therapeutic methods or drugs, etc.

A "computer-readable medium", is an information storage medium that can be accessed by a computer using a commercially available or custom-made interface. Exemplary compute-readable media include memory (e.g., RAM, ROM, flash memory, etc.), optical storage media (e.g., CD-ROM), magnetic storage media (*e.g.*, computer hard drives, floppy disks, etc.), punch cards, or other commercially available media. Information may be transferred between a system of interest and a medium, between computers, or between computers and the computer-readable medium for storage or acess of stored information. Such transmission can be electrical, or by other available methods, such as IR links, wireless connections, etc.

A "nucleic acid sample" as described herein, refers to a sample obtained from an individual that contains nucleic acid (DNA or RNA). In certain embodiments, i.e. the detection of specific polymorphic markers and/or haplotypes, the nucleic acid sample comprises genomic DNA. Such a nucleic acid sample can be obtained from any source that contains genomic DNA, including a blood sample, sample of amniotic fluid, sample of cerebrospinal fluid, or tissue sample from skin, muscle, buccal or conjunctival mucosa, placenta, gastrointestinal tract or other organs.

The term "schizophrenia therapeutic agent" refers to an agent that can be used to ameliorate or prevent symptoms associated with schizophrenia.

The term "schizophrenia-associated nucleic acid", as described herein, refers to a nucleic acid that has been found to be associated to schizophrenia. This includes, but is not limited to, the markers and haplotypes described herein and markers and haplotypes in strong linkage disequilibrium (LD) therewith.

The term "low copy repeat", or "LCR", as described herein, refers to chromosomal segments that are present in multiple (two or more) copies within a short interval. The repeated segment is usually a relatively short segment of 10-100,000 nucleotides, and is typically present in few copies, typically less than 10 copies. Some CNVs, including some of those described herein, are flanked by LCR regions.

The term "schizophrenia condition", as described herein, refers to the spectrum of mental disorders that includes schizophrenia and related psychotic disorders. The term is meant to include in particular schizophrenia, schizophreniform disorder, schizoaffective disorder, delusional disorder, brief psychotic disorder and brief psychotic disorder, as defined in the Diagnostic and Statistical Manual of Mental Disorder, fourth edition (DSM-IV-TR).

The present inventors have detected certain copy number variations (CNVs) in the human genome that confer risk of schizophrenia. The copy number variations were defined based on analysis of SNP genotypes obtained using the HumanHap317 chip (Illumina), as explained in more detail in Examples 1 - 3 herein.

The nature of the CNVs described herein is such that certain regions of the human genome is present in alternate copy number in certain individuals. The segment may be deleted, or it may be present in more than one copy on each particular chromosome. The segments are in general quite large, ranging from a few thousand nucleotides to over one million nucleotides in size. The absolute breakpoint at which the variation begins and ends can be difficult to define due to experimental limitations. Experimentally, what is determined is the last polymorphism (or probe) that is outside the CNV segment upstream (5') of the segment, the first polymorphism within the CNV segment, the last polymorphism within the CNV segment and the first polymorphism outside the CNV segment downstream (3') of the segment. Normally, the first two markers and the last two markers (or nucleotide probes) in the above will be adjacent markers. Thus the resulting CNV can be defined minimally as including the segment that begins with the first marker consistent with the CNV and ends with the last marker consistent with the CNV. Such a definition is however not inclusive of the physical boundaries of the CNV segment. An alternative way of defining the CNV is provided by the region flanked by the two polymorphisms that are inconsistent with the CNV (*i.e*. outside the CNV segment), but adjacent to the two polymorphisms corresponding to the first and last polymorphisms assayed within the CNV segment. The latter definition is inclusive of the actual boundaries of the CNV.

The following CNV table provides such definitions of the copy number variations described herein.

**CNV Table**

| Copy number variation | First upstream marker outside CNV | Position Build 36 | First marker within CNV | Position Build 36 | Last marker within CNV | Position Build 36 | First downstream marker outside CNV | Position Build 36 |
|---|---|---|---|---|---|---|---|---|
| 1q21.1 deletion short form | rs1284300 SEQ ID NO:1 | 144458820 | rs6656361 SEQ ID NO:2 | 144943150 | rs2932454 SEQ ID NO:3 | 146293282 | rs11587304 SEQ ID NO:4 | 147414362 |
| 1q21.1 deletion long form | rs11249395* SEQ ID NO:5 | 121013322* | rs10797649 SEQ ID NO:6 | 144106312 | rs2932454 SEQ ID NO:3 | 146293282 | rs11587304 SEQ ID NO:4 | 147414362 |
| 15q11.2 deletion | rs17728289 SEQ ID NO:7 | 19869474 | rs8040193 SEQ ID NO:8 | 20306549 | rs3883043 SEO ID NO:9 | 20777695 | rs4778531 SEQ ID NO:10 | 21240037 |
| 15q13.3 deletion | rs10152753 SEQ ID NO:11 | 28153539 | rs2046362 SEQ ID NO:12 | 28723577 | rs4779984 SEQ ID NO:13 | 30302218 | rs11635997 SEQ ID NO:14 | 30721385 |
| 16p13.1 duplication | rs8062460 SEQ ID NO: 15 | 14667269 | rs4985124 SEQ ID NO:16 | 15032942 | rs2547728 SEQ ID NO:17 | 18174650 | rs2641892 SEQ ID NO:18 | 18707116 |
| 5q35.2 duplication | rs4868651 SEQ ID NO:19 | 175921634 | rs1545976 SEQ ID NO:20 | 175939217 | rs2220368 SEQ ID NO:21 | 176073058 | rs10035561 SEQ ID NO:22 | 176081374 |

*The rs11249395 marker is on the p-side of the centromere on chromosome 1, which explains the large span of the region over which the 1q21.1 deletion could potentially stretch

As described herein, the 1q21.1 deletion, both long and short forms, the 15q11.2 deletion, the 15q13.3 deletion, the 16p13.1 duplication and the 5q35.2 duplication minimally span the regions between the first marker within the CNV and the last marker within the CNV, as described in the CNV table. Thus, the 1q21.1 deletion short form is defined as the region flanked by rs6656361 and rs2932454 (between position 144,943,150 and 146,293,282 on chr 1 of NCBI Build 36), the 1q21.1 deletion long form is defined as the region flanked by rs10797649 and rs2932454 (between position 144,106,312 and 146,293,282 on chr 1 of NCBI Build 36), the 15q11.2 deletion is defined as the region flanked by rs8040193 and rs3883043 (between position 20,306,549 and 20,777,695 on chr 15 of NCBI Build 36), the 15q13.3 deletion is defined as the region flanked by rs2046362 and rs4779984 (between position 28,723,577 and 30,302,218 on chr 15 of NCBI Build 36), the 16p13.1 duplication is defined as the region flanked by rs4985124 and rs2547728 (between position 15,032,942 and 18,174,650 on chr 16 of NCBI Build 36), and the 5q35.2 duplication is defined as the region flanked by rs1545976 and rs2220368 (between position 175,939,217 and 176,073,058 on chr 5 of NCBI Build 36).

However, it should be appreciated that the CNVs as defined may in fact stretch over a larger genomic region, and in fact are likely to do so, since the definitions as provided are confined to the markers that have been assessed. Thus, in an alternative fashion, the CNVs can be defined as maximially spanning a region that includes up to, but not including, the next marker assessed that is not consistent with the CNV. Therefore, in an alternative fashion, the 1q21.1 deletion, both long and short forms, the 15q11.2 deletion, the 15q13.3 deletion, the 16p13.1 duplication and the 5q35.2 duplication can be defined to span the regions between the first upstream marker outside the CNV and the first downstream marker outside the CNV, as further described in the CNV table above. Thus, the 1q21.1 deletion short form is in this context defined as the region flanked by rs1284300 and rs11587304, the 1q21.1 deletion long form is defined as the region flanked by rs11249395 and rs11587304, the 15q11.2 deletion is defined as the region flanked by rs11728289 and rs4778531, the 15q13.3 deletion the is defined as the region flanked by rs10152753 and rs11635997, 16p13.1 duplication is defined as the region flanked by rs8062460 and rs2641892, and the 5q35.2 duplication is defined as the region flanked by rs4868651 and rs10035561.

*Assessment for markers and haplotypes*

The genomic sequence within populations is not identical when individuals are compared. Rather, the genome exhibits sequence variability between individuals at many locations in the genome. Such variations in sequence are commonly referred to as polymorphisms, and there are many such sites within each genome. For example, the human genome exhibits sequence variations which occur on average every 500 base pairs. The most common sequence variant consists of base variations at a single base position in the genome, and such sequence variants, or polymorphisms, are commonly called Single Nucleotide Polymorphisms ("SNPs"). These SNPs are believed to have occurred in a single mutational event, and therefore there are usually two possible alleles possible at each SNPsite; the original allele and the mutated allele. Due to natural genetic drift and possibly also selective pressure, the original mutation has resulted in a polymorphism characterized by a particular frequency of its alleles in any given population. Many other types of sequence variants are found in the human genome, including mini- and microsatellites, and insertions, deletions and inversions (also called copy number variations (CNVs)). A polymorphic microsatellite has multiple small repeats of bases (such as CA repeats, TG on the complimentary strand) at a particular site in which the number of repeat lengths varies in the general population. In general terms, each version of the sequence with respect to the polymorphic site represents a specific allele of the polymorphic site. These sequence variants can all be referred to as polymorphisms, occurring at specific polymorphic sites characteristic of the sequence variant in question. In general terms, polymorphisms can comprise any number of specific alleles. Thus in one embodiment of the invention, the polymorphism is characterized by the presence of two or more alleles in any given population. In another embodiment, the polymorphism is characterized by the presence of three or more alleles. In other embodiments, the polymorphism is characterized by four or more alleles, five or more alleles, six or more alleles, seven or more alleles, nine or more alleles, or ten or more alleles. All such polymorphisms can be utilized in the methods of the present invention, and are thus within the scope of the invention.

Due to their abundance, SNPs account for a majority of sequence variation in the human genome. Over 6 million SNPs have been validated to date (http://www.ncbi.nlm.nih.gov/projects/SNP/snp_summary.cgi). However, CNVs are receiving increased attention. These large-scale polymorphisms (typically 1kb or larger) account for polymorphic variation affecting a substantial proportion of the assembled human genome; known CNVs covery over 15% of the human genome sequence (Estivill, X Armengol; L., Plos Genetics 3:1787-99 (2007); http://projects.tcag.ca/variation/). Most of these polymorphisms are however very rare, and on average affect only a fraction of the total genomic sequence of each individual. CNVs are known to affect gene expression, phenotypic variation and adaptation by disrupting gene dosage, and are also known to cause disease (microdeletion and microduplication disorders) and confer risk of common complex diseases, including HIV-1 infection and glomerulonephritis (Redon, R., et al. Nature 23:444-454 (2006)). Methods for detecting CNVs include comparative genomic hybridization (CGH) and genotyping, including use of genotyping arrays, as described by Carter (Nature Genetics 39:S16-S21 (2007)). The Database of Genomic Variants (http://projects.tcag.ca/variation/) contains updated information about the location, type and size of described CNVs. The database currently contains data for over 15,000 CNVs.

In some instances, reference is made to different alleles at a polymorphic site without choosing a reference allele. Alternatively, a reference sequence can be referred to for a particular polymorphic site. The reference allele is sometimes referred to as the "wild-type" allele and it usually is chosen as either the first sequenced allele or as the allele from a "non-affected" individual (*e.g.*, an individual that does not display a trait or disease phenotype).

Alleles for SNP markers as referred to herein refer to the bases A, C, G or T as they occur at the polymorphic site in the SNP assay employed. The allele codes for SNPs used herein are as follows: 1= A, 2=C, 3=G, 4=T. The person skilled in the art will however realise that by assaying or reading the opposite DNA strand, the complementary allele can in each case be measured. Thus, for a polymorphic site (polymorphic marker) characterized by an A/G polymorphism, the assay employed may be designed to specifically detect the presence of one or both of the two bases possible, i.e. A and G. Alternatively, by designing an assay that is designed to detect the complimentary strand on the DNA template, the presence of the complementary bases T and C can be measured. Quantitatively (for example, in terms of relative risk), identical results would be obtained from measurement of either DNA strand (+ strand or - strand).

Typically, a reference sequence is referred to for a particular sequence. Alleles that differ from the reference are sometimes referred to as "variant" alleles. A variant sequence, as used herein, refers to a sequence that differs from the reference sequence but is otherwise substantially similar. Alleles at the polymorphic genetic markers described herein are variants. Variants can include changes that affect a polypeptide. Sequence differences, when compared to a reference nucleotide sequence, can include the insertion or deletion of a single nucleotide, or of more than one nucleotide, resulting in a frame shift; the change of at least one nucleotide, resulting in a change in the encoded amino acid; the change of at least one nucleotide, resulting in the generation of a premature stop codon; the deletion of several nucleotides, resulting in a deletion of one or more amino acids encoded by the nucleotides; the insertion of one or several nucleotides, such as by unequal recombination or gene conversion, resulting in an interruption of the coding sequence of a reading frame; duplication of all or a part of a sequence; transposition; or a rearrangement of a nucleotide sequence,. Such sequence changes can alter the polypeptide encoded by the nucleic acid. For example, if the change in the nucleic acid sequence causes a frame shift, the frame shift can result in a change in the encoded amino acids, and/or can result in the generation of a premature stop codon, causing generation of a truncated polypeptide. Alternatively, a polymorphism associated with a disease or trait can be a synonymous change in one or more nucleotides (*i.e.*, a change that does not result in a change in the amino acid sequence). Such a polymorphism can, for example, alter splice sites, affect the stability or transport of mRNA, or otherwise affect the transcription or translation of an encoded polypeptide. It can also alter DNA to increase the possibility that structural changes, such as amplifications or deletions, occur at the somatic level. The polypeptide encoded by the reference nucleotide sequence is the "reference" polypeptide with a particular reference amino acid sequence, and polypeptides encoded by variant alleles are referred to as "variant" polypeptides with variant amino acid sequences.

A haplotype refers to a segment of DNA that is characterized by a specific combination of alleles arranged along the segment. For diploid organisms such as humans, a haplotype comprises one member of the pair of alleles for each polymorphic marker or locus. In a certain embodiment, the haplotype can comprise two or more alleles, three or more alleles, four or more alleles, or five or more alleles, each allele corresponding to a specific polymorphic marker along the segment. Haplotypes can comprise a combination of various polymorphic markers, e.g., SNPs and microsatellites, having particular alleles at the polymorphic sites. The haplotypes thus comprise a combination of alleles at various genetic markers.

Detecting specific polymorphic markers and/or haplotypes can be accomplished by methods known in the art for detecting sequences at polymorphic sites. For example, standard techniques for genotyping for the presence of SNPs and/or microsatellite markers can be used, such as fluorescence-based techniques (*e.g.*, Chen, X. et al., Genome Res. 9(5): 492-98 (1999); Kutyavin et al., Nucleic Acid Res. 34:e128 (2006)), utilizing PCR, LCR, Nested PCR and other techniques for nucleic acid amplification. Specific commercial methodologies available for SNP genotyping include, but are not limited to, TaqMan genotyping assays and SNPlex platforms (Applied Biosystems), gel electrophoresis (Applied Biosystems), mass spectrometry (e.g., MassARRAY system from Sequenom), minisequencing methods, real-time PCR, Bio-Plex system (BioRad), CEQ and SNPstream systems (Beckman), array hybridization technology(e.g., Affymetrix GeneChip; Perlegen), BeadArray Technologies (e.g., Illumina GoldenGate and Infinium assays), array tag technology (*e.g.*, Parallele), and endonuclease-based fuorescence hybridization technology (Invader; Third Wave). Some of the available array platforms, including Affymetrix SNP Array 6.0 and Illumina CNV370-Duo and 1M BeadChips, include SNPs that tag certain CNVs. This allows detection of CNVs via surrogate SNPs included in these platforms. Thus, by use of these or other methods available to the person skilled in the art, one or more alleles at polymorphic markers, including microsatellites, SNPs or other types of polymorphic markers, can be identified.

In the methods described herein, an individual at risk for a schizophrenia condition is one in whom a particular polymorphism, such as a copy number variation (CNV) is present. The copy number variation confers a particular risk of the condition; carriers of the CNV are at a different risk of the condition than non-carriers. In other words, the CNV is indicative of susceptibility or risk of the schizophrenia condition. In certain embodiments, significance associated with risk of a copy number variation is measured by a relative risk (RR). In another embodiment, significance associated with a copy number variation is measured by an odds ratio (OR). In a further embodiment, the significance is measured by a percentage. In one embodiment, a significant increased risk is measured as a risk (relative risk and/or odds ratio) of at least 1.2, including but not limited to: at least 1.5, at least 1.3, at least 1.4, at least 1.5, at least 1.6, at least 1.7, 1.8, at least 1.9, at least 2.0, at least 2.5, at least 3.0, at least 4.0, at least 5.0, at least 6.0, at least 7.0, at least 8.0, at least 9.0, at least 10.0, and at least 15.0. In a particular embodiment, a risk (relative risk and/or odds ratio) of at least 2.0 is significant. In another particular embodiment, a risk of at least 3.0 is significant. In yet another embodiment, a risk of at least 4.0 is significant. In a further embodiment, a relative risk of at least 5.0 is significant. In another further embodiment, a significant increase in risk is at least 10.0 is significant. However, other values for significant risk are also contemplated, e.g., at least 2.5, 3.5, 4.5, 5.5, or any suitable other numerical values, and such values are also within scope of the present invention. In other embodiments, a significant increase in risk is at least about 20%, including but not limited to about 25%, 30%, 35%, 40%, 45%, 50%, 55%, 60%, 65%, 70%, 75%, 80%, 85%, 90%, 95%, 100%, 150%, 200%, 300%, 400%, 500%, 600%, 700%, 800%, 900%, 1000%, and 1500%. In one particular embodiment, a significant increase in risk is at least 100%. In other embodiments, a significant increase in risk is at least 200%, at least 300%, at least 400%, at least 500%, at least 700%, at least 800%, at least 900% and at least 1000%. Other cutoffs or ranges as deemed suitable by the person skilled in the art to characterize the invention are however also contemplated, and those are also within scope of the present invention. In certain embodiments, a significant increase in risk is characterized by a p-value, such as a p-value of less than 0.05, less than 0.01, less than 0.001, less than 0.0001, less than 0.00001, less than 0.000001, less than 0.0000001, less than 0.00000001, or less than 0.000000001.

A copy number variation (CNV) predictive of risk of a schizphrenia condition, as described herein, is one where the particular CNV is more frequently present in an individual with the condition (affected), compared to the frequency of its presence in a comparison group (control), such that the presence of the CNV is indicative of susceptibility to the schizophrenia condition. The control group may in one embodiment be a population sample, i.e. a random sample from the general population. In another embodiment, the control group is represented by a group of individuals who are disease-free. Such disease-free control may in one embodiment be characterized by the absence of one or more specific disease-associated symptoms, e.g. individuals who have not experienced symptoms associated with schizophrenia. In another embodiment, the disease-free control group is characterized by the absence of one or more disease-specific risk factors. Such risk factors are in one embodiment at least one environmental risk factor. As an example of a simple test for correlation would be a Fisher-exact test on a two by two table. Given a cohort of chromosomes, the two by two table is constructed out of the number of chromosomes that include both of the markers or haplotypes, one of the markers or haplotypes but not the other and neither of the markers or haplotypes. Other statistical tests of association known to the skilled person are also contemplated and are also within scope of the invention.

In other embodiments of the invention, an individual who is at a decreased susceptibility (i.e., at a decreased risk) for a schizophrenia condition is an individual in whom at least one CNV, or one specific allele at one or more polymorphic marker or haplotype conferring decreased susceptibility for the disease or trait is identified. The marker alleles and/or haplotypes conferring decreased risk are also said to be protective. In one aspect, the protective marker or haplotype is one that confers a significant decreased risk (or susceptibility) of the disease or trait. In one embodiment, significant decreased risk is measured as a relative risk (or odds ratio) of less than 0.9, including but not limited to less than 0.9, less than 0.8, less than 0.7, less than 0.6, less than 0.5, less than 0.4, less than 0.3, less than 0.2 and less than 0.1. In one particular embodiment, significant decreased risk is less than 0.7. In another embodiment, significant decreased risk is less than 0.5. In yet another embodiment, significant decreased risk is less than 0.3. In another embodiment, the decrease in risk (or susceptibility) is at least 20%, including but not limited to at least 25%, at least 30%, at least 35%, at least 40%, at least 45%, at least 50%, at least 55%, at least 60%, at least 65%, at least 70%, at least 75%, at least 80%, at least 85%, at least 90%, at least 95% and at least 98%. In one particular embodiment, a significant decrease in risk is at least about 30%. In another embodiment, a significant decrease in risk is at least about 50%. In another embodiment, the decrease in risk is at least about 70%. Other cutoffs or ranges as deemed suitable by the person skilled in the art to characterize the invention are however also contemplated, and those are also within scope of the present invention.

The person skilled in the art will appreciate that for markers with two alleles present in the population being studied (such as SNPs), and wherein one allele is found in increased frequency in a group of individuals with a trait or disease in the population, compared with controls, the other allele of the marker will be found in decreased frequency in the group of individuals with the trait or disease, compared with controls. In such a case, one allele of the marker (the one found in increased frequency in individuals with the trait or disease) will be the at-risk allele, while the other allele will be a protective allele.

A genetic variant associated with a disease or a trait can be used alone to predict the risk of the disease for a given genotype. For a biallelic marker, such as a SNP, there are 3 possible genotypes: homozygote for the at risk variant, heterozygote, and non carrier of the at risk variant. Risk associated with variants at multiple loci can be used to estimate overall risk. For multiple SNP variants, there are *k* possible genotypes $k = 3^n \times 2^p$; where *n* is the number autosomal loci and p the number of gonosomal (sex chromosomal) loci. Overall risk assessment calculations usually assume that the relative risks of different genetic variants multiply, *i.e*. the overall risk (*e.g*., RR or OR) associated with a particular genotype combination is the product of the risk values for the genotype at each locus. If the risk presented is the relative risk for a person, or a specific genotype for a person, compared to a reference population with matched gender and ethnicity, then the combined risk - is the product of the locus specific risk values - and which also corresponds to an overall risk estimate compared with the population. If the risk for a person is based on a comparison to non-carriers of the at risk allele, then the combined risk corresponds to an estimate that compares the person with a given combination of genotypes at all loci to a group of Individuals who do not carry risk variants at any of those loci. The group of non-carriers of any at risk variant has the lowest estimated risk and has a combined risk compared with itself (i.e., non-carriers) of 1.0, but has an overall risk, compare with the population, of less than 1.0. It should be noted that the group of non-carriers can potentially be very small, especially for large number of loci, and in that case, its relevance is correspondingly small.

The multiplicative model is a parsimonious model that usually fits the data of complex traits reasonably well. Deviations from multiplicity have been rarely described in the context of common variants for common diseases, and if reported are usually only suggestive since very large sample sizes are usually required to be able to demonstrate statistical interactions between loci.

By way of an example, let us consider a total of eight variants that have been described to associate with prostate cancer (Gudmundsson, J., et al., Nat Genet 39:631-7 (2007), Gudmundsson, J., et al., Nat Genet 39:977-83 (2007); Yeager, M., et al, Nat Genet 39:645-49 (2007), Amundadottir, L., et al., Nat Genet 38:652-8 (2006); Haiman, C.A., et al., Nat Genet 39:638-44 (2007)). Seven of these loci are on autosomes, and the remaining locus is on chromosome X. The total number of theoretical genotypic combinations is then $3^7 \times 2^1 = 4374$. Some of those genotypic classes are very rare, but are still possible, and should be considered for overall risk assessment. It is likely that the multiplicative model applied in the case of multiple genetic variant will also be valid in conjugation with non-genetic risk variants assuming that the genetic variant does not clearly correlate with the "environmental" factor. In other words, genetic and non-genetic at-risk variants can be assessed under the multiplicative model to estimate combined risk, assuming that the non-genetic and genetic risk factors do not interact.

Using the same quantitative approach, the combined or overall risk associated with a plurality of variants associated with schizphrenia may be assessed. For example, the CNVs described herein to be associated with risk of schizophrenia may be combined with other common genetic risk factors. Combined risk for such genetic variants may be estimated in an analogous fashion to that described above.

*Assessment of Copy Number Variations*

*Detection of CNVs*

Detection of CNVs can be done by a range of techniques suitable for such purpose. In general, techniques that can selectively determine whether a particular chromosomal segment is present or absent in an individual can be used for genotyping CNVs. Ideally, the technique is able to quantify the amount of segment present, i.e. determine whether a segment is deleted, duplicated, triplicated, etc. in the individual. For example, Taqman assays can be used (Bieche, I. et al. Int J Cancer 78:661-6 (1998)), as well as Fluorescent In Situ Hybridization (FISH) techniques. A range of genotyping technologies can also be used, such as Molecular Inversion Probe array technology (*e.g.*, Affymetrix SNP Array 6.0), and BeadArray Technologies (*e.g.*, Illumina GoldenGate and Infinium assays, *e.g.* HumanHap chips, Human 1M-Duo), as can other platforms such as Nimblegen HD2.1, High-Definition CGH arrays (Agilent Technologies), tiling array technology (Affymetrix). Information about amplitude of particular probes, which is representative of particular alleles, provides a quantitative dosage information for the particular allele, and by consequence dosage information about the CNV in question, since the marker is selected as a marker representative of the CNV and is typically physcially located within the CNV. If the CNV is a deletion, then the absence of particular marker alleles is representative of the deletion. If the CNV is a duplication (or a higher order copy number variation), then the signal intensity representative of the allele correalating with the CNV is representative of the copy number. A summary of methodologies commonly used is provided in Perkel (Perkel. J Nature Methods 5:447-453 (2008)). Other suitable methods available to the skilled person can also be used, and are within scope of the present invention.

In certain embodiments of the methods of the invention, markers within the CNV are used to detect the presence or absence of the CNV. Markers selected from the group consisting of the markers set forth in Table 7, which are markers within the 15q11.2 deletion, are useful for determining a suscepbility to schizphrenia or a related condition. In such embodiments, determination of the presence of absence of a particular allele is indicative of the CNV, i.e. indicative of whether the CNV is prsesent in the individual or not.

*Haplotype Analysis*

One general approach to haplotype analysis involves using likelihood-based inference applied to NEsted MOdels (Gretarsdottir S., et al., Nat. Genet. 35:131-38 (2003)). The method is implemented in the program NEMO, which allows for many polymorphic markers, SNPs and microsatellites. The method and software are specifically designed for case-control studies where the purpose is to identify haplotype groups that confer different risks. It is also a tool for studying LD structures. In NEMO, maximum likelihood estimates, likelihood ratios and p-values are calculated directly, with the aid of the EM algorithm, for the observed data treating it as a missing-data problem.

Even though likelihood ratio tests based on likelihoods computed directly for the observed data, which have captured the information loss due to uncertainty in phase and missing genotypes, can be relied on to give valid p-values, it would still be of interest to know how much information had been lost due to the information being incomplete. The information measure for haplotype analysis is described in Nicolae and Kong (Technical Report 537, Department of Statistics, University of Statistics, University of Chicago; Biometrics, 60(2):368-75 (2004)) as a natural extension of information measures defined for linkage analysis, and is implemented in NEMO.

For single marker association to a disease, the Fisher exact test can be used to calculate two-sided p-values for each individual allele. Usually, all p-values are presented unadjusted for multiple comparisons unless specifically indicated. The presented frequencies (for microsatellites, SNPs and haplotypes) are allelic frequencies as opposed to carrier frequencies. To minimize any bias due the relatedness of the patients who were recruited as families to the study, first and second-degree relatives can be eliminated from the patient list. Furthermore, the test can be repeated for association correcting for any remaining relatedness among the patients, by extending a variance adjustment procedure previously described (Risch, N. & Teng, J. Genome Res., 8:1273-1288 (1998)) for sibships so that it can be applied to general familial relationships, and present both adjusted and unadjusted p-values for comparison. The method of genomic controls (Devlin, B. & Roeder, K. Biometrics 55:997 (1999)) can also be used to adjust for the relatedness of the individuals and possible stratification. The differences are in general very small as expected. To assess the significance of single-marker association corrected for multiple testing we can carry out a randomization test using the same genotype data. Cohorts of patients and controls can be randomized and the association analysis redone multiple times (*e.g.*, up to 500,000 times) and the p-value is the fraction of replications that produced a p-value for some marker allele that is lower than or equal to the p-value we observed using the original patient and control cohorts.

For both single-marker and haplotype analyses, relative risk (RR) and the population attributable risk (PAR) can be calculated assuming a multiplicative model (haplotype relative risk model) (Terwilliger, J.D. & Ott, J., Hum. Hered. 42:337-46 (1992) and Falk, C.T. & Rubinstein, P, Ann. Hum. Genet. 51 (Pt 3):227-33 (1987)), i.e., that the risks of the two alleles/haplotypes a person carries multiply. For example, if RR is the risk of A relative to a, then the risk of a person homozygote AA will be RR times that of a heterozygote Aa and $RR^2$ times that of a homozygote aa. The multiplicative model has a nice property that simplifies analysis and computations-haplotypes are independent, *i.e.*, in Hardy-Weinberg equilibrium, within the affected population as well as within the control population. As a consequence, haplotype counts of the affecteds and controls each have multinomial distributions, but with different haplotype frequencies under the alternative hypothesis. Specifically, for two haplotypes, $h_i$ and $h_j$, risk($h_i$)/risk($h_j$) = $(f_i/p_i)/(f_j/p_j)$, where $f$ and $p$ denote, respectively, frequencies in the affected population and in the control population. While there is some power loss if the true model is not multiplicative, the loss tends to be mild except for extreme cases. Most importantly, p-values are always valid since they are computed with respect to null hypothesis.

An association signal detected in one association study may be replicated in a second cohort, ideally from a different population (*e.g.*, different region of same country, or a different country) of the same or different ethnicity. The advantage of replication studies is that the number of tests performed in the replication study, and hence the less stringent the statistical measure that is applied. Replication studies in one or even several additional case-control cohorts have the added advantage of providing assessment of the association signal in additional populations, thus simultaneously confirming the initial finding and providing an assessment of the overall significance of the genetic variant(s) being tested in human populations in general.

The results from several case-control cohorts can also be combined to provide an overall assessment of the underlying effect. The methodology commonly used to combine results from multiple genetic association studies is the Mantel-Haenszel model (Mantel and Haenszel, J Natl Cancer Inst 22:719-48 (1959)). The model is designed to deal with the situation where association results from different populations, with each possibly having a different population frequency of the genetic variant, are combined. The model combines the results assuming that the effect of the variant on the risk of the disease, a measured by the OR or RR, is the same in all populations, while the frequency of the variant may differ between the poplations. Combining the results from several populations has the added advantage that the overall power to detect a real underlying association signal is increased, due to the increased statistical power provided by the combined cohorts. Furthermore, any deficiencies in individual studies, for example due to unequal matching of cases and controls or population stratification will tend to balance out when results from multiple cohorts are combined, again providing a better estimate of the true underlying genetic effect.

*Risk assessment and Diagnostics*

Within any given population, there is an absolute risk of developing a disease or trait, defined as the chance of a person developing the specific disease or trait over a specified time-period. For example, a woman's lifetime absolute risk of breast cancer is one in nine. That is to say, one woman in every nine will develop breast cancer at some point in their lives. Risk is typically measured by looking at very large numbers of people, rather than at a particular individual. Risk is often presented in terms of Absolute Risk (AR) and Relative Risk (RR). Relative Risk is used to compare risks associating with two variants or the risks of two different groups of people. For example, it can be used to compare a group of people with a certain genotype with another group having a different genotype. For a disease, a relative risk of 2 means that one group has twice the chance of developing a disease as the other group. The Risk presented is usually the relative risk for a person, or a specific genotype of a person, compared to the population with matched gender and ethnicity. Risks of two individuals of the same gender and ethnicity could be compared in a simple manner. For example, if, compared to the population, the first individual has relative risk 1.5 and the second has relative risk 0.5, then the risk of the first individual compared to the second individual is 1.5/0.5 = 3.

As described herein, certain copy number variations (CNVs) are found to be useful for risk assessment of schizophrenia. Risk assessment can involve detecting particular CNVs in the genome of individuals undergoing assessment. Particular CNVs are found more frequently in individuals with schizophrenia, than in individuals without diagnosis of schizophrenia. Therefore, these CNVs have predictive value for detecting schizophrenia, or a susceptibility to schizophrenia, in an individual. Tagging markers in linkage disequilibrium with the CNVs can be used as surrogates for the CNVs and can thus be used for risk assessment. Markers with values of $r^2$ equal to 1 are perfect surrogates for the at-risk CNVs, i.e. genotypes for the surrogate marker perfectly predicts the occurrence of the CNV. Markers with smaller values of $r^2$ than 1 can also be surrogates for the CNV, but the detected risk will tend to be lower than for the CNV itself, unless the risk conferred by the marker, or another genetic variant in LD with the marker, is higher than for the CNV. Without intending to be limited by theory, it is belived that the CNVs described herein to be associated with risk of schizophrenia represent functional variants predisposing to the disease. Alternatively, the CNVs are in linkage disequilibrium with a functional variant that is functionally causing the increased risk. In certain embodiments, the functional variant, in LD with the CNV, resides within the segment that defines the CNV, i.e. within the CNV itself. In other embodiments, the functional variant is in LD with the CNV, while physically located outside the CNV region. The functional variant may for example be a tandem repeat, such as a minisatellite or a microsatellite, a single base polymorphism (SNP), or a transposable element (*e.g.*, an *Alu* element). The assessment of such surrogate markers for the CNVs is disclosed herein. Such markers are annotated, mapped and listed in public databases, as well known to the skilled person, or can alternatively be readily identified by sequencing the region or a part of the region identified by the markers disclosed herein in a group of individuals, and identify polymorphisms in the resulting group of sequences. As a consequence, the person skilled in the art can readily and without undue experimentation genotype surrogate markers in linkage disequilibrium with the markers and CNVs described herein. The tagging or surrogate markers in LD with the at-risk CNVs also have predictive value for detecting association to schizophrenia, or a susceptibility to schizophrenia, in an individual.

The present invention can in certain embodiments be practiced by assessing a sample comprising genomic DNA from an individual for the presence of the chromosome 15q11.2 deletion. Such assessment includes steps of detecting the presence or absence of the particular CNV, using methods well known to the skilled person and further described herein, and based on the outcome of such assessment, determine whether the individual from whom the sample is derived is at increased or decreased risk (increased or decreased susceptibility) of schizophrenia. The presence of the CNV conferring increased risk of schizophrenia is indicative of the individual being at increased risk (increased susceptbility) to schizophrenia. The absence of the CNV conferring increased risk of schizophrenia in the individual is indicative of the individual not being at increased risk of schizophrenia conferred by the CNV assessed. Alternatively, the invention can be practiced utilizing a dataset comprising information about the genotype status of the CNV for at least one individual. In other words, a dataset containing information about such genetic status, for example in the form of genotype counts at certain polymorphic markers, an indication about the presence or absence of the particular CNV, an quantitative assessment of the CNV (such as number of copies of a particular region in the genome of the individual), or actual genotypes for one or more markers, can be queried for the presence or absence of the chromosome 15q11.2 deletion. A positive result for the CNV, as shown herein, is indicative of the individual from which the dataset is derived is at increased susceptibility (increased risk) of schizophrenia. A negative result, is indicative of the individual not having the elevated susceptibility (elevated risk) by the CNV. The dataset can in certain embodiments be comprised in a database containing genotype/CNV data for at least one individual.

In certain embodiments of the invention, a polymorphic marker is correlated to schizophrenia by referencing CNV data to a look-up table that comprises correlations between the CNV and schizophrenia. The CNV in certain embodiments comprises at least one indication of the CNV, i.e. an indication of the presence or absence of the CNV, or a quantitative numerical value representative of the CNV. In some embodiments, the table comprises a correlation for one CNV. In other embodiments, the table comprises a correlation for a plurality of CNVs. In both scenarios, by referencing to a look-up table that gives an indication of a correlation between a CNV and schizophrenia, a risk for schizophrenia, or a susceptibility to schizophrenia, can be identified in the individual from whom the sample is derived.

In some embodiments, the correlation is reported as a statistical measure. The statistical measure may be reported as a risk measure, such as a relative risk (RR), an absolute risk (AR) or an odds ratio (OR).

In general, the CNVs of described herein as conferring risk of schizophrenia, can be useful for risk assessment and diagnostic purposes for schizophrenia, either alone or in combination. The risk conferred by particular CNVs is quite high, and their frequency in the population quite low (see e.g. Tables 4 and 12). As a consequence, if the occurrence of multiple CNVs is independent, the likelihood of more than one CNV being present in one particular individual is correspondingly. Nevertheless, risk assessment for multiple CNVs can be performed using standard methodology. For example, if the CNVs are independent, their risk is expected to roughly multiply in carriers with more than one CNV present.

Likewise, the CNVs described herein can form the basis of risk analysis that combines other CNVs known to increase risk of schizophrenia, or other genetic risk variants (such as SNPs) for schizophrenia. Appropriate models, such as the multiplicative model, can be used for estimating overall risk.

Thus, in certain embodiments of the invention, a plurality of variants (CNVs, genetic markers, and/or haplotypes) is used for overall risk assessment. These variants are in one embodiment selected from the CNVs as disclosed herein. Other embodiments include the use of the variants of the present invention in combination with other variants known to be useful for diagnosing a susceptibility to schizophrenia. In such embodiments, the genotype status of a plurality of CNVs, markers and/or haplotypes is determined in an individual, and the status of the individual compared with the population frequency of the associated variants, or the frequency of the variants in clinically healthy subjects, such as age-matched and sex-matched subjects. Methods known in the art, such as multivariate analyses or joint risk analyses, including the use of multiplicative model for overall risk assessment, may subsequently be used to determine the overall risk conferred based on the genotype status at the multiple loci. Assessment of risk based on such analysis may subsequently be used in the methods of the invention, as described herein.

*Study population*

In a general sense, the methods of the invention can be utilized from samples containing nucleic acid material (DNA or RNA) from any source and from any individual. In preferred embodiments, the individual is a human individual. The individual can be an adult, child, or fetus. The nucleic acid source may be any sample comprising nucleic acid material, including biological samples, or a sample comprising nucleic acid material derived therefrom. The present invention also provides for assessing CNVs, markers and/or haplotypes in individuals who are members of a target population. Such a target population is in one embodiment a population or group of individuals at risk of developing the disease, based on other genetic factors, biomarkers, biophysical parameters, family history of schizophrenia or related diseases, previous diagnosis or medical history, etc.

The invention provides for embodiments that include individuals from specific age subgroups, such as those over the age of 15, over the age of 20, over the age of 25, over the age of 30, over the age of 35, over the age of 40, over the age of 45, or over the age of 50, 55, 60, 65, 70, 75, 80, or 85. Other embodiments of the invention pertain to other age groups, such as individuals aged less than 85, such as less than age 80, less than age 75, or less than age 70, 65, 60, 55, 50, 45, 40, 35, 30, 25, 20, or 15. Other embodiments relate to individuals with age at onset of the disease in any of particular age or age ranges defined by the numerical values described in the above or other numerical values bridging these numbers. It is also contemplated that a range of ages may be relevant in certain embodiments, such as age at onset at more than age 15 but less than age 20. Other age ranges are however also contemplated, including all age ranges bracketed by the age values listed in the above. The invention furthermore relates to individuals of either gender, males or females.

The Icelandic population is a Caucasian population of Northern European ancestry. A large number of studies reporting results of genetic linkage and association in the Icelandic population have been published in the last few years. Many of those studies show replication of variants, originally identified in the Icelandic population as being associating with a particular disease, in other populations (Sulem, P., et al. Nat Genet May 17 2009 (Epub ahead of print); Rafnar, T., et al. Nat Genet 41:221-7 (2009); Gretarsdottir, S., et al. Ann Neurol 64:402-9 (2008); Stacey, S.N., et al. Nat Genet 40:1313-18 (2008); Gudbjartsson, D.F., et al. Nat Genet 40:886-91 (2008); Styrkarsdottir, U., et al. N Engl J Med 358:2355-65 (2008); Thorgeirsson, T., et al. Nature 452:638-42 (2008); Gudmundsson, J., et al. Nat Genet. 40:281-3 (2008); Stacey, S.N., et al., Nat Genet. 39:865-69 (2007); Helgadottir, A., et al., Science 316:1491-93 (2007); Steinthorsdottir, V., et al., Nat Genet. 39:770-75 (2007); Gudmundsson, J., et al., Nat Genet. 39:631-37 (2007); Frayling, TM, Nature Reviews Genet 8:657-662 (2007); Amundadottir, L.T., et al., Nat Genet. 38:652-58 (2006); Grant, S.F., et al., Nat Genet. 38:320-23 (2006)). Thus, genetic findings in the Icelandic population have in general been replicated in other populations, including populations from Africa and Asia.

The CNVs disclosed herein to be associated with schizophrenia are believed to show similar association in other human populations. Particular embodiments comprising individual human populations are thus also contemplated and within the scope of the invention. Such embodiments relate to human subjects that are from one or more human population including, but not limited to, Caucasian populations, European populations, American populations, Eurasian populations, Asian populations, Central/South Asian populations, East Asian populations, Middle Eastern populations, African populations, Hispanic populations, and Oceanian populations. European populations include, but are not limited to, Swedish, Norwegian, Finnish, Russian, Danish, Icelandic, Irish, Kelt, English, Scottish, Dutch, Belgian, French, German, Spanish, Portugues, Italian, Polish, Bulgarian, Slavic, Serbian, Bosnian, Czech, Greek and Turkish populations. In certain embodiments, the invention relates to individuals of Caucasian origin.

The racial contribution in individual subjects may also be determined by genetic analysis. Genetic analysis of ancestry may be carried out using unlinked microsatellite markers such as those set out in Smith et al. (Am J Hum Genet 74, 1001-13 (2004)).

In certain embodiments, the invention relates to CNVs, markers and/or haplotypes identified in specific populations, as described in the above. The person skilled in the art will appreciate that measures of linkage disequilibrium (LD) may give different results when applied to different populations. This is due to different population history of different human populations as well as differential selective pressures that may have led to differences in LD in specific genomic regions. It is also well known to the person skilled in the art that certain genetic markers, e.g. CNVs or SNP markers, have different population frequncy in different populations, or are polymorphic in one population but not in another. The person skilled in the art will however apply the methods available and as thought herein to practice the present invention in any given human population. Thus, the at-risk variants of the present invention may reside on different haplotype background and in different frequencies in various human populations. However, utilizing methods known in the art and the marker of the present invention, the invention can be practiced in any given human population.

*Utility of Genetic Testing*

The person skilled in the art will appreciate and understand that the CNV variants described herein in general do not, by themselves, provide an absolute identification of individuals who will develop schizophrenia or related conditions. The variants described herein do however indicate increased and/or decreased likelihood that individuals carrying the at-risk or protective variants disclosed herein will develop symptoms associated with schizophrenia. This information is however extremely valuable in itself, as outlined in more detail in the below, as it can be used to, for example, initiate preventive measures at an early stage, perform regular physical and/or mental exams to monitor the progress and/or appearance of symptoms, or to schedule exams at a regular interval to identify early symptoms, so as to be able to apply treatment at an early stage. This is in particular important since schizophrenia and related disorders are heterogeneous disorders with symptoms that are individually vague. Diagnostic criteria require a number of symptoms to be present over a period of time; therefore, it is important to be able to establish additional risk factors that may aid in the diagnosis, or facilitate the diagnosis through in-depth phenotyping and/or more frequent examination, or both.

Thus, the knowledge about a genetic variant that confers a risk of developing schizophrenia offers the opportunity to apply a genetic test to identify those individuals with particularly increased risk of developing schizophrenia or a related condition (i.e. carriers of the at-risk variant). The CNV variants described herein confer high risk of developing schizophrenia; for example, the chr 15 deletion confers an 11-fold increased risk of schizophrenia, compared with the general population. This a very significant effect, and is useful in a diagnostic setting. For example, individuals with early symptoms that typically are not individually associated with a clinical diagnosis of schizpophrenia and carry an at-risk CNV may benefit from early therapeutic treatment, or other preventive measure, or more rigorous supervision or more frequent examination. Likewise, individuals that have a family history of the disease, or are carriers of other risk factors associated with schizophrenia may, in the context of additionally carrying at least one at-risk CNV benefit from early therapy or other treatment. The core values of genetic testing lie in the possibilities of being able to diagnose schizophrenia, or a predisposition to schizophrenia, at an early stage and provide such information to the appropriate person - such as a clinician, the individual him/herself, a genetic counselor, or guardian, in order to be able to apply the most appropriate therapeutic measure at an early disease stage.

Early symptoms of behavioural disorders such as schizophrenia and related conditions are usually not sufficient to fulfill standardized diagnostic criteria; to fulfill those, a certain pattern of symptoms and behavioural disturbance needs to manifest itslef over a period of time. Sometimes, certain physical characteristics may also be present. This makes at-risk genetic variants valuable in a diagnostic setting, in particular high-risk variants. Determination of the presence of such variants warrants increased monitoring of the individual in question. Appearance of behavioural symptoms combined with the presence of such variants facilitates early diagnosis, which makes early treatment possible. Genetic testing may thus be used to aid in the diagnosis of disease in its early stages, before all criteria for formal diagnostic criteria are all fulfilled. It is well established that early treatment is extremely important for disorders on the schizophrenic spectrum, which lends further support to the value of genetic testing for early diagnosis of these disorders.

**METHODS**

Methods for risk assessment and risk management of schizophrenia are described herein.

*Diagnostic and screening methods*

In particular embodiments, the invention is a method of determining a susceptibility to schizophrenia by detecting the chromosome 15q11.2 deletion. In other embodiments, the invention relates to a method of determining or diagnosing a susceptibility to schizophrenia by detecting at least one allele of at least one polymorphic marker. The present invention describes methods whereby detection of particular alleles of particular markers or haplotypes is indicative of a susceptibility to schizophrenia. Such prognostic or predictive assays can also be used to determine prophylactic treatment of a subject prior to the onset of symptoms of schizophrenia. The present invention pertains in some embodiments to methods of clinical applications of diagnosis, e.g., diagnosis performed by a medical professional. In other embodiments, the invention pertains to methods of diagnosis or determination of a susceptibility performed by a layman. The layman can be the customer of a genotyping service. The layman may also be a genotype service provider, who performs genotype analysis on a DNA sample from an individual, in order to provide service related to genetic risk factors for particular traits or diseases, based on the genotype status of the individual (*i.e.*, the customer). Recent technological advances in genotyping technologies, including high-throughput genotyping of SNP markers, such as Molecular Inversion Probe array technology (e.g., Affymetrix GeneChip), and BeadArray Technologies (e.g., Illumina GoldenGate and Infinium assays) have made it possible for individuals to have their own genome assessed for up to one million SNPs simultaneously, at relatively little cost. As described herein, certain of the commonly available SNP genotyping platforms include SNPs that represent tags for particular copy number variations. These platforms therefore are suitable for the detection of particular CNVs in an individual. The resulting genotype information, which can be made available to the individual, can be compared to information about disease or trait risk associated with various CNVs, or alternatively surrogate SNPs for particular CNVs, including information from public litterature and scientific publications. The diagnostic application of disease-associated alleles as described herein, can thus for example be performed by the individual, through analysis of his/her genotype data, by a health professional based on results of a clinical test, or by a third party, including the genotype service provider. The third party may also be service provider who interprets genotype information from the customer to provide service related to specific genetic risk factors, including the genetic markers described herein. In other words, the diagnosis or determination of a susceptibility of genetic risk can be made by health professionals, genetic counselors, third parties providing genotyping service, third parties providing risk assessment service or by the layman (*e.g.*, the individual), based on information about the genotype status of an individual and knowledge about the risk conferred by particular genetic risk factors (e.g., particular SNPs). The information derived from analyzing sequence data, including assessment of particular SNPs and/or CNVs can be communicated to any particular body, including the individual from which the sample, or sequence data, is derived, a guardian or representative of the individual, a clinician, a service provider, including a genotyping service provider, and a medical insurerer or insurance company. In the present context, the term "diagnosing", "diagnose a susceptibility" and "determine a susceptibility" is meant to refer to any available diagnostic method, including those mentioned above.

In certain embodiments, a sample containing genomic DNA from an individual is collected. Such sample can for example be a buccal swab, a saliva sample, a blood sample, or other suitable samples containing genomic DNA, as described further herein. The genomic DNA is then analyzed using any common technique available to the skilled person, such as high-throughput array technologies that can also include CNV-specific probes or SNPs. Results from such genotyping are stored in a convenient data storage unit, such as a data carrier, including computer databases, data storage disks, or by other convenient data storage means. In certain embodiments, the computer database is an object database, a relational database or a post-relational database. The genotype data is subsequently analyzed for the presence of certain variants known to be susceptibility variants for particular human conditions, such as the genetic variants (CNVs and/or their surrogates) described herein. Genotype data can be retrieved from the data storage unit using any convenient data query method. Calculating risk conferred by a particular genotype for the individual can be based on comparing the genotype of the individual to previously determined risk (expressed as a relative risk (RR) or and odds ratio (OR), for example) for the genotype, for example for a heterozygous carrier of an at-risk variant for schizophrenia. The calculated risk for the individual can be the relative risk for a person, or for a specific genotype of a person, compared to the average population with matched gender and ethnicity. The average population risk can be expressed as a weighted average of the risks of different genotypes, using results from a reference population, and the appropriate calculations to calculate the risk of a genotype group relative to the population can then be performed. Alternatively, the risk for an individual is based on a comparison of particular genotypes, for example heterozygous carriers of an at-risk allele of a marker compared with non-carriers of the at-risk allele. Using the population average may in certain embodiments be more convenient, since it provides a measure which is easy to interpret for the user, i.e. a measure that gives the risk for the individual, based on his/her genotype, compared with the average in the population. The calculated risk estimated can be made available to the customer via a website, preferably a secure website.

In certain embodiments, a service provider will include in the provided service all of the steps of isolating genomic DNA from a sample provided by the customer, performing genotyping of the isolated DNA, calculating genetic risk based on the genotype data, and report the risk to the customer. In some other embodiments, the service provider will include in the service the interpretation of genotype data for the individual, *i.e.*, risk estimates for particular genetic variants based on the genotype data for the individual. In some other embodiments, the service provider may include service that includes genotyping service and interpretation of the genotype data, starting from a sample of isolated DNA from the individual (the customer).

Overall risk for multiple risk variants can be performed using standard methodology. For example, assuming a multiplicative model, *i.e.* assuming that the risk of individual risk variants multiply to establish the overall effect, allows for a straight-forward calculation of the overall risk for multiple markers.

As described and exemplified herein, particular CNVs are associated with schizophrenia, in particular the chromosome 15q11.2 deletion. In one embodiment, the CNV is one that confers a significant risk or susceptibility to schizophrenia. In another embodiment, the invention relates to a method of diagnosing a susceptibility to schizophrenia in a human individual, the method comprising determining the presence or absence of the chromosome 15q11.2 deletion in a nucleic acid sample obtained from the individual. In another embodiment, the invention pertains to methods of diagnosing a susceptibility to schizophrenia in a human individual, by screening for the chromosome 15q11.2 deletion. In another embodiment, the CNV is more frequently present in a subject having, or who is susceptible to, schizophrenia (affected), as compared to the frequency of its presence in a healthy subject (control, such as population controls). In certain embodiments, the significance of association of the at least one marker allele or haplotype is characterized by a p value $< 0.05$. In other embodiments, the significance of association is characterized by smaller p-values, such as $< 0.01$, $<0.001$, $<0.0001$, $<0.00001$, $<0.000001$, $<0.0000001$, $<0.00000001$ or $<0.000000001$.

In these embodiments, the presence of the at least one CNV is indicative of increased susceptibility to schizophrenia. Detecting the presence or absence of the chromosome 15q11.2 deletion provides information about whether the particular susceptibility conferred by the CNV is present in the indiviudal. The presence of particular CNVs, or markers in LD with the CNVs, can be detected at the nucleic acid level (e.g., by direct nucleotide sequencing or by other means known to the skilled in the art) in a sample from the individual. The presence can also be determined by investigating a dataset or a database comprising information about the sequence of the individual with respect to the CNVs, or markers in LD with any one of the CNVs.

In certain embodiments, diagnosis susceptibility can be accomplished using hybridization methods. (see Current Protocols in Molecular Biology, Ausubel, F. et al., eds., John Wiley & Sons, including all supplements). The presence of a specific marker allele or a particular genomic segment comprising a CNV, or representative of a CNV, can be indicated by sequence-specific hybridization of a nucleic acid probe specific for the particular allele or the CNV. The presence of more than one specific marker allele or several CNVs can be indicated by using several sequence-specific nucleic acid probes, each being specific for a particular allele and/or CNV. A sequence-specific probe can be directed to hybridize to genomic DNA, RNA, or cDNA. A "nucleic acid probe", as used herein, can be a DNA probe or an RNA probe that hybridizes to a complementary sequence. One of skill in the art would know how to design such a probe so that sequence specific hybridization will occur only if a particular allele is present in a genomic sequence from a test sample. The invention can also be reduced to practice using any convenient genotyping method, including commercially available technologies and methods for genotyping particular polymorphic markers.

Susceptibility to schizophrenia can be determined by a hybridization sample can be formed by contacting the test sample containing schizophrenia-associated nucleic acid, such as a genomic DNA sample, with at least one nucleic acid probe. A non-limiting example of a probe for detecting mRNA or genomic DNA is a labeled nucleic acid probe that is capable of hybridizing to mRNA or genomic DNA sequences described herein. The nucleic acid probe can be, for example, a full-length nucleic acid molecule, or a portion thereof, such as an oligonucleotide of at least 15, 30, 50, 100, 250 or 500 nucleotides in length that is sufficient to specifically hybridize under stringent conditions to appropriate mRNA or genomic DNA. For example, the nucleic acid probe can comprise all or a portion of the nucleotide sequence of chromosomal segments comprising the chromosome 15q11.2 deletion, as described herein, or the probe can be the complementary sequence of such a sequence. Other suitable probes for use in the diagnostic assays of the invention are described herein. Hybridization can be performed by methods well known to the person skilled in the art (see, e.g., Current Protocols in Molecular Biology, Ausubel, F. et al., eds., John Wiley & Sons, including all supplements). In one embodiment, hybridization refers to specific hybridization, i.e., hybridization with no mismatches (exact hybridization). In one embodiment, the hybridization conditions for specific hybridization are high stringency.

Specific hybridization, if present, is detected using standard methods. If specific hybridization occurs between the nucleic acid probe and the nucleic acid in the test sample, then the sample contains the sequence that is complementary to the nucleotide that is present in the nucleic acid probe. If the nucleic acid probe contains a particular allele of a polymorphic marker, or particular alleles for a plurality of markers, then specific hybridization is indicative of the nucleic acid being completely complementary to the nucleic acid probe, including the particular alleles at polymorphic markers within the probe. It is also possible to design a single probe containing more than one marker alleles of a particular haplotype (e.g., a probe containing alleles complementary to 2, 3, 4, 5 or all of the markers that make up a particular haplotype). Detection of the particular markers of the haplotype in the sample is indicative that the source of the sample has the particular haplotype (e.g., a haplotype).

In one preferred embodiment of allele-specific hybridization, a method utilizing a detection oligonucleotide probe comprising a fluorescent moiety or group at its 3' terminus and a quencher at its 5' terminus, and an enhancer oligonucleotide, is employed, as described by Kutyavin et al. (Nucleic Acid Res. 34:e128 (2006)). The fluorescent moiety can be Gig Harbor Green or Yakima Yellow, or other suitable fluorescent moieties. The detection probe is designed to hybridize to a short nucleotide sequence that includes the SNP polymorphism to be detected. Preferably, the SNP is anywhere from the terminal residue to -6 residues from the 3' end of the detection probe. The enhancer is a short oligonucleotide probe which hybridizes to the DNA template 3' relative to the detection probe. The probes are designed such that a single nucleotide gap exists between the detection probe and the enhancer nucleotide probe when both are bound to the template. The gap creates a synthetic abasic site that is recognized by an endonuclease, such as Endonuclease IV. The enzyme cleaves the dye off the fully complementary detection probe, but cannot cleave a detection probe containing a mismatch. Thus, by measuring the fluorescence of the released fluorescent moiety, assessment of the presence of a particular allele defined by nucleotide sequence of the detection probe can be performed.

The detection probe can be of any suitable size, although preferably the probe is relatively short. In one embodiment, the probe is from 5-100 nucleotides in length. In another embodiment, the probe is from 10-50 nucleotides in length, and in another embodiment, the probe is from 12-30 nucleotides in length. Other lengths of the probe are possible and within scope of the skill of the average person skilled in the art.

In a preferred embodiment, the DNA template containing the SNP polymorphism is amplified by Polymerase Chain Reaction (PCR) prior to detection. In such an embodiment, the amplified DNA serves as the template for the detection probe and the enhancer probe.

Certain embodiments of the detection probe, the enhancer probe, and/or the primers used for amplification of the template by PCR include the use of modified bases, including modified A and modified G. The use of modified bases can be useful for adjusting the melting temperature of the nucleotide molecule (probe and/or primer) to the template DNA, for example for increasing the melting temperature in regions containing a low percentage of G or C bases, in which modified A with the capability of forming three hydrogen bonds to its complementary T can be used, or for decreasing the melting temperature in regions containing a high percentage of G or C bases, for example by using modified G bases that form only two hydrogen bonds to their complementary C base in a double stranded DNA molecule. In a preferred embodiment, modified bases are used in the design of the detection nucleotide probe. Any modified base known to the skilled person can be selected in these methods, and the selection of suitable bases is well within the scope of the skilled person based on the teachings herein and known bases available from commercial sources as known to the skilled person.

Additionally, or alternatively, a peptide nucleic acid (PNA) probe can be used in addition to, or instead of, a nucleic acid probe in the hybridization methods described herein. A PNA is a DNA mimic having a peptide-like, inorganic backbone, such as N-(2-aminoethyl)glycine units, with an organic base (A, G, C, T or U) attached to the glycine nitrogen via a methylene carbonyl linker (see, for example, Nielsen, P., et al., Bioconjug. Chem. 5:3-7 (1994)). The PNA probe can be designed to specifically hybridize to a molecule in a sample suspected of containing one or more of the marker alleles or haplotypes that are associated with schizophrenia. Hybridization of the PNA probe is thus diagnostic for a susceptibility to schizophrenia.

In one embodiment of the invention, a test sample containing genomic DNA obtained from the subject is collected and the polymerase chain reaction (PCR) is used to amplify a fragment of nucleic acid that comprisesone ore more polymorphic marker that is indicative of a susceptibility to schizophrenia. As described herein, identification of a particular marker alleles can be accomplished using a variety of methods (e.g., sequence analysis, analysis by restriction digestion, specific hybridization, single stranded conformation polymorphism assays (SSCP), electrophoretic analysis, etc.). In another embodiment, diagnosis is accomplished by expression analysis, for example by using quantitative PCR (kinetic thermal cycling). This technique can, for example, utilize commercially available technologies, such as TaqMan® (Applied Biosystems, Foster City, CA). The technique can assess the presence of an alteration in the expression or composition of a polypeptide or splicing variant(s) that is encoded by a nucleic acid associated with schizophrenia. Further, the expression of the variant(s) can be quantified as physically or functionally different.

In another embodiment of the methods of the invention, analysis by restriction digestion can be used to detect a particular allele if the allele results in the creation or elimination of a restriction site relative to a reference sequence. Restriction fragment length polymorphism (RFLP) analysis can be conducted, e.g., as described in Current Protocols in Molecular Biology, *supra.* The digestion pattern of the relevant DNA fragment indicates the presence or absence of the particular allele in the sample.

Sequence analysis can also be used to detect specific alleles or haplotypes associated. Therefore, in one embodiment, determination of the presence or absence of a particular marker alleles or haplotypes comprises sequence analysis of a test sample of DNA or RNA obtained from a subject or individual. PCR or other appropriate methods can be used to amplify a portion of a nucleic acid comprising at least one polymorphic marker, and the presence of a specific allele can then be detected directly by sequencing the polymorphic site (or multiple polymorphic sites in a haplotype) of the genomic DNA in the sample.

In another embodiment, arrays of oligonucleotide probes that are complementary to target nucleic acid sequence segments from a subject, can be used to identify polymorphisms in a nucleic acid. For example, an oligonucleotide array can be used. Oligonucleotide arrays typically comprise a plurality of different oligonucleotide probes that are coupled to a surface of a substrate in different known locations. These arrays can generally be produced using mechanical synthesis methods or light directed synthesis methods that incorporate a combination of photolithographic methods and solid phase oligonucleotide synthesis methods, or by other methods known to the person skilled in the art (see, *e.g.*, Bier, F.F., et al. Adv Biochem Eng Biotechnol 109:433-53 (2008); Hoheisel, J.D., Nat Rev Genet 7:200-10 (2006); Fan, J.B., et al. Methods Enzymol 410:57-73 (2006); Raqoussis, J. & Elvidge, G., Expert Rev Mol Diagn 6:145-52 (2006); Mockler, T.C., et al Genomics 85:1-15 (2005), and references cited therein). Many additional descriptions of the preparation and use of oligonucleotide arrays for detection of polymorphisms can be found, for example, in US 6,858,394, US 6,429,027, US 5,445,934, US 5,700,637, US 5,744,305, US 5,945,334, US 6,054,270, US 6,300,063, US 6,733,977, US 7,364,858, EP 619 321, and EP 373 203.

Other methods of nucleic acid analysis that are available to those skilled in the art can be used to detect a particular allele at a polymorphic site. Representative methods include, for example, direct manual sequencing (Church and Gilbert, Proc. Natl. Acad. Sci. USA, 81: 1991-1995 (1988); Sanger, F., et al., Proc. Natl. Acad. Sci. USA, 74:5463-5467 (1977); Beavis, et al., U.S. Patent No. 5,288,644); automated fluorescent sequencing; single-stranded conformation polymorphism assays (SSCP); clamped denaturing gel electrophoresis (CDGE); denaturing gradient gel electrophoresis (DGGE) (Sheffield, V., et al., Proc. Natl. Acad. Sci. USA, 86:232-236 (1989)), mobility shift analysis (Orita, M., et al., Proc. Natl. Acad. Sci. USA, 86:2766-2770 (1989)), restriction enzyme analysis (Flavell, R., et al., Cell, 15:25-41 (1978); Geever, R., et al., Proc. Natl. Acad. Sci. USA, 78:5081-5085 (1981)); heteroduplex analysis; chemical mismatch cleavage (CMC) (Cotton, R., et al., Proc. Natl. Acad. Sci. USA, 85:4397-4401 (1985)); RNase protection assays (Myers, R., et a/., Science, 230:1242-1246 (1985); use of polypeptides that recognize nudeotide mismatches, such as *E. coli* mutS protein; and allele-specific PCR.

In another embodiment of the invention, diagnosis of schizophrenia can be made by examining expression and/or composition of a polypeptide encoded by a nucleic acid associated with schizophrenia in those instances where the copy number variation of the present invention results in a change in the composition or expression of the polypeptide. Thus, diagnosis of a susceptibility to schizophrenia can be made by examining expression and/or composition of one of these polypeptides, or another polypeptide encoded by a nucleic acid associated with schizophrenia, in those instances where the genetic marker or haplotype of the present invention results in a change in the composition or expression of the polypeptide. The CNVs described herein that show association to schizophrenia may play a role through their effect on one or more of these nearby genes. For example, while not intending to be limted by theory, it is generally expected that a deletion of a chromosomal segment comprising a particular gene, or a fragment of a gene, will either result in a altered composition or expression, or both, of the encoded protein. Likewise, duplications (or high number copy number variations, such as triplications, etc.) are in general expected to result in increased expression of encoded polypeptide. Other possible mechanisms affecting genes within CNV region include, e.g., effects on transcription, effects on RNA splicing, alterations in relative amounts of alternative splice forms of mRNA, effects on RNA stability, effects on transport from the nucleus to cytoplasm, and effects on the efficiency and accuracy of translation.

Thus, in another embodiment, the CNV variant (or tagging markers or haplotypes) of the invention showing association to schizophrenia affects the expression of a gene within the CNV region, or a gene in LD with the CNV region. Certain CNV regions have flanking duplicated segments, and genes within such segments can have altered expression and/or composition as a result of such genomic alterations. It is also well known that regulatory element affecting gene expression may be located far away, even as far as tenths or hundreds of kilobases away, from the promoter region of a gene. Thus, regulatory elements for genes that are located outside the CNV region may be located within the CNV, and thus be affected by the copy number variation. It is thus contemplated that the detection of the CNVs described herein, or markers or haplotypes in LD with any one of those CNVs, can be used for assessing expression for one or more of associated genes.

A variety of methods can be used for detecting protein expression levels, including enzyme linked immunosorbent assays (ELISA), Western blots, immunoprecipitations and immunofluorescence. A test sample from a subject is assessed for the presence of an alteration in the expression and/or an alteration in composition of the polypeptide encoded by a nucleic acid associated with schizophrenia. Such alteration can, for example, be an alteration in the quantitative polypeptide expression (i.e., the amount of polypeptide produced). An alteration in the composition of a polypeptide can be an alteration in the qualitative polypeptide expression (e.g., expression of a mutant polypeptide or of a different splicing variant). In one embodiment, diagnosis of a susceptibility to schizophrenia is made by detecting a particular splicing variant encoded by a nucleic acid associated with schizophrenia, or a particular pattern of splicing variants.

Both such alterations (quantitative and qualitative) can also be present. An "alteration" in the polypeptide expression or composition, as used herein, refers to an alteration in expression or composition in a test sample, as compared to the expression or composition of the polypeptide in a control sample. A control sample is a sample that corresponds to the test sample (e.g., is from the same type of cells), and is from a subject who is not affected by, and/or who does not have a susceptibility to, or who has not been diagnosed with schizophrenia. In one embodiment, the control sample is from a subject that does not have a particular CNV (or a marker allele or haplotype in LD therewith) associated with schizophrenia, as described herein. Similarly, the presence of one or more different splicing variants in the test sample, or the presence of significantly different amounts of different splicing variants in the test sample, as compared with the control sample, can be indicative of a susceptibility to schizophrenia. An alteration in the expression or composition of the polypeptide in the test sample, as compared with the control sample, can be the result of a particular CNV. Various means of examining expression or composition of a polypeptide encoded by a nucleic acid are known to the person skilled in the art and can be used, including spectroscopy, colorimetry, electrophoresis, isoelectric focusing, and immunoassays (e.g., David et al., U.S. Pat. No. 4,376,110) such as immunoblotting (see, e.g., Current Protocols in Molecular Biology, particularly chapter 10, *supra).*

For example, in one embodiment, an antibody (e.g., an antibody with a detectable label) that is capable of binding to a particular target polypeptide (*e.g*., a polypeptide encoded by a nucleic acid associated with a CNV as described herein) can be used. Antibodies can be polyclonal or monoclonal. An intact antibody, or a fragment thereof (e.g., Fv, Fab, Fab', $F(ab')_2$) can be used. The term "labeled", with regard to the probe or antibody, is intended to encompass direct labeling of the probe or antibody by coupling (i.e., physically linking) a detectable substance to the probe or antibody, as well as indirect labeling of the probe or antibody by reactivity with another reagent that is directly labeled. Examples of indirect labeling include detection of a primary antibody using a labeled secondary antibody (e.g., a fluorescently-labeled secondary antibody) and end-labeling of a DNA probe with biotin such that it can be detected with fluorescently-labeled streptavidin.

In one embodiment of this method, the level or amount of polypeptide in a test sample is compared with the level or amount of the polypeptide in a control sample. A level or amount of the polypeptide in the test sample that is higher or lower than the level or amount of the polypeptide in the control sample, such that the difference is statistically significant, is indicative of an alteration in the expression of the polypeptide encoded by the nucleic acid, and is diagnostic for a particular allele or haplotype responsible for causing the difference in expression. Alternatively, the composition of the polypeptide in a test sample is compared with the composition of the polypeptide in a control sample. In another embodiment, both the level or amount and the composition of the polypeptide can be assessed in the test sample and in the control sample.

*Therapeutic agents*

The CVNs described herein can be used to identify novel therapeutic targets for schizophrenia. For example, genes containing, or in linkage disequilibrium with, the CNVs, or their products, as well as genes or their products that are directly or indirectly regulated by or interact with these variant genes or their products, can be targeted for the development of therapeutic agents to treat schizophrenia, or prevent or delay onset of symptoms associated with schizophrenia. Therapeutic agents may comprise one or more of, for example, small non-protein and non-nucleic acid molecules, proteins, peptides, protein fragments, nucleic acids (DNA, RNA), PNA (peptide nucleic acids), or their derivatives or mimetics which can modulate the function and/or levels of the target genes or their gene products.

The nucleic acids and/or described herein, or nucleic acids comprising their complementary sequence, may be used as antisense constructs to control gene expression in cells, tissues or organs. The methodology associated with antisense techniques is well known to the skilled artisan, and is described and reviewed in AntisenseDrug Technology: Principles, Strategies, and Applications, Crooke, ed., Marcel Dekker Inc., New York (2001). In general, antisense nucleic acid molecules are designed to be complementary to a region of mRNA expressed by a gene, so that the antisense molecule hybridizes to the mRNA, thus blocking translation of the mRNA into protein. Several classes of antisense oligonucleotide are known to those skilled in the art, including cleavers and blockers. The former bind to target RNA sites, activate intracellular nucleases (*e.g.,* RnaseH or Rnase L), that cleave the target RNA. Blockers bind to target RNA, inhibit protein translation by steric hindrance of the ribosomes. Examples of blockers include nucleic acids, morpholino compounds, locked nucleic acids and methylphosphonates (Thompson, Drug Discovery Today, 7:912-917 (2002)). Antisense oligonucleotides are useful directly as therapeutic agents, and are also useful for determining and validating gene function, for example by gene knock-out or gene knock-down experiments. Antisense technology is further described in Lavery et al., Curr. Opin. Drug Discov. Devel. 6:561-569 (2003), Stephens et al., Curr. Opin. Mol. Ther. 5:118-122 (2003), Kurreck, Eur. J. Biochem. 270:1628-44 (2003), Dias et al., Mol. Cancer Ter. 1:347-55 (2002), Chen, Methods Mol. Med. 75:621-636 (2003), Wang et al., Curr. Cancer Drug Targets 1:177-96 (2001), and Bennett, Antisense Nucleic Acid Drug.Dev. 12:215-24 (2002)

The variants described herein can be used for the selection and design of antisense reagents that are specific for particular variants (*e.g*., particular CNVs, or polymorphic markers in LD with particular CNVs). Using information about the variants described herein, antisense oligonucleotides or other antisense molecules that specifically target mRNA molecules that contain one or more variants described herein can be designed. In this manner, expression of mRNA molecules that contain one or more variants described herein (markers and/or haplotypes) can be inhibited or blocked. In one embodiment, the antisense molecules are designed to specifically bind a particular allelic form (i.e., one or several variants (alleles and/or haplotypes)) of the target nucleic acid, thereby inhibiting translation of a product originating from this specific allele or haplotype, but which do not bind other or alternate variants at the specific polymorphic sites of the target nucleic acid molecule.

As antisense molecules can be used to inactivate mRNA so as to inhibit gene expression, and thus protein expression, the molecules can be used to treat a disease or disorder, such as schizophrenia. The methodology can involve cleavage by means of ribozymes containing nucleotide sequences complementary to one or more regions in the mRNA that attenuate the ability of the mRNA to be translated. Such mRNA regions include, for example, protein-coding regions, in particular protein-coding regions corresponding to catalytic activity, substrate and/or ligand binding sites, or other functional domains of a protein.

The phenomenon of RNA interference (RNAi) has been actively studied for the last decade, since its original discovery in C. *elegans* (Fire et al.,Nature 391:806-11 (1998)), and in recent years its potential use in treatment of human disease has been actively pursued (reviewed in Kim & Rossi, Nature Rev. Genet. 8:173-204 (2007)). RNA interference (RNAi), also called gene silencing, is based on using double-stranded RNA molecules (dsRNA) to turn off specific genes. In the cell, cytoplasmic double-stranded RNA molecules (dsRNA) are processed by cellular complexes into small interfering RNA (siRNA). The siRNA guide the targeting of a protein-RNA complex to specific sites on a target mRNA, leading to cleavage of the mRNA (Thompson, Drug Discovery Today, 7:912-917 (2002)). The siRNA molecules are typically about 20, 21, 22 or 23 nucleotides in length. Thus, described herein are isolated nucleic acid molecules, and the use of those molecules for RNA interference, i.e. as small interfering RNA molecules (siRNA). In one embodiment, the isolated nucleic acid molecules are 18-26 nucleotides in length, preferably 19-25 nucleotides in length, more preferably 20-24 nucleotides in length, and more preferably 21, 22 or 23 nucleotides in length.

Another pathway for RNAi-mediated gene silencing originates in endogenously encoded primary microRNA (pri-miRNA) transcripts, which are processed in the cell to generate precursor miRNA (pre-miRNA). These miRNA molecules are exported from the nucleus to the cytoplasm, where they undergo processing to generate mature miRNA molecules (miRNA), which direct translational inhibition by recognizing target sites in the 3' untranslated regions of mRNAs, and subsequent mRNA degradation by processing P-bodies (reviewed in Kim & Rossi, Nature Rev. Genet. 8:173-204 (2007)).

Clinical applications of RNAi include the incorporation of synthetic siRNA duplexes, which preferably are approximately 20-23 nucleotides in size, and preferably have 3' overlaps of 2 nucleotides. Knockdown of gene expression is established by sequence-specific design for the target mRNA. Several commercial sites for optimal design and synthesis of such molecules are known to those skilled in the art.

Other applications provide longer siRNA molecules (typically 25-30 nucleotides in length, preferably about 27 nucleotides), as well as small hairpin RNAs (shRNAs; typically about 29 nucleotides in length). The latter are naturally expressed, as described in Amarzguioui et al. (FEBS Lett. 579:5974-81 (2005)). Chemically synthetic siRNAs and shRNAs are substrates for *in vivo* processing, and in some cases provide more potent gene-silencing than shorter designs (Kim et al., Nature Biotechnol. 23:222-226 (2005); Siolas et al., Nature Biotechnol. 23:227-231 (2005)). In general siRNAs provide for transient silencing of gene expression, because their intracellular concentration is diluted by subsequent cell divisions. By contrast, expressed shRNAs mediate long-term, stable knockdown of target transcripts, for as long as transcription of the shRNA takes place (Marques et al., Nature Biotechnol. 23:559-565 (2006); Brummelkamp et al., Science 296: 550-553 (2002)).

Since RNAi molecules, including siRNA, miRNA and shRNA, act in a sequence-dependent manner, variants described herein can be used to design RNAi reagents that recognize specific nucleic acid molecules comprising specific CNVs, alleles and/or haplotypes, while not recognizing nucleic acid molecules not comprising the CNV, or comprising other alleles or haplotypes. These RNAi reagents can thus recognize and destroy the target nucleic acid molecules. As with antisense reagents, RNAi reagents can be useful as therapeutic agents (i.e., for turning off disease-associated genes or disease-associated gene variants), but may also be useful for characterizing and validating gene function (*e.g.*, by gene knock-out or gene knock-down experiments).

Delivery of RNAi may be performed by a range of methodologies known to those skilled in the art. Methods utilizing non-viral delivery include cholesterol, stable nucleic acid-lipid particle (SNALP), heavy-chain antibody fragment (Fab), aptamers and nanoparticles. Viral delivery methods include use of lentivirus, adenovirus and adeno-associated virus. The siRNA molecules are in some embodiments chemically modified to increase their stability. This can include modifications at the 2' position of the ribose, including 2'-O-methylpurines and 2'-fluoropyrimidines, which provide resistance to Rnase activity. Other chemical modifications are possible and known to those skilled in the art.

The following references provide a further summary of RNAI, and possibilities for targeting specific genes using RNAi: Kim & Rossi, Nat. Rev. Genet. 8:173-184 (2007), Chen & Rajewsky, Nat. Rev. Genet. 8: 93-103 (2007), Reynolds, et al., Nat. Biotechnol. 22:326-330 (2004), Chi et al., Proc. Natl. Acad. Sci. USA 100:6343-6346 (2003), Vickers et al., J. Biol. Chem. 278:7108-7118 (2003), Agami, Curr. Opin. Chem. Biol. 6:829-834 (2002), Lavery, et al., Curr. Opin. Drug Discov. Devel. 6:561-569 (2003), Shi, Trends Genet. 19:9-12 (2003), Shuey et al., Drug Discov. Today 7:1040-46 (2002), McManus et al., Nat. Rev. Genet. 3:737-747 (2002), Xia et al., Nat. Biotechnol. 20:1006-10 (2002), Plasterk et al., curr. Opin. Genet. Dev. 10:562-7 (2000), Bosher et al., Nat. Cell Biol. 2: E31-6 (2000), and Hunter, Curr. Biol. 9:R440-442 (1999).

A genetic defect leading to increased predisposition or risk for development of a disease, including schizophrenia, or a defect causing the disease, may be corrected permanently by administering to a subject carrying the defect a nucleic acid fragment that incorporates a repair sequence that supplies the normal/wild-type nucleotide(s) at the site of the genetic defect. Such site-specific repair sequence may concompass an RNA/DNA oligonucleotide that operates to promote endogenous repair of a subject's genomic DNA. The administration of the repair sequence may be performed by an appropriate vehicle, such as a complex with polyethelenimine, encapsulated in anionic liposomes, a viral vector such as an adenovirus vector, or other pharmaceutical compositions suitable for promoting intracellular uptake of the adminstered nucleic acid. The genetic defect may then be overcome, since the chimeric oligonucleotides induce the incorporation of the normal sequence into the genome of the subject, leading to expression of the normal/wild-type gene product. The replacement is propagated, thus rendering a permanent repair and alleviation of the symptoms associated with the disease or condition.

Disclosed herein are methods for identifying compounds or agents that can be used to treat schizophrenia. Thus, the CNVs disclosed herein are useful as targets for the identification and/or development of therapeutic agents. In certain embodiments, such methods include assaying the ability of an agent or compound to modulate the activity and/or expression of a nucleic acid that is associated with at least one CNV described herein, or the encoded product of the nucleic acid. This in turn can be used to identify agents or compounds that inhibit or alter the undesired activity or expression of the encoded nucleic acid product. Assays for performing such experiments can be performed in cell-based systems or in cell-free systems, as known to the skilled person. Cell-based systems include cells naturally expressing the nucleic acid molecules of interest, or recombinant cells that have been genetically modified so as to express a certain desired nucleic acid molecule.

Variant gene expression in a patient can be assessed by expression of a variant-containing nucleic acid sequence (for example, a gene containing at least one variant disclosed herein, which can be transcribed into RNA containing the at least one variant, and in turn translated into protein), or by altered expression of a normal/wild-type nucleic acid sequence due to variants affecting the level or pattern of expression of the normal transcripts, for example variants in the regulatory or control region of the gene. Assays for gene expression include direct nucleic acid assays (mRNA), assays for expressed protein levels, or assays of collateral compounds involved in a pathway, for example a signal pathway. Furthermore, the expression of genes that are up- or down-regulated in response to the signal pathway can also be assayed. One embodiment includes operably linking a reporter gene, such as luciferase, to the regulatory region of the gene(s) of interest.

Modulators of gene expression can in one embodiment be identified when a cell is contacted with a candidate compound or agent, and the expression of mRNA is determined. The expression level of mRNA in the presence of the candidate compound or agent is compared to the expression level in the absence of the compound or agent. Based on this comparison, candidate compounds or agents for treating schizophrenia can be identified as those modulating the gene expression of the variant gene. When expression of mRNA or the encoded protein is statistically significantly greater in the presence of the candidate compound or agent than in its absence, then the candidate compound or agent is identified as a stimulator or up-regulator of expression of the nucleic acid. When nucleic acid expression or protein level is statistically significantly less in the presence of the candidate compound or agent than in its absence, then the candidate compound is identified as an inhibitor or down-regulator of the nucleic acid expression.

*Methods of assessing probability of response to therapeutic agents, methods of monitoring progress of treatment and methods of treatment*

Currently, treatment options for schizophrenia include (i) medication, (ii) psychological and social intervention and (iii) other therapies.

*Medication:* The most common medication is antipsychotic medication, which mainly serves to reduce positive symptoms of the disease. Antipsychotic medications include Chlorpromzine (Largactil, Thorazine), Fluphenzine (Prolixin), Haloperidol (Haldol, Serenace), Molindone, Thiothixene (Navane), Thioridzine (Mellaril), Trifluoperazine (Stelazine), Loxapine (Loxapac, Loxitane), Perphenazine, Prochlorperazine (Compazine, Buccastem, Stemetil), Pimozide (Orap) and Zuclopenthixol (Clopixol). The newer atypical antipsychotic drugs are usually preferred for initial treatment since they are often better tolerated and associated with lower rates of tardive dyskinesia, although they are more likely to induce weight gain and obesity-related diseases. Atypical antipsychotic drugs include clozapine (Clozaril), risperidone (Risperdal), Olanzapine (Zyprexa), Quetiapine (Seroquel), Ziprasidone (Geodon), Aripiprazole (Abilify), Paliperidone (Invega), Asenapine, Iloperidone (Zomaril), Sertindole (Serlect), Zotepine, Amisulpride, Bifeprunox, Melperone. Response of symptoms to medication is variable; "Treatment-resistant schizophrenia" is a term used for the failure of symptoms to respond satisfactorily to at least two different antipsychotics. Patients in this category may be prescribed clozapine a medication of superior effectiveness but several potentially lethal side effects including agranulocytosis and myocarditis.

*Psychological and social interventions:* Psychoterapy is widely recommended and used in the treatment of schizophrenia, although services may often be confined to pharmacotherapy because of reimbursement problems or lack of training. Cognitive behavioral therapy (CBT) is used to reduce symptoms and improve related issues such as self-esteem, social functioning, and insight. Although the results of early trials were inconclusive, more recent reviews suggest that CBT can be an effective treatment for the psychotic symptoms of schizophrenia. Another approach is cognitive remediation therapy, a technique aimed at remediating the neurocognitive deficits sometimes present in schizophrenia. Based on techniques of neuropsychological rehabilitation, early evidence has shown it to be cognitively effective, with some improvements related to measurable changes in brain activation as measured by functional MRI.A similar approach known as cognitive enhancement therapy, which focuses on social cognition as well as neurocognition, has shown efficacy.

Family Therapy or Education, which addresses the whole family system of an individual with a diagnosis of schizophrenia, has been consistently found to be beneficial, at least if the duration of intervention is longer-term. Aside from therapy, the impact of schizophrenia on families and the burden on carers has been recognized, with the increasing availability of self-help books on the subject. There is also some evidence for benefits from social skills training, although there have also been significant negative findings. Some studies have explored the possible benefits of music therapy and other creative therapies.

The Soteria model is alternative to inpatient hospital treatment using a minimal medication approach. It is described as a milieu-therapeutic recovery method, characterized by its founder as "the 24 hour a day application of interpersonal phenomenologic interventions by a nonprofessional staff, usually without neuroleptic drug treatment, in the context of a small, homelike, quiet, supportive, protective, and tolerant social environment". Although research evidence is limited, a 2008 systematic review found the programme equally as efffective as treatment with medication in people diagnosed with first and second episode schizophrenia.

*Other treatment options:* Electroconvulsive therapy is not considered a first line treatment but may be prescribed in cases where other treatments have failed. It is more effective where symptoms of catatonia are present, and is recommended for use under NICE guidelines in the UK for catatonia if previously effective, though there is no recommendation for use for schizophrenia otherwise. Psychosurgery has now become a rare procedure and is not a recommended treatment for schizophrenia. The patient's carrier status of any of the CNV risk variants described herein (or surrogate markers in LD with any one of the CNVs) is used to help determine whether a particular treatment modality for schizophrenia, such as any one of the above, or a combination thereof, should be administered. The value lies within the possibilities of being able to diagnose the disease at an early stage, and to select the most appropriate treatment at the earlies possible time point, so as to maximize the likelihood of positive response to the particular therapy.

Also disclosed are methods of monitoring progress or effectiveness of a treatment option for schizophrenia. The treatment option may include any of the above-mentioned treatment options commonly used. This can be done based on the outcome of determination of the presence of a particular CNV risk variant in the individual, or a genetic marker in LD with the CNV, or by monitoring expression of genes that are associated with the variants (CNVs, or markers and haplotypes in LD therewith) disclosed herein. The risk gene mRNA or the encoded polypeptide can be measured in a tissue sample (e.g., a peripheral blood sample, or a biopsy sample). Expression levels and/or mRNA levels can thus be determined before and during treatment to monitor its effectiveness. Alternatively, or concomitantly, the status with respect to a CNV, and or genotype and/or haplotype status of at least one risk variant for schizophrenia presented herein is determined before and during treatment to monitor its effectiveness.

Alternatively, biological networks or metabolic pathways related to the genes within, oassociated with, the CNVs described herein can be monitored by determining mRNA and/or polypeptide levels. This can be done for example, by monitoring expression levels or polypeptides for several genes belonging to the network and/or pathway, in samples taken before and during treatment. Alternatively, metabolites belonging to the biological network or metabolic pathway can be determined before and during treatment. Effectiveness of the treatment is determined by comparing observed changes in expression levels/metabolite levels during treatment to corresponding data from healthy subjects.

In a further aspect, the CNVs described herein, or markers in LD therewith, can be used to increase power and effectiveness of clinical trials. Thus, individuals who are carriers of at least one at-risk CNV or a surrogate marke for the CNV may be more likely to respond to a particular treatment modality for schizophrenia. In one embodiment, individuals who carry at-risk variants for gene(s) in a pathway and/or metabolic network for which a particular treatment (e.g., small molecule drug) is targeting, are more likely to be responders to the treatment. In another embodiment, individuals who carry at-risk variants for a gene, which expression and/or function is altered by the at-risk variant, are more likely to be responders to a treatment modality targeting that gene, its expression or its gene product. This application can improve the safety of clinical trials, but can also enhance the chance that a clinical trial will demonstrate statistically significant efficacy, which may be limited to a certain sub-group of the population. Thus, one possible outcome of such a trial is that carriers of certain genetic variants, e.g., the markers and haplotypes disclosed herein, are statistically significantly likely to show positive response to the therapeutic agent, i.e. experience alleviation of symptoms associated with schizophrenia when taking the therapeutic agent or drug as prescribed.

In a further aspect, the CNVs described herein can be used for targeting the selection of pharmaceutical agents for specific individuals. The pharmaceutical agent can be any of the agents described in the above (e.g., any of the typical and/or atypical antipsychotic medication described in the above). Personalized selection of treatment modalities, lifestyle changes or combination of the two, can be realized by the utilization of the at-risk CNVs or surrogate markers in LD with the CNVs. Thus, the knowledge of an individual's status for particular CNVs can be useful for selection of treatment options, for example for treatments that target genes or gene products affected by one or more of the CNVs. Certain combinations of variants, including those described herein, but also combinations with other risk variants for schizophrenia, may be suitable for one selection of treatment options, while other variant combinations may target other treatment options. Such combinations of variants may include one variant, two variants, three variants, or four or more variants, as needed to determine with clinically reliable accuracy the selection of treatment module.

*Computer-implemented aspects*

The CNVs shown herein to be associated with increased susceptibility (e.g., increased risk) of schizophrenia are in certain embodiments useful for interpretation and/or analysis of genotype data. Thus in certain embodiments, an identification of an at-risk allele for schizophrenia, as shown herein, or an allele at a polymorphic marker in LD with any one of the markers shown herein to be associated with schizophrenia, is indicative of the individual from whom the genotype data originates is at increased risk of schizophrenia. In one such embodiment, genotype data is generated for at CNV shown herein to be associated with risk of schizophrenia, or at least one polymorphic marker in LD with the CNV. The genotype data can be subsequently made available to a third person, for example the individual from whom the data originates, or a representative or guardian of the individual, a genotype service provider, a medical professional such as a medial doctor, a genetic counselor, an insurance provider, etc., for example via a user interface accessable over the internet, together with an interpretation of the genotype data, e.g., in the form of a risk measure (such as an absolute risk (AR), risk ratio (RR) or odds ration (OR)) for the disease (e.g., schizophrenia). In another embodiment, at-risk variants (CNVs or markers in LD therewith) identified in a genotype dataset derived from an individual are assessed and results from the assessment of the risk conferred by the presence of such at-risk varians in the dataset are made available, for example via a secure web interface, or by other communication means. The results of such risk assessment can be reported in numeric form (e.g., by risk values, such as absolute risk, relative risk, and/or an odds ratio, or by a percentage increase in risk compared with a reference), by graphical means, or by other means suitable to illustrate the risk to the individual from whom the genotype data is derived.

As understood by those of ordinary skill in the art, the methods and information described herein (CNV association with schizophrenia) may be implemented, in all or in part, as computer executable instructions on known computer readable media. For example, the methods described herein may be implemented in hardware. Alternatively, the method may be implemented in software stored in, for example, one or more memories or other computer readable medium and implemented on one or more processors. As is known, the processors may be associated with one or more controllers, calculation units and/or other units of a computer system, or implanted in firmware as desired. If implemented in software, the routines may be stored in any computer readable memory such as in RAM, ROM, flash memory, a magnetic disk, a laser disk, or other storage medium, as is also known. Likewise, this software may be delivered to a computing device via any known delivery method including, for example, over a communication channel such as a telephone line, the Internet, a wireless connection, etc., or via a transportable medium, such as a computer readable disk, flash drive, etc.

More generally, and as understood by those of ordinary skill in the art, the various steps described above may be implemented as various blocks, operations, tools, modules and techniques which, in turn, may be implemented in hardware, firmware, software, or any combination of hardware, firmware, and/or software. When implemented in hardware, some or all of the blocks, operations, techniques, etc. may be implemented in, for example, a custom integrated circuit (IC), an application specific integrated circuit (ASIC), a field programmable logic array (FPGA), a programmable logic array (PLA), etc.

When implemented in software, the software may be stored in any known computer readable medium such as on a magnetic disk, an optical disk, or other storage medium, in a RAM or ROM or flash memory of a computer, processor, hard disk drive, optical disk drive, tape drive, etc. Likewise, the software may be delivered to a user or a computing system via any known delivery method including, for example, on a computer readable disk or other transportable computer storage mechanism.

Fig. 8 illustrates an example of a suitable computing system environment 100 on which a system for the steps of the claimed method and apparatus may be implemented. The computing system environment 100 is only one example of a suitable computing environment and is not intended to suggest any limitation as to the scope of use or functionality of the method or apparatus of the claims. Neither should the computing environment 100 be interpreted as having any dependency or requirement relating to any one or combination of components illustrated in the exemplary operating environment 100.

The steps of the claimed method and system are operational with numerous other general purpose or special purpose computing system environments or configurations. Examples of well known computing systems, environments, and/or configurations that may be suitable for use with the methods or system of the claims include, but are not limited to, personal computers, server computers, hand-held or laptop devices, multiprocessor systems, microprocessor-based systems, set top boxes, programmable consumer electronics, network PCs, minicomputers, mainframe computers, distributed computing environments that include any of the above systems or devices, and the like.

The steps of the claimed method and system may be described in the general context of computer-executable instructions, such as program modules, being executed by a computer. Generally, program modules include routines, programs, objects, components, data structures, etc. that perform particular tasks or implement particular abstract data types. The methods and apparatus may also be practiced in distributed computing environments where tasks are performed by remote processing devices that are linked through a communications network. In both integrated and distributed computing environments, program modules may be located in both local and remote computer storage media including memory storage devices.

With reference to Fig. 8, an exemplary system for implementing the steps of the claimed method and system includes a general purpose computing device in the form of a computer 110. Components of computer 110 may include, but are not limited to, a processing unit 120, a system memory 130, and a system bus 121 that couples various system components including the system memory to the processing unit 120. The system bus 121 may be any of several types of bus structures including a memory bus or memory controller, a peripheral bus, and a local bus using any of a variety of bus architectures. By way of example, and not limitation, such architectures include Industry Standard Architecture (ISA) bus, Micro Channel Architecture (MCA) bus, Enhanced ISA (EISA) bus, Video Electronics Standards Association (VESA) loca bus, and Peripheral Component Interconnect (PCI) bus also known as Mezzanine bus.

Computer 110 typically includes a variety of computer readable media. Computer readable media can be any available media that can be accessed by computer 110 and includes both volatile and nonvolatile media, removable and non-removable media. By way of example, and not limitation, computer readable media may comprise computer storage media and communication media. Computer storage media includes both volatile and nonvolatile, removable and non-removable media implemented in any method or technology for storage of information such as computer readable instructions, data structures, program modules or other data. Computer storage media includes, but is not limited to, RAM, ROM, EEPROM, flash memory or other memory technology, CD-ROM, digital versatile disks (DVD) or other optical disk storage, magnetic cassettes, magnetic tape, magnetic disk storage or other magnetic storage devices, or any other medium which can be used to store the desired information and which can accessed by computer 110. Communication media typically embodies computer readable instructions, data structures, program modules or other data in a modulated data signal such as a carrier wave or other transport mechanism and includes any information delivery media. The term "modulated data signal" means a signal that has one or more of its characteristics set or changed in such a manner as to encode information in the signal. By way of example, and not limitation, communication media includes wired media such as a wired network or direct-wired connection, and wireless media such as acoustic, RF, infrared and other wireless media. Combinations of the any of the above should also be included within the scope of computer readable media.

The system memory 130 includes computer storage media in the form of volatile and/or nonvolatile memory such as read only memory (ROM) 131 and random access memory (RAM) 132. A basic input/output system 133 (BIOS), containing the basic routines that help to transfer information between elements within computer 110, such as during start-up, is typically stored in ROM 131. RAM 132 typically contains data and/or program modules that are immediately accessible to and/or presently being operated on by processing unit 120. By way of example, and not limitation, Fig. 8 illustrates operating system 134, application programs 135, other program modules 136, and program data 137.

The computer 110 may also include other removable/non-removable, volatile/nonvolatile computer storage media. By way of example only, Fig. 8 illustrates a hard disk drive 140 that reads from or writes to non-removable, nonvolatile magnetic media, a magnetic disk drive 151 that reads from or writes to a removable, nonvolatile magnetic disk 152, and an optical disk drive 155 that reads from or writes to a removable, nonvolatile optical disk 156 such as a CD ROM or other optical media. Other removable/non-removable, volatile/nonvolatile computer storage media that can be used in the exemplary operating environment include, but are not limited to, magnetic tape cassettes, flash memory cards, digital versatile disks, digital video tape, solid state RAM, solid state ROM, and the like. The hard disk drive 141 is typically connected to the system bus 121 through a non-removable memory interface such as interface 140, and magnetic disk drive 151 and optical disk drive 155 are typically connected to the system bus 121 by a removable memory interface, such as interface 150.

The drives and their associated computer storage media discussed above and illustrated in Fig. 8, provide storage of computer readable instructions, data structures, program modules and other data for the computer 110. In Fig. 8, for example, hard disk drive 141 is illustrated as storing operating system 144, application programs 145, other program modules 146, and program data 147. Note that these components can either be the same as or different from operating system 134, application programs 135, other program modules 136, and program data 137. Operating system 144, application programs 145, other program modules 146, and program data 147 are given different numbers here to illustrate that, at a minimum, they are different copies. A user may enter commands and information into the computer 20 through input devices such as a keyboard 162 and pointing device 161, commonly referred to as a mouse, trackball or touch pad. Other input devices (not shown) may include a microphone, joystick, game pad, satellite dish, scanner, or the like. These and other input devices are often connected to the processing unit 120 through a user input interface 160 that is coupled to the system bus, but may be connected by other interface and bus structures, such as a parallel port, game port or a universal serial bus (USB). A monitor 191 or other type of display device is also connected to the system bus 121 via an interface, such as a video interface 190. In addition to the monitor, computers may also include other peripheral output devices such as speakers 197 and printer 196, which may be connected through an output peripheral interface 190.

The computer 110 may operate in a networked environment using logical connections to one or more remote computers, such as a remote computer 180. The remote computer 180 may be a personal computer, a server, a router, a network PC, a peer device or other common network node, and typically includes many or all of the elements described above relative to the computer 110, although only a memory storage device 181 has been illustrated in Fig. 8. The logical connections depicted in Fig. 8 include a local area network (LAN) 171 and a wide area network (WAN) 173, but may also include other networks. Such networking environments are commonplace in offices, enterprise-wide computer networks, intranets and the Internet.

When used in a LAN networking environment, the computer 110 is connected to the LAN 171 through a network interface or adapter 170. When used in a WAN networking environment, the computer 110 typically includes a modem 172 or other means for establishing communications over the WAN 173, such as the Internet. The modem 172, which may be internal or external, may be connected to the system bus 121 via the user input interface 160, or other appropriate mechanism. In a networked environment, program modules depicted relative to the computer 110, or portions thereof, may be stored in the remote memory storage device. By way of example, and not limitation, Fig. 8 illustrates remote application programs 185 as residing on memory device 181. It will be appreciated that the network connections shown are exemplary and other means of establishing a communications link between the computers may be used.

Although the forgoing text sets forth a detailed description of numerous different embodiments of the invention, it should be understood that the scope of the invention is defined by the words of the claims set forth at the end of this patent. The detailed description is to be construed as exemplary only and does not describe every possibly embodiment of the invention because describing every possible embodiment would be impractical, if not impossible. Numerous alternative embodiments could be implemented, using either current technology or technology developed after the filing date of this patent, which would still fall within the scope of the claims defining the invention.

While the risk evaluation system and method, and other elements, have been described as preferably being implemented in software, they may be implemented in hardware, firmware, etc., and may be implemented by any other processor. Thus, the elements described herein may be implemented in a standard multi-purpose CPU or on specifically designed hardware or firmware such as an application-specific integrated circuit (ASIC) or other hard-wired device as desired, including, but not limited to, the computer 110 of Fig. 8. When implemented in software, the software routine may be stored in any computer readable memory such as on a magnetic disk, a laser disk, or other storage medium, in a RAM or ROM of a computer or processor, in any database, etc. Likewise, this software may be delivered to a user or a diagnostic system via any known or desired delivery method including, for example, on a computer readable disk or other transportable computer storage mechanism or over a communication channel such as a telephone line, the internet, wireless communication, etc. (which are viewed as being the same as or interchangeable with providing such software via a transportable storage medium).

*Nucleic acids and polypeptides*

The nucleic acids and polypeptides described herein can be used in methods of the present invention. An "isolated" nucleic acid molecule, as used herein, is one that is separated from nucleic acids that normally flank the gene or nucleotide sequence (as in genomic sequences) and/or has been completely or partially purified from other transcribed sequences (e.g., as in an RNA library). For example, an isolated nucleic acid disclosed herein can be substantially isolated with respect to the complex cellular milieu in which it naturally occurs, or culture medium when produced by recombinant techniques, or chemical precursors or other chemicals when chemically synthesized. In some instances, the isolated material will form part of a composition (for example, a crude extract containing other substances), buffer system or reagent mix. In other circumstances, the material can be purified to essential homogeneity, for example as determined by polyacrylamide gel electrophoresis (PAGE) or column chromatography (e.g., HPLC). An isolated nucleic acid molecule disclosed herein can comprise at least about 50%, at least about 80% or at least about 90% (on a molar basis) of all macromolecular species present. With regard to genomic DNA, the term "isolated" also can refer to nucleic acid molecules that are separated from the chromosome with which the genomic DNA is naturally associated. For example, the isolated nucleic acid molecule can contain less than about 250 kb, 200 kb, 150 kb, 100 kb, 75 kb, 50 kb, 25 kb, 10 kb, 5 kb, 4 kb, 3 kb, 2 kb, 1 kb, 0.5 kb or 0.1 kb of the nucleotides that flank the nucleic acid molecule in the genomic DNA of the cell from which the nucleic acid molecule is derived.

The nucleic acid molecule can be fused to other coding or regulatory sequences and still be considered isolated. Thus, recombinant DNA contained in a vector is included in the definition of "isolated" as used herein. Also, isolated nucleic acid molecules include recombinant DNA molecules in heterologous host cells or heterologous organisms, as well as partially or substantially purified DNA molecules in solution. "Isolated" nucleic acid molecules also encompass *in vivo* and *in vitro* RNA transcripts of the DNA molecules disclosed herein. An isolated nucleic acid molecule or nucleotide sequence can include a nucleic acid molecule or nucleotide sequence that is synthesized chemically or by recombinant means. Such isolated nucleotide sequences are useful, for example, in the manufacture of the encoded polypeptide, as probes for isolating homologous sequences (e.g., from other mammalian species), for gene mapping (e.g., by *in situ* hybridization with chromosomes), or for detecting expression of the gene in tissue (e.g., human tissue), such as by Northern blot analysis or other hybridization techniques.

Also disclosed are nucleic acid molecules that hybridize under high stringency hybridization conditions, such as for selective hybridization, to a nucleotide sequence described herein (e.g., nucleic acid molecules that specifically hybridize to a nucleotide sequence containing a polymorphic site associated with a marker or haplotype described herein). Such nucleic acid molecules can be detected and/or isolated by allele- or sequence-specific hybridization (e.g., under high stringency conditions). Stringency conditions and methods for nucleic acid hybridizations are well known to the skilled person (see, *e.g.,* Current Protocols in Molecular Biology, Ausubel, F. et al, John Wiley & Sons, (1998), and Kraus, M. and Aaronson, S., Methods Enzymol., 200:546-556 (1991).

The percent identity of two nucleotide or amino acid sequences can be determined by aligning the sequences for optimal comparison purposes (e.g., gaps can be introduced in the sequence of a first sequence). The nucleotides or amino acids at corresponding positions are then compared, and the percent identity between the two sequences is a function of the number of identical positions shared by the sequences (i.e., % identity = # of identical positions/total # of positions x 100). In certain embodiments, the length of a sequence aligned for comparison purposes is at least 30%, at least 40%, at least 50%, at least 60%, at least 70%, at least 80%, at least 90%, or at least 95%, of the length of the reference sequence. The actual comparison of the two sequences can be accomplished by well-known methods, for example, using a mathematical algorithm. A non-limiting example of such a mathematical algorithm is described in Karlin, S. and Altschul, S., Proc. Natl. Acad. Sci. USA, 90:5873-5877 (1993). Such an algorithm is incorporated into the NBLAST and XBLAST programs (version 2.0), as described in Altschul, S. et al., Nucleic Acids Res., 25:3389-3402 (1997). When utilizing BLAST and Gapped BLAST programs, the default parameters of the respective programs (e.g., NBLAST) can be used. See the website on the world wide web at ncbi.nlm.nih.gov. In one embodiment, parameters for sequence comparison can be set at score=100, wordlength=12, or can be varied (e.g., W=5 or W=20). Another example of an algorithm is BLAT (Kent, W.J. Genome Res. 12:656-64 (2002)).

Other examples include the algorithm of Myers and Miller, CABIOS (1989), ADVANCE and ADAM as described in Torellis, A. and Robotti, C., Comput. Appl. Biosci. 10:3-5 (1994); and FASTA described in Pearson, W. and Lipman, D., Proc. Natl. Acad. Sci. USA, 85:2444-48 (1988). In another embodiment, the percent identity between two amino acid sequences can be accomplished using the GAP program in the GCG software package (Accelrys, Cambridge, UK).

Also disclosed are isolated nucleic acid molecules that contain a fragment or portion that hybridizes under highly stringent conditions to a nucleic acid that comprises, or consists of, the nucleotide sequence, the chromosome 15q11.2 deletion, or a nucleotide sequence comprising, or consisting of, the complement of the nucleotide sequence of the chromosome 15q11.2 deletion, wherein the nucleotide sequence comprises at least one polymorphic allele contained in the markers and haplotypes described herein. The nucleic acid fragments disclosed herein are at least about 15, at least about 18, 20, 23 or 25 nucleotides, and can be 30, 40, 50, 100, 200, 500, 1000, 10,000 or more nucleotides in length.

The nucleic acid fragments disclosed herein are used as probes or primers in assays such as those described herein. "Probes" or "primers" are oligonucleotides that hybridize in a base-specific manner to a complementary strand of a nucleic acid molecule. In addition to DNA and RNA, such probes and primers include polypeptide nucleic acids (PNA), as described in Nielsen, P. et al., Science 254:1497-1500 (1991). A probe or primer comprises a region of nucleotide sequence that hybridizes to at least about 15, typically about 20-25, and in certain embodiments about 40, 50 or 75, consecutive nucleotides of a nucleic acid molecule. In one embodiment, the probe or primer comprises at least one allele of at least one polymorphic marker or at least one haplotype described herein, or the complement thereof. In particular embodiments, a probe or primer can comprise 100 or fewer nucleotides; for example, in certain embodiments from 6 to 50 nucleotides, or, for example, from 12 to 30 nucleotides. In other embodiments, the probe or primer is at least 70% identical, at least 80% identical, at least 85% identical, at least 90% identical, or at least 95% identical, to the contiguous nucleotide sequence or to the complement of the contiguous nucleotide sequence. In another embodiment, the probe or primer is capable of selectively hybridizing to the contiguous nucleotide sequence or to the complement of the contiguous nucleotide sequence. Often, the probe or primer further comprises a label, e.g., a radioisotope, a fluorescent label, an enzyme label, an enzyme co-factor label, a magnetic label, a spin label, an epitope label.

The nucleic acid molecules of the invention, such as those described above, can be identified and isolated using standard molecular biology techniques well known to the skilled person. The amplified DNA can be labeled (e.g., radiolabeled, fluorescently labeled) and used as a probe for screening a cDNA library derived from human cells. The cDNA can be derived from mRNA and contained in a suitable vector. Corresponding clones can be isolated, DNA obtained following *in vivo* excision, and the cloned insert can be sequenced in either or both orientations by art-recognized methods to identify the correct reading frame encoding a polypeptide of the appropriate molecular weight. Using these or similar methods, the polypeptide and the DNA encoding the polypeptide can be isolated, sequenced and further characterized.

*Antibodies*

Polyclonal antibodies and/or monoclonal antibodies that specifically bind one form of the gene product but not to the other form of the gene product are also provided. Antibodies are also provided which bind a portion of either the variant or the reference gene product that contains the polymorphic site or sites. The term "antibody" as used herein refers to immunoglobulin molecules and immunologically active portions of immunoglobulin molecules, *i.e.*, molecules that contain antigen-binding sites that specifically bind an antigen. A molecule that specifically binds to a polypeptide disclosed herein is a molecule that binds to that polypeptide or a fragment thereof, but does not substantially bind other molecules in a sample, *e.g.*, a biological sample, which naturally contains the polypeptide. Examples of immunologically active portions of immunoglobulin molecules include F(ab) and $F(ab')_2$ fragments which can be generated by treating the antibody with an enzyme such as pepsin. Also disclosed are polyclonal and monoclonal antibodies that bind to a polypeptide disclosed herein. The term "monoclonal antibody" or "monoclonal antibody composition", as used herein, refers to a population of antibody molecules that contain only one species of an antigen binding site capable of immunoreacting with a particular epitope of a polypeptide disclosed herein. A monoclonal antibody composition thus typically displays a single binding affinity for a particular polypeptide disclosed herein with which it immunoreacts.

Polyclonal antibodies can be prepared as described above by immunizing a suitable subject with a desired immunogen, *e.g.*, polypeptide disclosed herein or a fragment thereof. The antibody titer in the immunized subject can be monitored over time by standard techniques, such as with an enzyme linked immunosorbent assay (ELISA) using immobilized polypeptide. If desired, the antibody molecules directed against the polypeptide can be isolated from the mammal (*e.g.*, from the blood) and further purified by well-known techniques, such as protein A chromatography to obtain the IgG fraction. At an appropriate time after immunization, *e.g.*, when the antibody titers are highest, antibody-producing cells can be obtained from the subject and used to prepare monoclonal antibodies by standard techniques, such as the hybridoma technique originally described by Kohler and Milstein, Nature 256:495-497 (1975), the human B cell hybridoma technique (Kozbor et al., Immunol. Today 4: 72 (1983)), the EBV-hybridoma technique (Cole et al., Monoclonal Antibodies and Cancer Therapy, Alan R. Liss,1985, Inc., pp. 77-96) or trioma techniques. The technology for producing hybridomas is well known (see generally Current Protocols in Immunology (1994) Coligan et al., (eds.) John Wiley & Sons, Inc., New York, NY). Briefly, an immortal cell line (typically a myeloma) is fused to lymphocytes (typically splenocytes) from a mammal immunized with an immunogen as described above, and the culture supernatants of the resulting hybridoma cells are screened to identify a hybridoma producing a monoclonal antibody that binds a polypeptide disclosed herein.

Any of the many well known protocols used for fusing lymphocytes and immortalized cell lines can be applied for the purpose of generating a monoclonal antibody to a polypeptide disclosed herein (see, *e.g.*, *Current Protocols in Immunology, supra;* Galfre et al., Nature 266:55052 (1977); R.H. Kenneth, in Monoclonal Antibodies: A New Dimension In Biological Analyses, Plenum Publishing Corp., New York, New York (1980); and Lerner, Yale J. Biol. Med. 54:387-402 (1981)). Moreover, the ordinarily skilled worker will appreciate that there are many variations of such methods that also would be useful. Alternative to preparing monoclonal antibody-secreting hybridomas, a monoclonal antibody to a polypeptide can be identified and isolated by screening a recombinant combinatorial immunoglobulin library (*e.g.*, an antibody phage display library) with the polypeptide to thereby isolate immunoglobulin library members that bind the polypeptide. Kits for generating and screening phage display libraries are commercially available (*e.g.*, the Pharmacia *Recombinant Phage Antibody System,* Catalog No. 27-9400-01; and the Stratagene *Surf*ZAP™ Phage Display Kit, Catalog No. 240612). Additionally, examples of methods and reagents particularly amenable for use in generating and screening antibody display library can be found in, for example, U.S. Patent No. 5,223,409; PCT Publication No. WO 92/18619; PCT Publication No. WO 91/17271; PCT Publication No. WO 92/20791; PCT Publication No. WO 92/15679; PCT Publication No. WO 93/01288; PCT Publication'No. WO 92/01047; PCT Publication No. WO 92/09690; PCT Publication No. WO 90/02809; Fuchs et al., Bio/Technology 9: 1370-1372 (1991); Hay et al., Hum. Antibod. Hybridomas 3:81-85 (1992); Huse et al., Science 246: 1275-1281 (1989); and Griffiths et al., EMBO J. 12:725-734 (1993).

Additionally, recombinant antibodies, such as chimeric and humanized monoclonal antibodies, comprising both human and non-human portions, which can be made using standard recombinant DNA techniques, are disclosed. Such chimeric and humanized monoclonal antibodies can be produced by recombinant DNA techniques known in the art.

In general, antibodies (*e.g.,* a monoclonal antibody) can be used to isolate a polypeptide by standard techniques, such as affinity chromatography or immunoprecipitation. A polypeptide-specific antibody can facilitate the purification of natural polypeptide from cells and of recombinantly produced polypeptide expressed in host cells. Moreover, an antibody specific for a polypeptide can be used to detect the polypeptide (*e.g.*, in a cellular lysate, cell supernatant, or tissue sample) in order to evaluate the abundance and pattern of expression of the polypeptide. Antibodies can be used diagnostically to monitor protein levels in tissue as part of a clinical testing procedure, *e.g.*, to, for example, determine the efficacy of a given treatment regimen. The antibody can be coupled to a detectable substance to facilitate its detection. Examples of detectable substances include various enzymes, prosthetic groups, fluorescent materials, luminescent materials, bioluminescent materials, and radioactive materials. Examples of suitable enzymes include horseradish peroxidase, alkaline phosphatase, beta-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include $^{125}I$, $^{131}I$, $^{35}S$ or $^{3}H$.

Antibodies may also be useful in pharmacogenomic analysis. In such embodiments, antibodies against variant proteins encoded by nucleic acids disclosed herein, such as variant proteins that are encoded by nucleic acids that contain at least one polymorpic marker disclosed herein, can be used to identify individuals that require modified treatment modalities.

Antibodies can furthermore be useful for assessing expression of variant proteins in disease states, such as in active stages of a disease, or in an individual with a predisposition to a disease related to the function of the protein, in particular schizophrenia. Antibodies specific for a variant protein of the present invention that is encoded by a nucleic acid that comprises at least one polymorphic marker or haplotype as described herein can be used to screen for the presence of the variant protein, for example to screen for a predisposition to schizophrenia as indicated by the presence of the variant protein.

Antibodies can be used in other methods. Thus, antibodies are useful as diagnostic tools for evaluating proteins, such as variant proteins disclosed herein, in conjunction with analysis by electrophoretic mobility, isoelectric point, tryptic or other protease digest, or for use in other physical assays known to those skilled in the art. Antibodies may also be used in tissue typing. In one such embodiment, a specific variant protein has been correlated with expression in a specific tissue type, and antibodies specific for the variant protein can then be used to identify the specific tissue type.

Subcellular localization of proteins, including variant proteins, can also be determined using antibodies, and can be applied to assess aberrant subcellular localization of the protein in cells in various tissues. Such use can be applied in genetic testing, but also in monitoring a particular treatment modality. In the case where treatment is aimed at correcting the expression level or presence of the variant protein or aberrant tissue distribution or developmental expression of the variant protein, antibodies specific for the variant protein or fragments thereof can be used to monitor therapeutic efficacy.

Antibodies are further useful for inhibiting variant protein function, for example by blocking the binding of a variant protein to a binding molecule or partner. Such uses can also be applied in a therapeutic context in which treatment involves inhibiting a variant protein's function. An antibody can be for example be used to block or competitively inhibit binding, thereby modulating (i.e., agonizing or antagonizing) the activity of the protein. Antibodies can be prepared against specific protein fragments containing sites required for specific function or against an intact protein that is associated with a cell or cell membrane. For administration *in vivo,* an antibody may be linked with an additional therapeutic payload, such as radionuclide, an enzyme, an immunogenic epitope, or a cytotoxic agent, including bacterial toxins (diphtheria or plant toxins, such as ricin). The *in vivo* half-life of an antibody or a fragment thereof may be increased by pegylation through conjugation to polyethylene glycol.

The present invention will now be exemplified by the following non-limiting examples.

**EXAMPLE 1.** Large recurrent microdeletions at 1q21.1, 15q11.2 and 15q13.3 associated with Schizophrenia

Reduced fecundity, associated with severe mental disorders, places negative selection pressure on risk alleles and may explain in part why common variants have not been found conferring risk of disorders such as autism, schizophrenia and mental retardation. Thus, rare variants may account for a larger fraction of the overall genetic risk than previously assumed. In contrast to rare single nucleotide mutations, rare copy number variations (CNVs) can be detected using genome-wide SNP arrays. This has led to the identification of CNVs associated with mental retardation and autism.

The approach we employed here was to use a large population-based discovery sample to identify *de novo* CNVs, followed by testing for association in a sample of schizophrenia and psychoses patients (phase I) and finally replicating the most promising variants from phase I in a second larger sample (phase II). The discovery phase, where we searched for *de novo* CNVs, enriches for those regions that mutate most often. If the CNVs identified are in very low frequency in the population despite relatively high mutation rate (> 1/10,000 meiosis), they are likely to be under negative selection pressure. Such variants may confer risk of disorders that reduce the fecundity of those affected.

To uncover de *novo* CNVs genome-wide we analyzed data from a population based sample (2,160 trios and 5,558 parent offspring pairs, none of which were known to have schizophrenia, Table 1) providing information on 9,878 transmissions. Of the 66 *de novo* CNVs identified, 23 were flanked by low copy repeats (LCRs) and nine additional had a LCR flanking only one of the deletion breakpoints. Of the remaining 34 CNVs (not flanked by LCRs), 27 were only found in a single control sample (the discovery trio) out of the 33,250 tested whereas 18 out of the 23 CNVs flanked by LCRs were found in higher frequency in the large control sample (Table 2).

The 66 CNVs were tested for association in our phase I sample of 1,433 schizophrenia and related psychoses patients and 33,250 controls from the SGENE consortium (http://sgene.eu/), all typed at deCODE genetics using the HumanHap300 chip. For eight of the 66 CNVs tested, at least one schizophrenia patient carried the CNV, and for three large deletions, nominal association with schizophrenia was detected (uncorrected *P*-value < 0.05, Table 3). The three deletions nominally associating with schizophrenia in the first sample (Table 3) were followed up in as many as six samples consisting in total of 3,293 cases and 7,951 controls (Table 4). All three deletions, at 1q21.1, 15q11.2 and 15q13.3, significantly associate with schizophrenia and psychosis in the combined sample ($P$=2.9$\times$10$^{-5}$, OR=14.83, $P$=6.0$\times$10$^{-4}$, OR=2.73 and $P$=5.4$\times$10$^{-4}$, OR=11.51, respectively). Removing cases with psychosis, other than DSMIV and RDC defined schizophrenia (in total 161 cases, 49 with unspecified functional psychosis, 89 with schizoaffective disorder, 17 with schizophreniform and six with persistent delusional disorders), gave comparable results for the 1q21.1 deletion ($P$=2.31$\times$10$^{-5}$, OR =15.44) while the association for 15q11.2 and 15q13.3 deletions was no longer significant ($P$=9.57$\times$10$^{-4}$, OR=2.66, and $P$=1.02$\times$10$^{-3}$, OR=11.29, respectively (uncorrected for 66 tests)). Historically, classification schemes tend to group diseases by their signs and symptoms. There is, however, no reason why the phenotypes associating with a particular CNV should be confined to the current nosological boundaries of any single psychiatric disorder. Our findings, in this respect, resemble those from the 16p11.2 deletion (Weiss, L. A. et al. Association between Microdeletion and Microduplication at 16p11.2 and Autism. N Engl J Med (2008)) and the translocation disrupting the *DISC1* gene in a large Scottish pedigree (Millar, J. K. et al. Disruption of two novel genes by a translocation co-segregating with schizophrenia. Hum Mol Genet 9, 1415-23 (2000)) and support the idea that the same mutation can increase risk of a broad range of clinical psychopathology. It is therefore worth noting that among the eight controls carrying the 15q13.3 deletion there is one autistic individual (there are samples from 299 autistic individuals among the 39,800 control samples genotyped for this CNV).

Eleven out of the 4,726 cases tested (0.23%) carry the 1q21.1 deletion compared to eight of the 41,199 controls tested (0.02%). In seven of the eleven patients, the deletion spans about 1.38 Mb (chr1:144,943,150-146,293,282). Four cases have a larger form of the deletion (Table 5). The larger form contains the shorter form and extends to 144,106,312 Mb, about 2.19 Mb (Figure 1A and Figure 2). Seven of the eight Icelandic controls have the shorter form of the deletion and one control has the longer form. Previously reported 1q21.1 deletions in two cases of mental retardation Inoue, K. & Lupski, J. R. Molecular mechanisms for genomic disorders. Annu Rev Genomics Hum Genet 3, 199-242 (2002), Lee, J. A., Carvalho, C. M. & Lupski, J. R. A DNA replication mechanism for generating nonrecurrent rearrangements associated with genomic disorders. Cell 131, 1235-47 (2007)), two autistic individuals (Ni, X. et al. Connexin 50 gene on human chromosome 1q21 is associated with schizophrenia in matched case control and family-based studies. J Med Genet 44, 532-6 (2007)) and one schizophrenia case (Walsh, T. et al. Rare structural variants disrupt multiple genes in neurodevelopmental pathways in schizophrenia. Science 320, 539-43 (2008)) are consistent with the shorter form of the deletion.

The 1.38 Mb deleted segment common to both the large and the small form of the 1q21.1 deletion is gene rich (Figure 1A). The *GJA8* gene has previously been reported as associated with schizophrenia (Ni, X. et al. Connexin 50 gene on human chromosome 1q21 is associated with schizophrenia in matched case control and family-based studies. J Med Genet 44, 532-6 (2007)). On the HumanHap300 chip there are no SNP markers within this gene that is located in a repeat region within the boundary of the 1.38 Mb deletion segment. In at least four reports (Brzustowicz, L. et al., Science 288, 678-82 (2000); Gurling, H. M. et al., Am J Hum Genet 68, 661-73 (2001); Hwu, H. G., et al., Mol Psychiatry 8, 445-52 (2003); Zheng, Y. et al., Biochem Biophys Res Commun 342, 1049-57 (2006)), the 1q21 locus has been linked to schizophrenia, however, the deletion is rare and therefore unlikely to account for much of the linkage previously reported. Analysis of cells from a case with the 1q21.1 deletion and a case with the reciprocal duplication, using FISH (Figure 3), show that other rearrangements, such as chromosomal translocations, are unlikely to be associated with the deletion. The deletion at 15q11.2 was significant in the combined schizophrenia and related psychosis sample (Table 4). In the combined sample 26 of 4726 cases (0.55%) carry the deletion and 79 of 41,190 controls (0.19%). The deletion spans approximately 470 kb (chr15:20,306,549-20,777,695) and several genes are deleted (Figure 1B and Figure 4). A single case with mental retardation and severe speech impairment has previously been reported with the 15q11.2 deletion (Murthy, S. K. et al., Cytogenet Genome Res 116, 135-40 (2007). Although the region is not imprinted, it is depleted in a minority of cases of Angelman syndrome (AS) and Prader Willi syndrome (PWS). Recent analysis show that AS cases with class I deletions (includes the 15q11.2 deletion) are significantly more likely to meet criteria for autism.

PWS type I deletions are associated with increased risk of preservative/obsessive compulsive behavior, deficits in adaptive skills and lower intellectual ability. Thus, the autistic features in AS and the preservative behavior of PWS may arise from deletion of the genes in the proximal portion of the region, the site at the breakpoints of the chromosome 15 deletions found in the current study. The gene deletions in the 15q11.2 region are most likely to be responsible for both the autistic and obsessive compulsive features observed in AS and PWS with class one deletions, and the schizophrenia phenotype in this study is *CYFIP1* (Figure 1C). CYFIP1 interacts with fragile X mental retardation protein (FMRP) as well as with the Rho GTPase Rac1, which is involved in regulating axonal and dendritic outgrowth and the development and maintenance of neuronal structures. Over 30% of children with Fragile X syndrome meet criteria for autism(Rogers, S. J., et al., The behavioral phenotype in fragile X: symptoms of autism in very young children with fragile X syndrome, idiopathic autism, and other developmental disorders. J Dev Behav Pediatr 22, 409-17 (2001)) with highest rates observed in cases with Prader Willi features without the deletion on 15q. Notably, the Fragile X mutation results in a reduction in expression levels of the CYFIP1 gene (Nowicki, S. T. et al. The Prader-Willi phenotype of fragile X syndrome. J Dev Behav Pediatr 28, 133-8 (2007) and Fragile X syndrome behavioral abnormalities resemble features of schizophrenia. Fragile X syndrome is due to complete loss of function of FMRP, whereas the hemizygous deletion of *CYFIP1* would only cause partial disturbance of FMRP function, in which case an effect similar to that observed in Fragile X in females and obligate carriers might be expected. These women have attentional deficit and extreme shyness and anxiety, and they may also present with psychiatric disturbances of which psychotic behavior is the most frequent (Borghgraef, M., et al., The female and the fragile X syndrome: data on clinical and psychological findings in 7 fra(X) carriers. Clin Genet 37, 341-6 (1990), Thompson, N. M. et al., Neurobehavioral characteristics of CGG amplification status in fragile X females. Am J Med Genet 54, 378-83 (1994).

The 15q13.3 deletion is also significantly associated with schizophrenia and related psychoses in the combined samples (Table 4). Seven of 4,221 cases (0.17%) carry the deletion and 8 of 39,800 controls (0.02%). One of several affected genes (Figure 1C and Figure 5), the alpha 7 nicotinic receptor gene (CHRNA7), is targeted to axons by Neuregulin 1 (Hancock, M. L., et al., Presynaptic type III neuregulin1-ErbB signaling targets {alpha}7 nicotinic acetylcholine receptors to axons. J Cell Biol 181, 511-21 (2008), has been implicated in schizophrenia (Freedman, R. et al., Linkage of a neurophysiological deficit in schizophrenia to a chromosome 15 locus. Proc Natl Acad Sci U S A 94, 587-92 (1997) and also in mental retardation (Sharp, A. J. et al., Discovery of previously unidentified genomic disorders from the duplication architecture of the human genome. Nat Genet 38, 1038-42 (2006). Mice lacking the alpha7 subunit of the neural nicotinic receptor show a minor impairment in the matching-to-place task of the Morris water maze, taking longer to find the hidden platform than their wild type controls. This suggests a role for CHRNA7 in working/episodic memory and a potential role for CHRNA7 in schizophrenia and its endophenotypes (Fernandes, C., et al., Performance deficit of alpha7 nicotinic receptor knockout mice in a delayed matching-to-place task suggests a mild impairment of working/episodic-like memory. Genes Brain Behav 5, 433-40 (2006).

On the HumanHap300 array, 99 SNPs are affected by the deletion on 1q21.1, 54 by the 15q11.2 deletion and 166 by the 15q13.3 deletion. Statistically significant association, after correction for the number of tests performed, was not found with schizophrenia and individual SNPs at the three deletion loci, although some of the markers show nominally significant association (Tables 6 - 8). It is however possible that some of these markers represent real association signals that may show statistically signficant association given a larger sample size. Furthermore, rare variants at these loci might still associate with schizophrenia as they are not tagged by markers on the HumanHap300 chip. Finding such variants probably requires re-sequencing of the deleted interval in a large sample of cases and testing identified variants for enrichment in schizophrenia.

From available records, we see that cases carrying the 1q21.1, 15q11.2 and 15q13.3 deletions have clinical response rates to neuroleptics that are comparable to the general schizophrenic patient population. Family history of schizophrenia in close relatives is also comparable to other schizophrenia patients in our sample (although these affected relatives are not available for genotyping) and there is no obvious sex bias, as both males and females carrying the deletions are affected. Assessment of cognitive abilities was only available for a fraction of the cases with deletions. None of the cases carrying the three deletions were known to be mentally retarded; however, three cases carrying the 1q21.1 deletion had learning disabilities and two controls had dyslexia (Tables 5, 9 and 10).

The frequency of the deletions identified here are comparable to the frequency of the VCFS deletion on 22q11, previously shown to associate with schizophrenia (Murphy, K. C. Schizophrenia and velo-cardio-facial syndrome. Lancet 359, 426-30 (2002), Ousley, O., et al., A review of neurocognitive and behavioral profiles associated with 22q11 deletion syndrome: implications for clinical evaluation and treatment. Curr Psychiatry Rep 9, 148-58 (2007). T he large VCFS deletion was present in eight out of 3,846 cases tested (0.2%) (Icelandic (N=1), Scottish (N=5), Dutch (N=1) and German (Bonn, N=1)) but was absent in 39,299 controls ($P$=4.2$\times 10^{-5}$, OR=Inf).

Apart from association to schizophrenia, the deletions at 1q21.1, 15q11.2 and 15q13.3 also otherwise exhibited a pattern of negative selection. In the 33,090 Icelanders (648 schizophrenia patients and 32,442 controls) who are CNV typed, nine carry the deletion on 1q21.1 and 62 carry the deletion on 15q11.2. But not all of these cases resulted from 'first-generation' *de novo* events, i.e. some cases inherited the deletions from their parents. Specifically, by examining the haplotype (sequence of SNPs) background of the deletions and the known familial relationship between the carriers, we deduced that the nine 1q21.1 deletions correspond to six independent mutation/deletion events, the eight 15q13.3 deletions correspond to six independent mutation/deletion events and the 62 15q11.2 deletions correspond to approximately 32 separate events (it is noted that the 15q11.2 deletions in the four Icelandic schizophrenia cases correspond to four separate events, which are shared by a few of the controls). Two conclusions could be drawn from these observations. Firstly, carriers of these deletions are not infertile and, moreover, could pass on the deletion to their children. However, the probability that the carriers could pass on the deletion to a child appears to be substantially lower than that under a model of neutrality and fecundity of carriers therefore reduced. All three deletions, particularly the 15q11.2, occurred rather frequently as a *de novo* event. Assuming that the deletions do not repair themselves (or only doing so with very low probability) during successive meioses, being neutral, the deletions would be expected to have a much higher frequency in the population than observed. Consider the 15q11.2 deletion. When we study the carriers pair-wise, we found that if two carriers are separated by six meioses (second cousins) or less, their deletions are very likely to result from the same deletion event. For example, if two cousins both carry the deletion, they probably both inherited it from a common grandparent who is also a carrier. However, for two carriers that are separated by more than six meioses, it is nearly always that the deletions they carry are results from two separate deletion events. This implies that the deletions that we observe in carriers, if not first generation *de novo,* would only go back a few generations. If we assume that each deletion carried could be traced back on average five generations, the 62 carriers observed out of 33,090 would correspond to an estimated mutation/deletion rate of $62/(5 \times 33090 \times 2)$ (notice that the factor 2 comes in because a person carries two chromosomes), or about 1.9 events in 10,000. This is slightly higher, but not inconsistent, with the 1 in 9878 transmissions that we directly observed. Suppose we assume a mutation rate of 1 in 10,000. Notice that the chromosome a person carried would include all mutations that happened in its history. Even if we consider only the past 30 meioses (or tracing back to about 900 years ago), under a neutral model, the carrier frequency of 15q11.2 in the population would be expected to be around $30 \times (1/10{,}000) \times 2 = 0.006$, or about 198 carriers in 33090 individuals examined. This is substantially higher than the 62 carriers we actually observed.

We emphasize that the analysis described above is only meant to be descriptive. More rigorous investigation is needed to fully understand the selection pressure on the 1q21.1, 15q11.2 and 15q13.3 deletions. Given that the deletions are associated with schizophrenia patients, who are known to have fewer children than the general population, a pattern of negative selection might be expected. However, further negative selection pressure could result from reduced fecundity of carriers due to other phenotypes, and also transmission disequilibrium from carrier to child, i.e. the normal chromosome had a higher probability to be passed on than the chromosome with the deletion.

All the CNVs are flanked by large and complex LCRs sequences (Figures 2, 4 and 5). The LCR can mediate non-allelic homologous recombination (NAHR) which may result in loss or gain of genomic segments (Inoue, K. & Lupski, J. R. Molecular mechanisms for genomic disorders. Annu Rev Genomics Hum Genet 3, 199-242 (2002). Through this process CNVs under negative selection can be maintained at low frequency in the population. Other mechanisms for generating rearrangements (Lee, J. A. et al., A DNA replication mechanism for generating nonrecurrent rearrangements associated with genomic disorders. Cell 131, 1235-47 (2007)) cannot be excluded. For none of the deletions, associating with schizophrenia, are we able to pinpoint which LCRs are mediating the NAHR due to the complexity of the regions flanking the deletions. It is noteworthy that the same CNVs are implicated in schizophrenia and autism and an important area for future study is to determine whether deletions conferring schizophrenia-like syndrome should be considered as classical schizophrenia or a new microdeletion syndromes.

In the present study we searched for variants we think are most likely to confer risk of schizophrenia, namely large recurrent CNVs likely to be under negative selection pressure, rather than testing a large number of selectively neutral CNVs. It is important to identify all recurrent CNVs under negative selection and test those variants for enrichment in well powered samples of schizophrenia cases as well as cases of autism and mental retardation. To determine diagnostic and treatment implications it is also important to study the CNVs conferring risk with respect to drug response, disease progression and symptomatology. Two of the three deletions described here confer high risk of schizophrenia (OR>11) whereas the third is more common and with modest risk (OR=2.73). Already identified CNVs associating with schizophrenia may point the way towards underlying pathogenic pathways in the disease; furthermore, high resolution scans for copy number variants may well identify more CNVs associated with the disease, and given the high odds ratio, these are likley to be clinically useful in diagnosis and risk assessment. Although the CNVs reported here only account for a very small fraction of the genetic risk of schizophrenia this is an exciting step toward what promises to be a fruitful field for further investigation.

## METHODS

**Subjects.** This study was approved by the National Bioethics Committees or the Local Research Ethical Committees and Data Protection Commissions or laws in the respective countries, Iceland, Scotland, UK, Germany, Finland, Italy, Denmark, Norway, The Netherlands and China. Informed consent was obtained from all patients Of the 4,726 genotyped cases, 4,565 were diagnosed with schizophrenia, 49 with unspecified functional psychosis, 89 with schizoaffective disorder, 17 with schizophreniform and six with persistant delusional disorders.

**Genotyping.** The SGENE samples (samples from seven European groups, http:\\www.SGENE.eu) typed on the HumanHap300 chip, were used in phase I of the study (Table 3). In phase II, (Table 4) CNV data were derived from: the HumanHap300 chip, the HumanHap550 chip, the Affymetrix GeneChip(r) GenomeWide SNP 6.0 or dosage measured using Taqman probes (Bieche, I. et al., Novel approach to quantitative polymerase chain reaction using real-time detection: application to the detection of gene amplification in breast cancer. Int J Cancer 78, 661-6 (1998. The Scottish samples in Table 4, were typed at Duke University (HumanHap550) in collaboration with GSK as were 420 of the German samples all from Munich (HumanHap300). The remaining CNV data (HumanHap550) from Germany (Table 4, N=491) were obtained from the University of Bonn. Norwegian samples (Affymetrix GeneChip(r) GenomeWide SNP 6.0 array) were analyzed using the Affymetrix Power Tools 1.8.0. Dosage data for Danish and Chinese samples were generated at deCODE using Taqman assays (Bieche, I. et al. Novel approach to quantitative polymerase chain reaction using real-time detection: application to the detection of gene amplification in breast cancer. Int J Cancer 78, 661-6 (1998)). Samples with CNVs were verified by genotyping respective samples using the HumanCNV370 chip.

**Statistical analysis.** For the genome-wide study of *de novo* CNV associating with schizophrenia the significance threshold was set at $7.6\times10^{-4}$, which is approximately 0.05/66, the number of *de novo* CNVs identified and tested. All P-values are two-sided and there is no overlap between samples in Tables 3 and 4. An exact conditional Cochran-Mantel-Haenszel test (conditional on the strata margins) was used to test for association of schizophrenia and the various CNVs.

**De novo CNV analysis and Dosage measurements**

***De novo* CNV analysis.** To uncover *de novo* CNVs. genome-wide we analyzed data from a population based sample of 2,160 trios and 5,558 parent offspring pairs, totaling 9,878 transmissions. Samples were genotyped using the Illumina HumanHap300 or the HumanCNV370 chips. To identify *de novo* deletions, we combined two complementary methods: DosageMiner, a Hidden Markov Model algorithm based on intensity data that is similar to that reported by Colella et al. (QuantiSNP: an Objective Bayes Hidden-Markov Model to detect and accurately map copy number variation using SNP genotyping data. Nucleic Acids Res 35, 2013-25 (2007)) and a procedure utilizing inheritance errors and the neighboring genotype configurations comparable to that described by Conrad et al. (A high-resolution survey of deletion polymorphism in the human genome. Nat Genet 38, 75-81 (2006). When only one parent was typed, using genotype information allowed us to identify deletions as putatively *de novo* by assessment of regional parental heterozygosity. To identify *de novo* duplications we analyzed CNV data from the 2,160 trios using DosageMiner.

CNVs in phase I were identified by using the DosageMiner software developed by deCODE genetics and loss of heterozygosity analysis. CNV events stand out in the data from two perspectives. First, all sample intensities for SNPs/probes within a CNV should be increased or decreased relative to neighboring SNPs/probes that are not in a CNV region, secondly CNVs can be detected from the transmission from parent to child. To determine deviations in signal intensity we start by normalizing the intensities. The normalized intensities for each color channel were determined by a fit of the following equation:

$$\log(x_{ij}) = f(\alpha_i, gc(j)) + \mu_{j,gen(i,j)} + \beta_i + \varepsilon_{ij}$$

where i is sample index, j is SNP index, $x_{ij}$ is colour intensity for sample i in SNP j, gc(j) is an indicator of GC-content around SNP j, f is a smooth function of GC-content, $\alpha_i$ are sample specific parameters for GC content, gen(i,j) is the genotype for sample i for SNP j, $\mu_{i.gt}$ is the SNP effect for genotype gt and SNP j, $\beta_j$ is sample effect, $\varepsilon_{ij}$ is the unexplained part of the signal, including noise. The same model with another set of parameters is used for the other colour $y_{ij}$. A generalized additive model (Hastie, T. a. T., R. Generalized additive models (with discussion). Statist Sci 1, 297-318 (1986)) is used to fit the smooth function f. After fitting the model, the data is normalized by removing the systematic model components. We consider a region to be a deletion/duplication if the average intensity over at least ten markers in a region falls below/above an empirically determined threshold.

To identify regions demonstrating loss of heterozygosity (LOH) markers are split into three classes: 1) Shows LOH, 2) Inconsistent with LOH, 3) Consistent with LOH. Class 3 is further split into these subclasses: a) consistent with transmitted LOH b) consistent with *de novo* LOH. A marker shows LOH if a child is homozygous for one allele and a parent is homozygous for the other allele. A marker is inconsistent with LOH if the child is heterozygous. A marker is consistent with LOH if the child is homozygous and the parent is homozygous for the same allele or heterozygous. In case the parent is homozygous for the same allele as the child the marker is consistent with transmitted LOH and in case the parent is homozygous for the other allele the marker is only consistent with de novo LOH.

A stretch containing a single marker showing LOH is likely be due to a genotyping error but as our genotyping error rate is low and independent of position on the genome the occurrence of more than one marker showing LOH in a consecutive stretch on the genome is more likely to be evidence of a deletion in the child. We consider a region to be a putative deletion if at least two markers are showing LOH and *de novo* if consistent with *de novo* LOH.

We analyzed 9,878 offspring/parent pairs consisting of a total of 7,718 offspring and 7,121 parents. Using LOH analysis we define a candidate deleted region if more than one marker shows inheritance error within a region of homozygous markers. We identified a total of 270 candidate *de novo* deletions using this approach. Of these, 80 belong to six distinct individuals which all had multiple regions identified as *de novo* deletions on the same chromosome. Upon further inspection of the data for these individuals we concluded that they were examples of uniparental disomy. Once these individuals were removed, the remaining 190 putative *de novo* deletions were compared with the output of DosageMiner, and 55 were consistently called deletions by both approaches. These 55 *de novo* deletions represent 51 loci. In addition 15 large duplications, of 20 or more consecutive markers, were also identified in the trio sample by DosageMiner.

**Dosage measurements - Taqman.** The Danish and Chinese samples in Table 4 were typed using Taqman assays (Bieche, I. et al., Novel approach to quantitative polymerase chain reaction using real-time detection: application to the detection of gene amplification in breast cancer. Int J Cancer 78, 661-6 (1998)). The 1q21.1 assay (*PRK* assay) and 15q11.2 *(NIPA2* assay) were designed using Primer Express software. Applied Biosystems provided FAM labeled probes for the assay which were run as described by Bieche (Bieche, I. et al. Novel approach to quantitative polymerase chain reaction using real-time detection: application to the detection of gene amplification in breast cancer. Int J Cancer 78, 661-6 (1998)). For the reference assay we used a probe in the *CFTR* gene and use the same protocol. The second reference assay, RNASEP ready to use assay, was supplied by Applied Biosystems (Foster City, CA, USA). Samples identified with deletions or duplications by the Taqman dosage measurements were confirmed by typing the sample on the Illumina HumanCNV370 array.

Probe and primers used for the 1q21.1 assay: 6FAM - CCTGCTGTGTGGGCT - MGB,
PRK - F CCTTCAGACCAGCGGATAACA and PRK-R CATGGCAGCAGGATTTGGA

Probe and primers used for the 15q11.2 assay: 6FAM - CAGAGCAGATTGTTATGTAC - MGB, - NIPA2 - F GACTGAAAACGCGCCGATT and NIPA2 - R CCATGGACAGACAAACATTCTTG

Probe and primers used for the CFTR assay: 6FAM - ATT AAG CAC AGT GGA AGA A - MGBNFQ, CFTR-F AACTGGAGCCTTCAGAGGGTAA and CFTR- R CCAGGAAAACTGAGAACAGAATGA

Plates were sealed with optical adhesive cover (Applied Biosystems) and the Real time PCR carried out on an ABI 7900 HT machine, for 40 cycles of 15 seconds at 95° and 1 min at 60° starting out with an initial step of 10 min at 95°.

**SUBJECTS AND ASCERTAINMENT**

*Iceland.* The Icelandic sample consists of 646 schizophrenics and 32,442 controls. Patients and controls are all Icelandic and were recruited from all over Iceland. Diagnoses were assigned according to Research Diagnostic Criteria (RDC) (Spitzer, RL., et al. Research diagnostic criteria: rationale and reliability. Arch Gen Psychiatry 35, 773-82 (1978)) through the use of the lifetime version of the Schizophrenia and Affective Disorders Schedule (SADS-L) (Spitzer RL., et al. The schedule for affective disorders and schizophrenia, lifetime version, New York State Psychiatric Institute, New York, 1977). Of the 646 subjects, 617 were diagnosed with schizophrenia, 24 with schizoaffective disorder and five with unspecified functional psychosis.

The 32,250 Icelandic controls used for this study were recruited as a part of various genetic programs at deCODE and were not screened for psychiatric disorders. The individuals came from genetic programs in the following diseases (approximate number of participants in brackets): Abdominal Aortic Aneurism (400), Addiction (5400), Age-related Macular Degeneration (600), Alzheimer's Disease (700), Anxiety and Panic Disorder (1100), Asthma (1400), Attention Deficit Hyperactivity Disorder (500), Benign Prostatic Hyperplasia (900), Breast Cancer (1600), Chronic Obstructive Pulmonary Disease (900), Colon Cancer (1000), Coronary Artery Disease (4000), Deep Vein Thrombosis (1000), Dyslexia (700), Endometriosis (300), Enuresis (900), Obesity (800), Glaucoma (200), Hypertension (2400), Infectious Diseases (2500), Longevity (1600), Lung Cancer (300), Melanoma (500), Migraine (1300), Osteoarthritis (2600), Osteoporosis (3000), Polycystic Ovary Syndrome (1400), Peripheral Artery Disease (1500), Preeclampsia (800), Prostate Cancer (1400), Psoriasis (900), Restless Legs Syndrome (500), Rheumatoid Arthritis (700), Stroke (1900), Essential Tremor (400), Type II Diabetes (1500), Autism (299) and a set of population controls (900). Because some of the individuals used as controls were participants in more than one program, the numbers of participants in individual programs sum to more than 32,442.

*Finland.* The Finnish sample consists of 191 schizophrenics and 200 regionally selected controls that had no medical history of schizophrenia. Approximately half of the sample originated from an internal isolate of Finland having a 3 % age corrected lifetime risk for schizophrenia compared to the 1.1% of the general population. Two independent psychiatrist blind to family structures made a consensus diagnosis to give a best-estimate lifetime diagnoses according to the criteria of Diagnostic and Statistical Manual of Mental Disorders, 4th edition (DSM-IV) (Diagnostic and statistical manual of mental disorders, fourth edition (DSM-IV), American Psychiatric Press, Inc, Washington DC, 1994).

*Scotland.* The Scottish sample is comprised of 211 schizophrenia cases and 229 controls used in phase I and a replication cohort, 451 schizophrenia cases and 441 controls. All participants self-identified as born in the British Isles (95% in Scotland). All cases met DSM-IV and an ICD-10 criteria for schizophrenia. Diagnosis was made by OPCRIT. Controls were volunteers recruited through general practices in Scotland. Practice lists were screened for potentially suitable volunteers by age and sex and by exclusion of subjects with major mental illness or use of antipsychotic medication.

*UK.* Samples from the UK subjects (N=105) were drawn from the Maudsley Family Study of psychosis (Rosa, A., et al. Further evidence that congenital dermatoglyphic abnormalities are associated with psychosis: a twin study. Schizophr Bull 28, 697-701 (2002)),

the psychosis twin study (Toulopoulou, T., et al. Episodic memory in schizophrenic patients and their relatives. Schizophr Res 63, 261-71 (2003)), and the genetics and psychosis (GAP) study. All controls were unrelated white European Caucasians (N=96). All patients were interviewed with the Schedule for Affective Disorders and Schizophrenia Lifetime Version (SADS-L; Endicott and Spitzer, 1978) which was supplemented with information from case notes and other relatives to assign a lifetime DSM-IV diagnosis of schizophrenia. The GAP cases were diagnosed using the Item Group Checklist (IGC) of the Schedule for Clinical Assessment in Neuropsychiatry (SCAN, Manual,World Health Organization, 1994). Only patients with an ICD-10 research diagnosis of schizophrenia were finally included as cases. Patients were receiving a variety of antipsychotic medications at the time of assessment. The study received approval from the Ethics Committee of the South London and Maudsley Trust and after complete description of the study to the participants, written informed consent was obtained.

*Italy.* Diagnosis of the 85 Italian subjects was identical to that for the GAP sample (See UK subjects). Patients with a diagnosis of psychotic disorders (ICD-10, F20-F25) attending the South Verona CMHS were identified from the South Verona Psychiatric Case Register, and cases with ICD-10 research diagnosis of schizophrenia were finally included. The controls (N=91) were unrelated volunteers randomly selected from the general population of South Verona. The study received ethical approval and after complete description of the study to the participants, written informed consent was obtained.

*Germany - Munich.* The Munich sample consisted of Caucasian 615 cases and 614 controls. Cases diagnosed with DSM-IV schizophrenia were ascertained from the Munich area in Germany. Samples from 195 cases and 192 controls were typed for phase I and the remaining samples used in the replication phase. Detailed medical and psychiatric histories were collected, including a clinical interview using the Structured Clinical Interview for Axis I DSM-IV Disorders (SCID) (First, MB., et al. Structured Clinical Interview for Axis I DSM-IV Disorders, Biometrics Research, New York, 1994). Exclusion criteria included a history of head injury or neurological diseases. The controls were unrelated volunteers randomly selected from the general population of Munich.

*Germany - Bonn.* The Bonn sample is comprised of 491 patients and 875 controls. Patients were recruited from consecutive hospital admissions and were all of German descent. In patients, lifetime best estimate diagnoses according to DSM-IV criteria were based on multiple sources of information including structured interview with the SCID (First, MB., et al. Structured Clinical Interview for Axis I DSM-IV Disorders, Biometrics Research, New York, 1994) or SADS-L (Endicott and Spitzer, 1978) the OPCRIT (McGuffin, P., et al. A polydiagnostic application of operational criteria in studies of psychotic illness. Development and reliability of the OPCRIT system. Arch Gen Psychiatry 48, 764-70 (1991)), medical records and the family history. Best estimate diagnoses were obtained from at least two experienced psychiatrists/psychologists. Controls were derived from two German population-based cohorts, PopGen (N=492) and Heinz Nixdorf Recall (N=383). Ethical approval was obtained from the local Ethics Committees. All participants gave written informed consent.

*The Netherlands - Utrecht/ Nijmegen.* The Dutch sample consisted of 806 patients and 706 controls from Utrecht and additional 3,333 control individuals from Nijmegen in the Netherlands. Inpatients and outpatients were recruited from different psychiatric hospitals and institutions throughout the Netherlands, coordinated via academic hospitals in Amsterdam, Groningen, Maastricht and Utrecht. Detailed medical and psychiatric histories were collected, including the Comprehensive Assessment of Symptoms and History (CASH), an instrument for assessing diagnosis and psychopathology. To exclude related patients and controls, all subjects were fingerprinted (Illumina DNA panel, 400 SNPs). Only patients with a DSM-IV diagnosis of schizophrenia were included as cases. All patients and controls were of Dutch descent, with at least three out of four grandparents of Dutch ancestry. The controls were volunteers and were free of any psychiatric history. Ethical approval was obtained from the local Ethics Committees. All participants gave written informed consent.

The additional controls consisted of 3,333 samples, collected by the Radboud University Nijmegen Medical Centre (RUNMC) for genetic studies (cancer and control samples). All 3,333 participants used in the present study are of self-reported European descent. The study protocol was approved by the Institutional Review Board of Radboud University and all study subjects gave written informed consent.

*Denmark.* The Danish sample included 442 patients who have been recruited to Danish Psychiatric Biobank from the psychiatric departments at the six hospitals in the Copenhagen region. All patients had been clinically diagnosed with schizophrenia according to ICD-10 (F20 and F25) without ever having received a diagnosis of mania or bipolar illness (F30-31). An experienced research- and consultant psychiatrist verified high reliability of the clinical diagnoses, using OPCRIT. Of the 442 patients 30 were schizoaffective, and six persistent delusional disorder. 994 healthy controls subjects were recruited through the Danish Blood Donor Corps in the Copenhagen area. Apparent behavioral abnormality was an exclusion criterion and all individuals stated that they felt completely healthy and were able to discuss health related issues with a physician. Additional 445 population control samples from the Copenhagen area Population controls were recruited by the Danish Headache Center. The Danish Scientific Committees and the Danish Data Protection Agency approved the study and all the patients have give written informed consent prior to inclusion into the project.

*Norway.* The Norwegian sample included 245 patients who had been recruited to the TOP study from all the psychiatric hospitals in the Oslo area. The patients were diagnosed according to Structural Clinical Interview for DSM-IV (SCID) as schizophrenia (N=154) schizoaffective (N=35), schizophreniform disorder (N=12) and psychosis NOS (N=44). The healthy control subjects (N=272) were randomly selected from statistical records of persons from the same catchments area as the patient groups. Only subjects born in Norway, all of Caucasian origin, were contacted by letter and invited to participate. All subjects have given written informed consent prior to inclusion into the project and The Norwegian Scientific-Ethical Committee and the Norwegian Data Protection Agency approved the study.

*China.* The Chinese sample was from Sichuan Province, Southwest China, Cases (N=438) were ascertained from West China Hospital, and were interviewed by a psychiatrist using the SCID. Diagnosis of schizophrenia was assigned on the basis of the interview and medical records according to DSM-IV criteria. Patients were excluded if they had a history of neurological disorders or head injury, or reported intellectual disability. The unrelated controls (N=463) were volunteers from the local population and were free of major mental illness. Ethical approval for the project was granted by West China Hospital and written informed consent was obtained from all participants.

The phase I samples were all typed at deCODE using the HumanHap300 chip. The additional samples (phase II) were typed at Duke University (HumanHap300 or HumanHap550), Bonn University (HumanHap550), UCLA (HumanHap550) and Expression Analysis, Durham (Affymetrix GeneChip(r) GenomeWide SNP 6.0 array) and at deCODE (Dosage analysis, Taqman assays). All subjects identified with a CNV using the Taqman assays were confirmed by typing the respective samples on HumanCNV370 chip. Data from the individual follow up (phase II) samples are shown in Table 4 and a summary of the samples used in the various stages of the study can be found in Table 1.

## FLUORESCENT IN SITU HYBRIDIZATION (FISH)

FISH was carried out at deCODE genetics. Interphases were harvested according to standard CYTOGENETIC methods from human B-lymphoblastoid cell lines (EBV transformed) from six individuals, based on information from the Taqman dosage analysis done previously. We used two BAC probes, RP11-431G14 (covers PRK gene on chromosome 1q21) labelled with biotin (green) and an anchor BAC, RP11-458I7 labelled with digoxigenin (red). The BAC probes were labelled with either Biotin-16-dUTP or Digoxigenin-11-dUTP utilizing a nick translation kit (Roche Applied Science).

The hybridization procedure followed a standard protocol. In short the probes were denatured at 72°C for 5 minutes and pre-annealed at 37°C for 15 minutes, before being applied to denatured slides. The slides were denatured in 70% formamide at 70°C for 2 minutes, quenched in 2×SSC at 4°C and then dehydrated in an ethanol series. Following an overnight hybridization the slides were washed in 50% formamide at 42°C for 10 minutes and 2xSSC at 42°C for 5 minutes. The biotinylated probe was detected with avidin/streptavidin FITC (Vector Lab) followed by a layer of biotinylated Anti Avidin (Vector Lab) and again one layer of avidin FITC was added. The digoxigenin probe was detected using Sheep anti Digoxigenin Rhodamine (Roche Applied science) followed by a layer of Donkey anti Sheep Texas red (Jackson Immuno Research). After detection the interphases were counter-stained with $9\times10^{-3}\mu g$ 4',6-Diamidino-2-phenylindole Dihydrochloride:Hydrate (DAPI) (Sigma) in AF1 mounting medium (Citifluor). The digital imaging was done using a Zeiss Axioplan 2 microscope with Asiocam MRm Zeiss camera, automatic Scanning System Metafer software from Metasystems.

Table 1. Summary of the samples used in the various stages of the study

| Site | CNV identification | | Phase I | | Phase II | |
|---|---|---|---|---|---|---|
| | Aff | Ctrl | Aff | Ctrl | Aff | Ctrl |
| Iceland | - | 17596 | 646 | 32442 | - | - |
| Scotland | - | - | 211 | 229 | 451 | 441 |
| Germany (Munich) | - | - | 195 | 192 | 420 | 422 |
| Germany (Bonn) | - | - | - | - | 491 | 875 |

(continued)

| Site | CNV identification | | Phase I | | Phase II | |
|---|---|---|---|---|---|---|
| | Aff | Ctrl | Aff | Ctrl | Aff | Ctrl |
| UK | - | - | 105 | 96 | - | - |
| The Nethertands | - | - | - | - | 806 | 4039 |
| Italy | - | - | 85 | 91 | - | - |
| Finland | - | - | 191 | 200 | - | - |
| Denmark | - | - | - | - | 442 | 1439 |
| Norway | - | - | - | - | 245 | 272 |
| China | - | - | - | - | 438 | 463 |

**Table 2.**

Low copy repeats flanking CNVs found *de novo*. Of the 66 identified CNVs tested for association 23 are flanked by large repetitive segments (distal or proximal) likely to harbor LCRs. Those flanked by repetitive segments are in most cases seen in more (count) of the 32,442 controls tested. Reference is given where we have found the CNV in a CNV database. Coordinates are based on Build 36 of the human genome.
Iafrate: BAC microarray analysis of 236 putative CNP regions in 55 individuals[9].
Tuzun: Fosmid mapping paired-end sequences from a human fosmid DN A library (297 ISV sites)[10].
Redon: SNP and BAC microarray analysis of HapMap data phase II (270 Individuals)[11].
Locke: CNP in duplication-rich regions using array CGH in the HapMap populations(269 individuals)[12].
McCarroll: Deletions from analysis of SNP genotypes, using the HapMap Phase I data, release 16a. (269 individuals)[13].

| Chromosomal Regions in NCBI Build 36 | CNV | Carriers found in Phase I | Number of flanking LCRsDistal Proximal | | Homology | Reference if present in CNV databases |
|---|---|---|---|---|---|---|
| chr1:144943150..146293282 | del | 8 | >5 | >5 | many different | |
| chr1:144943150..146293282 | dup | 12 | >5 | >5 | many different | |
| chr1:241675290..241717030 | del | 1 | - | >5 | many different | |
| chr1:66487172..66981676 | del | 2 | - | - | | |
| chr2:19443..11594900 | dup | 1 | - | - | | |
| chr2:197605805..204072966 | dup | 1 | - | - | | |
| chr2:198783049..199060613 | del | 1 | - | - | | |
| chr2:239980943..242692820 | del | 1 | - | 1 | 99.30% | |
| chr2:50947040..51164471 | del | 1 | - | - | | |
| chr2:95514686..97033113 | del | 1 | >5 | - | many different | |
| chr3:174806420..176937369 | del | 1 | - | - | | |
| chr3:197326041..197704191 | del | 1 | >5 | >5 | many different | Iafrate/Tuzun:196918333-198862488 |
| chr3:71223511..71819797 | dup | 1 | - | - | | |
| chr3:95019980..99373057 | del | 1 | - | - | | |
| chr3:97879021..101883423 | del | 1 | - | - | | |

(continued)

| Low copy repeats flanking CNVs found *de novo.* Of the 66 identified CNVs tested for association 23 are flanked by large repetitive segments (distal or proximal) likely to harbor LCRs. Those flanked by repetitive segments are in most cases seen in more (count) of the 32,442 controls tested. Reference is given where we have found the CNV in a CNV database. Coordinates are based on Build 36 of the human genome.<br>Iafrate: BAC microarray analysis of 236 putative CNP regions in 55 individuals[9].<br>Tuzun: Fosmid mapping paired-end sequences from a human fosmid DN A library (297 ISV sites)[10].<br>Redon: SNP and BAC microarray analysis of HapMap data phase II (270 Individuals)[11].<br>Locke: CNP in duplication-rich regions using array CGH in the HapMap populations(269 individuals)[12].<br>McCarroll: Deletions from analysis of SNP genotypes, using the HapMap Phase I data, release 16a. (269 individuals)[13]. | | | | | | |
|---|---|---|---|---|---|---|
| **Chromosomal Regions in NCBI Build 36** | **CNV** | **Carriers found in Phase I** | **Number of flanking LCRsDistal Proximal** | | **Homology** | **Reference if present in CNV databases** |
| chr4:151856718..151884547 | del | 4 | - | - | | |
| chr5:34603067..34668956 | del | 1 | >5 | - | many different | |
| chr5:58116787..72845587 | del | 1 | - | - | | |
| chr6:162767020..162943840 | del | 35 | - | - | | Redon: 162760913-163153251 |
| chr6:16699739..16803452 | del | 1 | - | - | | |
| chr7:146077700..147445123 | del | 1 | 4 | - | 98% | |
| chr7:149081..295765 | del | 1 | - | - | | Redon: 106472-298664 |
| chr7:15609872..16251148 | dup | 1 | - | - | | |
| chr7:157553706..158812247 | del | 1 | - | - | | |
| chr7:5050267..5190933 | del | 1 | - | 2 | 99.1% | |
| chr7:5229720..5653268 | dup | 1 | - | 2 | 99.1% | Iafrate:5431460-5671684 |
| chr7:57212608..57659300 | dup | 74 | >5 | >5 | many different | |
| chr7:72388281..73777987 | del | 1 | >5 | >5 | many different | |
| chr7:83887393..85199723 | del | 1 | - | - | | |
| chr8:3931576..4252805 | del | 1 | 2 | - | 98.7 | Iatrate:3586932-5909600 & 3611006-4928252 & 3671288- |
| chr9:194201..5739305 | del | 1 | >5 | - | | |

(continued)

Low copy repeats flanking CNVs found *de novo.* Of the 66 identified CNVs tested for association 23 are flanked by large repetitive segments (distal or proximal) likely to harbor LCRs. Those flanked by repetitive segments are in most cases seen in more (count) of the 32,442 controls tested. Reference is given where we have found the CNV in a CNV database. Coordinates are based on Build 36 of the human genome.
Iafrate: BAC microarray analysis of 236 putative CNP regions in 55 individuals[9].
Tuzun: Fosmid mapping paired-end sequences from a human fosmid DN A library (297 ISV sites)[10].
Redon: SNP and BAC microarray analysis of HapMap data phase II (270 Individuals)[11].
Locke: CNP in duplication-rich regions using array CGH in the HapMap populations(269 individuals)[12].
McCarroll: Deletions from analysis of SNP genotypes, using the HapMap Phase I data, release 16a. (269 individuals)[13].

| Chromosomal Regions in NCBI Build 36 | CNV | Carriers found in Phase I | Number of flanking LCRsDistal Proximal | | Homology | Reference if present in CNV databases |
|---|---|---|---|---|---|---|
| chr10:67880428..68013385 | del | 1 | - | - | | |
| chr10:7917790..8021528 | del | 2 | - | - | | |
| chr10:81567594..81962366 | del | 3 | >5 | >5 | many different | |
| chr11:128201807..134435899 | del | 1 | - | - | | |
| chr11:84603291..85465999 | dup | 1 | - | - | | |
| chr12:115338506..115813464 | dup | 1 | - | - | | |
| chr12:98512325..98707024 | del | 1 | - | - | | |
| chr15:20306549..20777695 | del | 58 | >5 | >5 | many different | Redon/lafrate:18263733-21365850 & lafrate:18403666-21241985 |
| chr15:20306549..20777695 | dup | 128 | >5 | >5 | many different | Redon/lafrate:18263733-21365850 & lafrate:18403666-21241985 |
| chr15:20306549..26208861 | dup | 6 | >5 | >5 | many different | |
| chr15:28723577..30302218 | del | 7 | >5 | >5 | many different | |
| chr15:47635303..47679448 | del | 94 | - | - | | |
| chr16:15032942..16197033 | del | 10 | >5 | >5 | many different | |

(continued)

Low copy repeats flanking CNVs found *de novo.* Of the 66 identified CNVs tested for association 23 are flanked by large repetitive segments (distal or proximal) likely to harbor LCRs. Those flanked by repetitive segments are in most cases seen in more (count) of the 32,442 controls tested. Reference is given where we have found the CNV in a CNV database. Coordinates are based on Build 36 of the human genome.
Iafrate: BAC microarray analysis of 236 putative CNP regions in 55 individuals[9].
Tuzun: Fosmid mapping paired-end sequences from a human fosmid DN A library (297 ISV sites)[10].
Redon: SNP and BAC microarray analysis of HapMap data phase II (270 Individuals)[11].
Locke: CNP in duplication-rich regions using array CGH in the HapMap populations(269 individuals)[12].
McCarroll: Deletions from analysis of SNP genotypes, using the HapMap Phase I data, release 16a. (269 individuals)[13].

| Chromosomal Regions in NCBI Build 36 | CNV | Carriers found in Phase I | Number of flanking LCRsDistal Proximal | | Homology | Reference if present in CNV databases |
|---|---|---|---|---|---|---|
| chr16:21515973..21647775 | del | 31 | >5 | >5 | many different | Iafrate:21241957-21833734 & Tuzun:21485317-22595351 & |
| chr16:21856623..22331199 | del | 17 | >5 | >5 | many different | Tuzun:21485317-22595351 & 21500522-22586272 |
| chr16:29563365..30085308 | del | 11 | >5 | >5 | many different | |
| chr16:77757915..78273834 | del | 1 | - | - | | |
| chr16:81429793..81491808 | del | 1 | - | - | | |
| chr16:86921984..87097884 | del | 1 | - | - | | Redon: 86986674-87137417 |
| chr17:14041754..15390352 | del | 5 | 2 | >5 | many different | |
| chr17:15390352..20231611 | del | 1 | >5 | >5 | many different | |
| chr17:31889664..33323543 | dup | 11 | >5 | >5 | many different | |
| chr17:796976..1155912 | del | 1 | 2 | 4 | many different | |
| chr17:9071043..9382978 | del | 1 | - | - | | |
| chr18:75020837..75408356 | dup | 1 | - | - | | |
| chr19:20844764..20914290 | dup | 6 | 2 | 1 | 98% | |

(continued)

Low copy repeats flanking CNVs found *de novo.* Of the 66 identified CNVs tested for association 23 are flanked by large repetitive segments (distal or proximal) likely to harbor LCRs. Those flanked by repetitive segments are in most cases seen in more (count) of the 32,442 controls tested. Reference is given where we have found the CNV in a CNV database. Coordinates are based on Build 36 of the human genome.
Iafrate: BAC microarray analysis of 236 putative CNP regions in 55 individuals[9].
Tuzun: Fosmid mapping paired-end sequences from a human fosmid DN A library (297 ISV sites)[10].
Redon: SNP and BAC microarray analysis of HapMap data phase II (270 Individuals)[11].
Locke: CNP in duplication-rich regions using array CGH in the HapMap populations(269 individuals)[12].
McCarroll: Deletions from analysis of SNP genotypes, using the HapMap Phase I data, release 16a. (269 individuals)[13].

| Chromosomal Regions in NCBI Build 36 | CNV | Carriers found in Phase I | Number of flanking LCRsDistal Proximal | | Homology | Reference if present in CNV databases |
|---|---|---|---|---|---|---|
| chr19:267040..1822341 | del | 1 | - | - | | |
| chr19:54264641..54560863 | del | 1 | >5 | >5 | many different | |
| chr20:14610721..14884935 | del | 1 | - | - | | |
| chr20:14849776..15034277 | del | 22 | - | - | | |
| chr20:14874333..15174767 | del | 5 | - | - | | |
| chr21:34846103..35391627 | dup | 1 | - | - | | |
| chr22:17257787..17373128 | del | 56 | >5 | >5 | many different | Iafrate: 16931796-17441713 & 17011366-17417535 |
| chr12:19063495..19792353 | del | 3 | >5 | >5 | many different | |
| chr22:21063401..21394287 | del | 3 | >5 | >5 | many different | McCarroll: 21032391-21564096 & Iafrate: 20487965-21442582 & 20759608-21442582 & 21032391-21564096 |

**Table 3.** Nominal association of deletions at 1q21.1, 15q11.2 and 15q13.3 with Schizophrenia in the phase I sample.

Three deletions show nominal association with schizophrenia and related psychoses in the first sample of 1433 patients and 33,250 controls. These deletions are large, the 1q21 deletion spans approximately 1.38 Mb, the one on 15q11.2 approximately 0.58 Mb and the one on 15q13.3 approximately 1.57 Mb. P-values (uncorrected for the 66 tests) are from the exact Cochran-Mantel-Haenszel test and are two-sided. Coordinates are based on Build 36 assembly of the human genome. 95% CI are given within brackets.

| Locus | chr1:144.94-146.29 | | chr15:20.31-20.78 | | chr15:28.72-30.30 | |
|---|---|---|---|---|---|---|
| | Aff | Ctrl | Aff | Ctrl | Aff | Ctrl |
| Iceland | 1/646 | 8/32442 | 4/646 | 58/32442 | 1/646 | 7/32442 |
| Scotland | 2/211 | 0/229 | 2/211 | 0/229 | 1/211 | 0/229 |
| Germany | 1/195 | 0/192 | 3/195 | 0/192 | 1/195 | 0/192 |
| UK | 0/105 | 0/96 | 1/105 | 0/96 | 0/105 | 0/96 |
| Italy | 0/85 | 0/91 | 0/85 | 0/91 | 0/85 | 0/91 |
| Finland | 0/191 | 0/200 | 0/191 | 1/200 | 0/191 | 0/200 |
| OR | 8.68 (1.02, 49.76) | | 3.90 (1.42, 9.37) | | 8.94 (0.79, 58.15) | |
| *P*-value | 0.024 | | 0.007 | | 0.040 | |

**Table 4.** Significant association of deletions at 1q21.1, 15q11.2 and 15q13.3 with Schizophrenia in the combined phase I and phase II samples

| Locus | chr1:144.94-146.29 | | chr15:20.31-20.78 | | chr15:28 72-30.30 | |
|---|---|---|---|---|---|---|
| | Aff | Ctrl | Aff | Ctrl | Aff | Ctrl |
| Germany | 2/911 | 0/1297 | 3/911 | 4/1297 | 0/911 | 0/1297 |
| Scotland | 2/451 | 0/441 | 5/451 | 1/441 | 0/451 | 0/441 |
| The Netherlands | 0/806 | 0/4039 | 4/806 | 12/4039 | 3/806 | 1/4039 |
| Norway | 0/245 | 0/272 | 0/245 | 0/272 | 1/245 | 0/272 |
| Denmark* | 3/442 | 0/1437 | 4/442 | 3/1432 | 0/375 | 0/501 |
| China* | 0/438 | 0/463 | 0/438 | 0/463 | na | na |
| **Phase II** | | | | | | |
| OR | Inf (2.85, Inf) | | 2.18 (1.01, 4.60) | | 16.43(1.51, 831.91) | |
| *P*-value | $5.6x10^{-4}$ | | 0.032 | | $8.0x10^{-3}$ | |
| **Phase I & II** | | | | | | |
| OR | 14.83 (3.55, 60.40) | | 2.73 (1.50, 4.89) | | 11.51 (2.51, 49.52) | |
| *P*-value | $2.9x10^{-5}$ | | $6.0x10^{-4}$ | | $5.4x10^{-4}$ | |

**Table 5.** Diagnosis, family history, age of onset, response to neuroleptics based on available records and learning ability in cases carrying the 1q21.1 deletion associating with schizophrenia.

| Case ID | Diagnosis | Family history | Age of onset | Gender | Response | Other |
|---|---|---|---|---|---|---|
| Munich 1 | DSMIV:295.3 | Yes | 24 | male | Yes | Aggressive, learning disability. Not MR |

(continued)

| Case ID | Diagnosis | Family history | Age of onset | Gender | Response | Other |
|---|---|---|---|---|---|---|
| Bonn 1* | DSMIV:295.3 | No | 33 | female | Yes | Not MR |
| Bonn 2 | DSMIV:295.3 | No | 16 | female | relapse under medication | Not MR, depressive symptoms |
| Iceland 1 | RDC:126.3 | No | 26 | female | Yes | Not MR |
| Scotland 1 | DSMIV:295 | No | 43 | female | Yes | Not MR |
| Scotland 2* | DSM-IV:295 | Yes | 21 | male | Yes | Not MR |
| Scotland 3* | DSMIV:295 | No | 32 | male | Yes | Not MR, mother with low IQ |
| Scotland 4* | DSMIV:295 | No | 33 | female | Yes | Not MR, borderline learning disability |
| Denmark 1 | DSMIV: 295 | Yes | 24 | female | Yes | Not MR |
| Denmark 2 | DSMIV: 295 | No | 23 | male | Yes | Boarderline metal retardation |
| Denmark 3 | ICD10: Scz (F20) | No | 20 | male | No | Not MR |
| * There are two forms of the 1q21.1 deletion, long and short. Those marked with an asterisk in the table above have the larger form. MR=mentally retarded. | | | | | | |

**Table 6.** Markers on the Illumina HumanHap300 within the 1q21.1 deletion. Shown are results of association of the markers with schizophrenia, and genes associated with the marker are also indicated. Data from 2,687 cases and 13,484 controls were used in the association analysis.

| Marker | Allele | OR | *P*-value | Chr | Pos. In Build 36 | Gene |
|---|---|---|---|---|---|---|
| rs12406844 | C | 1.14 | 0.001 | 1 | 145436035 | |
| rs12141187 | C | 1.13 | 0.0023 | 1 | 145449387 | |
| rs10465885 | C | 1.12 | 0.0033 | 1 | 145699364 | GJA5 |
| rs6684174 | C | 1.11 | 0.0067 | 1 | 145683484 | |
| rs2644577 | C | 0.9 | 0.0075 | 1 | 145409110 | |
| rs4950437 | A | 0.9 | 0.0076 | 1 | 145394019 | OR13Z2P |
| rs952477 | A | 1.1 | 0.0113 | 1 | 145716820 | GJA5 |
| rs10793705 | C | 1.1 | 0.015 | 1 | 145706931 | GJA5 |
| rs4132958 | C | 1.09 | 0.0258 | 1 | 145430649 | |
| rs12755965 | C | 0.92 | 0.03 | 1 | 145658465 | |
| rs12022413 | A | 0.92 | 0.0328 | 1 | 145463869 | BCL9 |
| rs613089 | C | 1.09 | 0.0356 | 1 | 145547811 | BCL9 |
| rs4950322 | A | 1.1 | 0.0372 | 1 | 145321460 | |
| rs10900321 | C | 0.92 | 0.0431 | 1 | 145096540 | PRKAB2,LOC400780 |
| rs1342709 | C | 1.08 | 0.0432 | 1 | 145744388 | |
| rs3766510 | A | 0.89 | 0.0434 | 1 | 145596846 | ACP6 |

(continued)

| Marker | Allele | OR | *P*-value | Chr | Pos. In Build 36 | Gene |
|---|---|---|---|---|---|---|
| rs4950361 | A | 1.09 | 0.0472 | 1 | 145025789 | LOC441904,LOC440677 |
| rs2236570 | A | 0.92 | 0.0495 | 1 | 145560511 | BCL9 |
| rs1932977 | A | 0.92 | 0.0603 | 1 | 145155565 | FMO5 |
| rs11240007 | C | 1.08 | 0.0662 | 1 | 145304073 | |
| rs945742 | A | 0.93 | 0.0728 | 1 | 145251781 | |
| rs4950402 | G | 1.08 | 0.0787 | 1 | 145258026 | |
| rs903786 | C | 1.09 | 0.0839 | 1 | 145830625 | LOC391092,GJA8 |
| rs11240147 | A | 1.13 | 0.0856 | 1 | 145824905 | LOC391092,GJA8 |
| rs10494251 | A | 1.14 | 0.1012 | 1 | 145490518 | BCL9 |
| rs903784 | A | 0.92 | 0.102 | 1 | 145830723 | LOC391092,GJA8 |
| rs999095 | A | 0.92 | 0.1048 | 1 | 145676851 | |
| rs11811023 | C | 1.07 | 0.1075 | 1 | 145047742 | LOC440678 |
| rs21327 | C | 1.07 | 0.1119 | 1 | 144995145 | LOC440677 |
| rs3820129 | A | 1.06 | 0.1145 | 1 | 145558596 | BCL9 |
| rs11239984 | A | 0.94 | 0.1174 | 1 | 145258353 | |
| rs1417279 | A | 1.09 | 0.1212 | 1 | 145574517 | BCL9 |
| rs2883318 | G | 1.06 | 0.1216 | 1 | 145315767 | |
| rs2353974 | A | 1.06 | 0.1297 | 1 | 145322880 | |
| rs1932978 | C | 0.95 | 0.1532 | 1 | 145194387 | CHD1L |
| rs12408395 | A | 1.07 | 0.1535 | 1 | 145372992 | |
| rs11239953 | T | 1.06 | 0.1559 | 1 | 145184188 | CHD1L |
| rs2275552 | C | 1.06 | 0.1566 | 1 | 145598569 | ACP6 |
| rs647596 | G | 1.05 | 0.1593 | 1 | 145002018 | LOC440677 |
| rs6593752 | C | 1.06 | 0.1766 | 1 | 145196592 | CHD1L |
| rs2353986 | C | 0.95 | 0.1781 | 1 | 145288493 | |
| rs2077749 | A | 1.05 | 0.181 | 1 | 145119261 | PRKAB2, LOC400780,FMO5 |
| rs11576760 | C | 1.09 | 0.1956 | 1 | 145806592 | LOC391092 |
| rs2353987 | G | 0.95 | 0.202 | 1 | 145294180 | |
| rs4950328 | C | 1.05 | 0.2235 | 1 | 145471435 | BCL9 |
| rs2353544 | A | 0.95 | 0.2365 | 1 | 145515224 | BCL9 |
| rs2353983 | C | 1.04 | 0.2809 | 1 | 145279761 | |
| rs7541090 | C | 1.04 | 0.3058 | 1 | 145353686 | OR13Z1P |
| rs627219 | G | 0.92 | 0.3068 | 1 | 145539979 | BCL9 |
| rs10900403 | G | 0.95 | 0.3305 | 1 | 145807358 | |
| rs2999613 | A | 1.06 | 0.3586 | 1 | 146286966 | |
| rs10494246 | A | 1.1 | 0.3592 | 1 | 145614928 | ACP6 |
| rs2354432 | A | 1.05 | 0.3811 | 1 | 145159853 | FMO5 |

(continued)

| Marker | Allele | OR | *P*-value | Chr | Pos. In Build 36 | Gene |
|---|---|---|---|---|---|---|
| rs885239 | A | 0.95 | 0.3831 | 1 | 145594226 | ACP6 |
| rs1353431 | c | 0.94 | 0.3926 | 1 | 145764604 | LOC391092 |
| rs1390510 | A | 1.07 | 0.3975 | 1 | 145497947 | BCL9 |
| rs4950392 | G | 0.96 | 0.4064 | 1 | 145203172 | CHD1L |
| rs10494245 | A | 1.04 | 0.4135 | 1 | 145637476 | |
| rs1853782 | C | 1.04 | 0.43 | 1 | 144975398 | LOC440677 |
| rs4504949 | A | 1.06 | 0.4304 | 1 | 145368301 | OR13Z2P |
| rs584323 | C | 0.97 | 0.4348 | 1 | 145442845 | |
| rs1541187 | A | 1.04 | 0.4534 | 1 | 145518117 | BCL9 |
| rs1572825 | A | 0.97 | 0.4567 | 1 | 145473996 | BCL9 |
| rs6664767 | G | 1.03 | 0.4681 | 1 | 145776067 | LOC391092 |
| rs1814653 | A | 0.96 | 0.4757 | 1 | 146209659 | LOC440679,LOC388684 |
| rs894469 | A | 1.05 | 0.4853 | 1 | 145139530 | FMO5 |
| rs1015235 | A | 0.97 | 0.5032 | 1 | 145510166 | BCL9 |
| rs894467 | C | 0.94 | 0.5305 | 1 | 145128642 | PRKAB2,FMO5 |
| rs1908627 | C | 0.95 | 0.5319 | 1 | 145727389 | GJA5 |
| rs4950574 | A | 1.03 | 0.5358 | 1 | 146216845 | LOC440679,LOC388684 |
| rs6937 | A | 0.97 | 0.5686 | 1 | 145093546 | PRKAB2 |
| rs946904 | C | 0.98 | 0.5768 | 1 | 145589455 | ACP6 |
| rs2992453 | A | 0.98 | 0.5966 | 1 | 146253348 | LOC440680 |
| rs596561 | C | 0.97 | 0.611 | 1 | 145447612 | |
| rs1353428 | G | 0.98 | 0.6115 | 1 | 145792846 | |
| rs7526407 | C | 1.03 | 0.6163 | 1 | 145537233 | BCL9 |
| rs11804045 | A | 1.03 | 0.6551 | 1 | 145628401 | ACP6 |
| rs4950494 | A | 1.02 | 0.6672 | 1 | 145838200 | LOC391092,GJA8 |
| rs10494257 | A | 0.98 | 0.6923 | 1 | 145721193 | GJA5 |
| rs1495956 | C | 1.02 | 0.7095 | 1 | 145705110 | GJA5 |
| rs1344 | A | 1.01 | 0.7439 | 1 | 145585897 | ACP6 |
| rs12141387 | A | 1.01 | 0.7529 | 1 | 144970465 | LOC440677 |
| rs11261254 | C | 0.98 | 0.7631 | 1 | 146185099 | |
| rs10494243 | C | 1.03 | 0.7723 | 1 | 145146427 | FMO5 |
| rs6593746 | A | 1.03 | 0.8049 | 1 | 145153273 | FMO5 |
| rs2932454 | G | 1.01 | 0.8354 | 1 | 146293282 | FLJ39739,RNU1P10 |
| rs12061877 | C | 0.99 | 0.8369 | 1 | 145730876 | GJAS |
| rs1763457 | C | 0.99 | 0.8455 | 1 | 146262302 | LOC440680 |
| rs7530962 | A | 1.01 | 0.8523 | 1 | 145614797 | ACP6 |
| rs2452 | A | 0.99 | 0.8609 | 1 | 145220003 | CHD1L |
| rs6693109 | A | 1.01 | 0.8686 | 1 | 145287960 | |

(continued)

| Marker | Allele | OR | *P*-value | Chr | Pos. In Build 36 | Gene |
|---|---|---|---|---|---|---|
| rs11240009 | A | 0.99 | 0.8697 | 1 | 145308966 | |
| rs9661159 | A | 0.99 | 0.874 | 1 | 145224547 | CHD1L |
| rs1001193 | C | 1.01 | 0.8784 | 1 | 145633001 | |
| rs1857208 | A | 1.01 | 0.907 | 1 | 145758611 | |
| rs671205 | C | 1 | 0.9479 | 1 | 144989346 | LOC440677 |
| rs2000072 | A | 1 | 0.9581 | 1 | 145437192 | |

Table 7. Markers on the Illumina HumanHap300 within the 15q11.2 deletion. Shown are results of association of the markers with schizophrenia, and genes associated with the marker are also indicated. Data from 2,687 cases and 13,484 controls were used in the association analysis.

| Marker | Allele | OR | *P*-value | Chr | Pos. In Build 36 | Gene |
|---|---|---|---|---|---|---|
| rs8029320 | A | 1.17 | 0.0008 | 15 | 20437666 | CYFIP1 |
| rs1897786 | A | 1.15 | 0.0061 | 15 | 20545323 | CYFIP1 |
| rs999842 | C | 0.91 | 0.0163 | 15 | 20551713 | CYFIP1,NIPA2 |
| rs4778413 | C | 1.09 | 0.0507 | 15 | 20560833 | NIPA2,CYFIP1 |
| rs6606817 | C | 1.08 | 0.0647 | 15 | 20567999 | NIPA2 |
| rs4778370 | C | 0.91 | 0.0764 | 15 | 20578289 | NIPA2 |
| rs8034210 | C | 0.93 | 0.081 | 15 | 20347960 | |
| rs12911925 | C | 1.1 | 0.0917 | 15 | 20568493 | NIPA2 |
| rs4778334 | A | 0.93 | 0.11 | 15 | 20592297 | |
| rs7168000 | G | 1.08 | 0.1283 | 15 | 20564567 | CYFIP1,NIPA2 |
| rs7170838 | C | 0.93 | 0.1334 | 15 | 20572679 | NIPA2 |
| rs4778464 | A | 0.93 | 0.1518 | 15 | 20537129 | CYFIP1 |
| rs2289819 | C | 0.93 | 0.1522 | 15 | 20512379 | CYFIP1 |
| rs4778575 | T | 0.95 | 0.2031 | 15 | 20605280 | NIPA2,NIPA1 |
| rs1009153 | C | 0.95 | 0.2039 | 15 | 20528352 | CYFIP1 |
| rs4293342 | C | 1.05 | 0.2069 | 15 | 20455753 | CYFIP1 |
| rs1991922 | C | 0.92 | 0.2168 | | 20610835 | NIPA1 |
| rs12594495 | A | 1.05 | 0.2193 | 15 | 20499445 | CYFIP1 |
| rs7181789 | A | 0.96 | 0.2454 | 15 | 20595337 | NIPA2,NIPA1 |
| rs12441373 | A | 1.13 | 0.2619 | 15 | 20541359 | CYFIP1 |
| rs2289824 | C | 0.94 | 0.268 | 15 | 20477670 | CYFIP1 |
| rs2028794 | A | 0.96 | 0.2818 | 15 | 20470856 | CYFIP1 |
| rs2278458 | A | 0.9 | 0.3075 | 15 | 20551298 | CYFIP1,NIPA2 |
| rs8031642 | C | 1.04 | 0.3118 | 15 | 20351272 | LOC390544 |
| rs3693 | A | 1.04 | 0.329 | 15 | 20556334 | CYFIP1,NIPA2 |
| rs2289815 | G | 0.96 | 0.3483 | 15 | 20421301 | TUBGCP5 |
| rs4778470 | C | 0.96 | 0.3797 | 15 | 20523005 | CYFIP1 |

(continued)

| Marker | Allele | OR | *P*-value | Chr | Pos. In Build 36 | Gene |
|---|---|---|---|---|---|---|
| rs7167658 | C | 1.04 | 0.421 | 15 | 20460862 | CYFIP1 |
| rs1347314 | C | 0.94 | 0.445 | 15 | 20585443 | NIPA2,NIPA1 |
| rs7168367 | C | 1.04 | 0.4805 | 15 | 20618177 | NIPA1 |
| rs5006363 | A | 0.95 | 0.4848 | 15 | 20398953 | TUBGCP5 |
| rs722410 | A | 1.03 | 0.4896 | 15 | 20475538 | CYFIP1 |
| rs765763 | C | 0.97 | 0.5022 | 15 | 20428330 | TUBGCP5,CYFIP1 |
| rs6606825 | A | 1.04 | 0.5038 | 15 | 20614243 | NIPA1 |
| rs4932679 | C | 1.03 | 0.5296 | 15 | 20322108 | LOC390544 |
| rs2289823 | A | 0.97 | 0.539 | 15 | 20479393 | CYFIP1 |
| rs956120 | C | 1.02 | 0.5545 | 15 | 20489279 | CYFIP1 |
| rs4592619 | C | 0.97 | 0.562 | 15 | 20585244 | NIPA2,NIPA1 |
| rs8040193 | C | 1.05 | 0.6146 | 15 | 20306549 | LOC390544 |
| rs7182576 | G | 0.98 | 0.6284 | 15 | 20546036 | CYFIP1 |
| rs1579821 | C | 1.02 | 0.6338 | 15 | 20501269 | CYFIP1 |
| rs3812924 | A | 1.02 | 0.6381 | 15 | 20599983 | NIPA2,NIPA1 |
| rs3751566 | C | 0.98 | 0.6918 | 15 | 20492111 | CYFIP1 |
| rs2304341 | C | 0.97 | 0.7614 | 15 | 20542471 | CYFIP1 |
| rs722411 | A | 1.01 | 0.7741 | 15 | 20475585 | CYFIP1 |
| rs7174982 | C | 1.01 | 0.8269 | 15 | 20517099 | CYFIP1 |
| rs7168653 | C | 0.99 | 0.8308 | 15 | 20516088 | CYFIP1 |
| rs3883043 | A | 1.01 | 0.8321 | 15 | 20777695 | LOC339005 |
| rs11636068 | A | 0.99 | 0.8639 | 15 | 20629449 | NIPA1,LOC400320 |
| rs8043036 | A | 1 | 0.9396 | 15 | 20434983 | CYFIP1 |
| rs1544285 | A | 1 | 0.9665 | 15 | 20405438 | TUBGCPS |
| rs4778298 | A | 1 | 0.974 | 15 | 20505022 | CYFIP1 |
| rs11263687 | G | 1 | 0.9838 | 15 | 20635884 | LOC400320,NIPA1 |
| rs2289816 | G | 1 | 0.9906 | 15 | 20506454 | CYFIP1 |

**Table 8.** Markers on the Illumina HumanHap300 within the 15q13.3 deletion. Shown are results of association of the markers with schizophrenia, and genes associated with the marker are also indicated. Data from 2,687 cases and 13,484 controls were used in the association analysis.

| Marker | Allele | OR | *P*-value | Chr | Pos. In Build 36 | Gene |
|---|---|---|---|---|---|---|
| rs1463408 | A | 0.88 | 0.0055 | 15 | 29243936 | |
| rs12915265 | C | 0.89 | 0.0089 | 15 | 30196358 | CHRNA7 |
| rs8038654 | C | 0.83 | 0.0095 | 15 | 30072156 | |
| rs10438342 | A | 0.91 | 0.0169 | 15 | 30189338 | |
| rs4779824 | C | 0.91 | 0.0174 | 15 | 29191586 | TRPM1 |

(continued)

| Marker | Allele | OR | *P*-value | Chr | Pos. In Build 36 | Gene |
|---|---|---|---|---|---|---|
| rs1223889 | A | 0.92 | 0.0243 | 15 | 29258764 | |
| rs2241494 | A | 0.92 | 0.0301 | 15 | 29155896 | TRPM1 |
| rs10152238 | A | 1.15 | 0.0377 | 15 | 30057610 | |
| rs1647992 | A | 0.91 | 0.0459 | 15 | 29245430 | |
| rs4779984 | A | 0.89 | 0.052 | 15 | 30302218 | |
| rs1863279 | A | 1.08 | 0.053 | 15 | 29282405 | |
| rs1477534 | A | 0.93 | 0.0539 | 15 | 29271979 | |
| rs4779536 | A | 1.08 | 0.0598 | 15 | 29574400 | C15orf16 |
| rs2651418 | A | 0.93 | 0.0642 | 15 | 30226573 | CHRNA7 |
| rs999876 | A | 0.93 | 0.0642 | 15 | 29272626 | |
| rs7173280 | C | 0.93 | 0.0759 | 15 | 29128656 | TRPM1 |
| rs1035706 | A | 1.1 | 0.0795 | 15 | 29130377 | TRPM1 |
| rs1978801 | A | 0.94 | 0.088 | 15 | 29294328 | LOC283710 |
| rs919001 | A | 1.07 | 0.0893 | 15 | 29144430 | TRPM1 |
| rs6493543 | G | 0.94 | 0.0923 | 15 | 29324788 | LOC283710 |
| rs8042511 | A | 1.16 | 0.0971 | 15 | 29222034 | |
| rs803534 | C | 0.94 | 0.1062 | 15 | 29215548 | |
| rs6493688 | A | 0.94 | 0.1139 | 15 | 29560167 | LOC400347.C15orf16 |
| rs4779937 | C | 1.06 | 0.1178 | 15 | 29975287 | |
| rs7162289 | C | 1.08 | 0.131 | 15 | 29373158 | |
| rs1672407 | C | 1.06 | 0.1344 | 15 | 29227096 | |
| rs12442141 | C | 1.16 | 0.1345 | 15 | 29266578 | |
| rs1672409 | A | 0.95 | 0.1446 | 15 | 29228600 | |
| rs1001555 | A | 1.12 | 0.1452 | 15 | 30060958 | |
| rs1514254 | A | 0.94 | 0.1456 | 15 | 29998226 | |
| rs1465778 | c | 1.06 | 0.146 | 15 | 29408613 | KLF13 |
| rs1580141 | A | 1.05 | 0.1981 | 15 | 29232062 | |
| rs3784595 | A | 1.07 | 0.2043 | 15 | 29129507 | TRPM1 |
| rs6493540 | A | 1.05 | 0.2115 | 15 | 29321882 | LOC283710 |
| rs1465779 | C | 1.07 | 0.2226 | 15 | 29397182 | KLF13 |
| rs1865873 | c | 1.05 | 0.2226 | 15 | 29303300 | LOC283710 |
| rs2278133 | A | 1.05 | 0.2238 | 15 | 29140680 | TRPM1 |
| rs8034505 | A | 1.05 | 0.227 | 15 | 29460239 | KLF13,LOC440262 |
| rs2241493 | C | 1.06 | 0.2295 | 15 | 29149644 | TRPM1 |
| rs8035668 | A | 0.94 | 0.2296 | 15 | 30178638 | CHRNA7 |
| rs2879262 | C | 0.95 | 0.2459 | 15 | 29344873 | |
| rs4417522 | C | 1.04 | 0.2735 | 15 | 29974412 | |

(continued)

| Marker | Allele | OR | *P*-value | Chr | Pos. In Build 36 | Gene |
|---|---|---|---|---|---|---|
| rs7179733 | C | 0.96 | 0.2814 | 15 | 30160985 | CHRNA7 |
| rs1459200 | A | 1.04 | 0.2991 | 15 | 29594877 | C15orf16 |
| rs2288242 | A | 1.05 | 0.3062 | 15 | 29117572 | TRPM1 |
| rs2338834 | C | 1.04 | 0.3069 | 15 | 29125017 | TRPM1 |
| rs890158 | C | 1.04 | 0.3097 | 15 | 29157929 | TRPM1 |
| rs12900301 | C | 0.95 | 0.3122 | 15 | 29619936 | C15orf16 |
| rs1503004 | A | 1.06 | 0.3286 | 15 | 29827425 | |
| rs3964705 | C | 0.96 | 0.3343 | 15 | 28822861 | LOC440261 |
| rs6494039 | C | 1.07 | 0.3401 | 15 | 29979194 | |
| rs12440180 | C | 1.04 | 0.3677 | 15 | 30072148 | |
| rs1606659 | A | 0.96 | 0.3731 | 15 | 30119745 | CHRNA7 |
| rs4779939 | C | 0.95 | 0.3764 | 15 | 29985165 | |
| rs4779814 . | C | 0.97 | 0.3814 | 15 | 29143717 | TRPM1 |
| rs7169523 | A | 0.96 | 0.3831 | 15 | 29250670 | |
| rs2137856 | A | 0.97 | 0.3882 | 15 | 30016646 | |
| rs7163696 | C | 0.96 | 0.3902 | 15 | 29313681 | LOC283710 |
| rs11630449 | C | 0.96 | 0.3953 | 15 | 29402033 | KLF13 |
| rs7163763 | A | 0.94 | 0.3977 | 15 | 29609507 | C15orf16 |
| rs953326 | C | 1.03 | 0.409 | 15 | 30004979 | |
| rs3784601 | C | 0.96 | 0.4097 | 15 | 29180766 | TRPM1 |
| rs3096464 | C | 1.03 | 0.4122 | 15 | 29256215 | |
| rs898212 | G | 1.03 | 0.4134 | 15 | 29579128 | C15orf16 |
| rs4779862 | C | 1.03 | 0.42 | 15 | 29420453 | KLF13 |
| rs4779759 | A | 1.03 | 0.4212 | 15 | 28751864 | |
| rs1456212 | A | 1.05 | 0.4215 | 15 | 29211346 | |
| rs3743234 | A | 1.03 | 0.4329 | 15 | 29126965 | TRPM1 |
| rs1459198 | A | 1.03 | 0.4337 | 15 | 29649740 | C15orf16 |
| rs12901022 | C | 0.97 | 0.4341 | 15 | 29100035 | TRPM1 |
| rs11638348 | A | 1.03 | 0.4352 | 15 | 29714219 | C15orf16 |
| rs1524878 | G | 1.03 | 0.437 | 15 | 28941992 | |
| rs2125615 | A | 1.03 | 0.4493 | 15 | 29587441 | C15orf16 |
| rs2046362 | C | 0.97 | 0.4581 | 15 | 28723577 | |
| rs8041717 | G | 1.03 | 0.4719 | 15 | 29063737 | FLJ20313 |
| rs4779816 | A | 0.97 | 0.473 | 15 | 29156415 | TRPM1 |
| rs3865090 | c | 1.06 | 0.4734 | 15 | 29319602 | LOC283710 |
| rs8026705 | A | 0.97 | 0.4835 | 15 | 29704566 | C15orf16 |
| rs16956382 | A | 1.07 | 0.4848 | 15 | 28986264 | KIAA1018 |

(continued)

| Marker | Allele | OR | *P*-value | Chr | Pos. In Build 36 | Gene |
|---|---|---|---|---|---|---|
| rs12439925 | C | 1.03 | 0.4852 | 15 | 29386793 | KLF13 |
| rs971330 | C | 1.03 | 0.4885 | 15 | 29538956 | LOC400347 |
| rs7174744 | A | 0.97 | 0.4961 | 15 | 28971039 | KIAA1018,LOC388104 |
| rs12442622 | A | 1.03 | 0.4971 | 15 | 30045195 | |
| rs11071179 | C | 0.97 | 0.4975 | 15 | 29635750 | C15orf16 |
| rs7175258 | A | 1.04 | 0.518 | 15 | 29484934 | LOC440262 |
| rs2337980 | C | 0.98 | 0.5194 | 15 | 30231488 | CHRNA7 |
| rs10519712 | A | 1.03 | 0.523 | 15 | 29997162 | |
| rs4779889 | G | 1.03 | 0.5245 | 15 | 29601495 | C15orf16 |
| rs7169662 | A | 0.98 | 0.5292 | 15 | 29438608 | KLF13 |
| rs11632955 | C | 0.98 | 0.54 | 15 | 29336409 | |
| rs9672615 | A | 1.03 | 0.5436 | 15 | 30298847 | |
| rs8025698 | C | 1.02 | 0.5444 | 15 | 29186010 | TRPM1 |
| rs7175507 | C | 1.02 | 0.5606 | 15 | 30007740 | |
| rs6493623 | A | 1.03 | 0.5622 | 15 | 29444540 | KLF13 |
| rs4779809 | C | 0.98 | 0.5771 | 15 | 29131323 | TRPM1 |
| rs12439621 | C | 1.05 | 0.5937 | 15 | 30096476 | CHRNA7 |
| rs12442954 | A | 0.98 | 0.5955 | 15 | 30029658 | |
| rs1060493 | G | 1.02 | 0.602 | 15 | 29303762 | LOC283710 |
| rs7402321 | C | 1.02 | 0.6061 | 15 | 30207700 | CHRNA7 |
| rs16956762 | A | 0.98 | 0.614 | 15 | 29539275 | LOC400347 |
| rs964925 | C | 1.02 | 0.6145 | 15 | 29093271 | TRPM1 |
| rs2337233 | c | 0.98 | 0.6206 | 15 | 30094507 | CHRNA7 |
| rs7182946 | G | 0.98 | 0.6232 | 15 | 29182160 | TRPM1 |
| rs7178760 | C | 0.97 | 0.6243 | 15 | 29318665 | LOC283710 |
| rs17228178 | C | 0.98 | 0.6295 | 15 | 29257220 | |
| rs6493352 | C | 1.02 | 0.6331 | 15 | 29021356 | FLJ20313,KIAA1018 |
| rs11070871 | C | 0.97 | 0.6503 | 15 | 29299944 | LOC283710 |
| rs11636101 | A | 0.98 | 0.6721 | 15 | 30061449 | |
| rs1524876 | C | 0.98 | 0.6726 | 15 | 29050564 | FLJ20313 |
| rs4779948 | C | 0.98 | 0.673 | 15 | 30046352 | |
| rs8042404 | A | 0.98 | 0.6904 | 15 | 29467308 | KLF13,LOC440262 |
| rs2113945 | C | 0.98 | 0.6905 | 15 | 29111823 | TRPM1 |
| rs7174211 | A | 0.98 | 0.693 | 15 | 29425288 | KLF13 |
| rs1474380 | A | 0.98 | 0.6997 | 15 | 29056527 | FLJ20313 |
| rs2338679 | A | 0.99 | 0.7029 | 15 | 29608133 | C15orf16 |
| rs13329490 | A | 1.02 | 0.7102 | 15 | 30195523 | CHRNA7 |

(continued)

| Marker | Allele | OR | *P*-value | Chr | Pos. In Build 36 | Gene |
|---|---|---|---|---|---|---|
| rs4321165 | A | 0.98 | 0.7117 | 15 | 29863575 | LOC440263 |
| rs12323980 | C | 0.97 | 0.7147 | 15 | 29363969 | |
| rs4238558 | A | 0.99 | 0.7193 | 15 | 29933027 | |
| rs11636160 | C | 0.98 | 0.7239 | 15 | 29489142 | LOC440262 |
| rs4268714 | A | 0.99 | 0.7304 | 15 | 29462745 | KLF13,LOC440262 |
| rs965435 | C | 1.02 | 0.74 | 15 | 30104501 | CHRNA7 |
| rs7167632 | A | 1.01 | 0.7425 | 15 | 29935438 | |
| rs4779520 | C | 0.99 | 0.7456 | 15 | 29452735 | KLF13 |
| rs8028220 | A | 1.01 | 0.7461 | 15 | 29214684 | |
| rs12441324 | A | 1.01 | 0.7535 | 15 | 28830254 | LOC440261 |
| rs7182547 | C | 1.01 | 0.7558 | 15 | 29084964 | FLJ20313,TRPM1 |
| rs9302175 | C | 0.99 | 0.7596 | 15 | 29530870 | LOC400347 |
| rs2289126 | G | 0.99 | 0.771 | 15 | 29308957 | LOC283710 |
| rs798081 | A | 0.98 | 0.7775 | 15 | 28910527 | LOC390561 |
| rs2611605 | C | 0.99 | 0.7856 | 15 | 30228925 | CHRNA7 |
| rs11070619 | C | 1.02 | 0.7926 | 15 | 28896081 | LOC390561 |
| rs753636 | A | 0.98 | 0.7935 | 15 | 29478345 | LOC440262 |
| rs1514260 | A | 1.01 | 0.7981 | 15 | 30086242 | |
| rs1567885 | A | 1.01 | 0.8297 | 15 | 30088094 | |
| rs2063722 | A | 0.99 | 0.8311 | 15 | 30083665 | |
| rs10519688 | C | 0.99 | 0.8362 | 15 | 29921270 | |
| rs10519726 | A | 0.99 | 0.8404 | 15 | 29109167 | TRPM1 |
| rs12594231 | C | 0.99 | 0.854 | 15 | 29963596 | |
| rs17816055 | C | 1.01 | 0.8543 | 15 | 29619386 | C15orf16 |
| rs4779527 | C | 1.01 | 0.8566 | 15 | 29523383 | LOC440262,LOC400347 |
| rs1524877 | C | 0.99 | 0.8618 | 15 | 29058472 | FLJ20313 |
| rs2293314 | A | 0.99 | 0.868 | 15 | 28997943 | KIAA1018 |
| rs1035707 | C | 1.01 | 0.8721 | 15 | 29172089 | TRPM1 |
| rs2081455 | C | 0.99 | 0.8741 | 15 | 29210624 | |
| rs6493741 | C | 1.01 | 0.8747 | 15 | 29609127 | C15orf16 |
| rs11638086 | A | 0.99 | 0.8765 | 15 | 28853522 | LOC440261,LOC390561 |
| rs9672180 | C | 1.01 | 0.8818 | 15 | 30300468 | |
| rs1088475 | C | 1.01 | 0.888 | 15 | 28927992 | LOC390561 |
| rs2219507 | A | 1.01 | 0.8928 | 15 | 29646927 | C15orf16 |
| rs2873 | A | 1 | 0.9116 | 15 | 29018547 | FLJ20313.KIAA1018 |
| rs2339046 | A | 1.01 | 0.9146 | 15 | 29059962 | FLJ20313 |
| rs798104 | C | 1.01 | 0.9313 | 15 | 28894118 | LOC390561 |

(continued)

| Marker | Allele | OR | *P*-value | Chr | Pos. In Build 36 | Gene |
|---|---|---|---|---|---|---|
| rs3784589 | A | 0.99 | 0.9331 | 15 | 29082006 | FLJ20313,TRPM1 |
| rs8027035 | C | 1.01 | 0.9334 | 15 | 30149996 | CHRNA7 |
| rs1392808 | G | 1 | 0.9471 | 15 | 30198807 | CHRNA7 |
| rs4779556 | C | 1 | 0.9564 | 15 | 29960537 | |
| rs4779910 | C | 1 | 0.9612 | 15 | 29734334 | C15orf16 |
| rs1075232 | A | 1 | 0.9619 | 15 | 29528508 | LOC400347 |
| rs1378847 | C | 1 | 0.9674 | 15 | 29234640 | |
| rs12898600 | A | 1 | 0.9694 | 15 | 29816985 | |
| rs6494223 | C | 1 | 0.9697 | 15 | 30183749 | CHRNA7 |
| rs1983459 | A | 1 | 0.9703 | 15 | 28996041 | KIAA1018 |
| rs7178637 | C | 1 | 0.9774 | 15 | 29665644 | C15orf16 |
| rs4779794 | A | 1 | 0.9844 | 15 | 28984856 | KIAA1018 |
| rs905426 | A | 1 | 0.9955 | 15 | 29870041 | |

**Table 9.** Diagnosis, family history, age of onset, response to neuroleptics based on available

| Case ID | Diagnosis | Family history | Ageof onset | Gender | Response | Other |
|---|---|---|---|---|---|---|
| Munich 1 | DSMIV: 295 | monozygotic twin brother with unknown psychiatric diagnosis | 24 | Male | yes | Not MR, very aggressive as child |
| Munich 2 | DSMIV: 295 | no | 25 | Female | yes | Not MR |
| Munich 3 | DSMIV: 295 | mother depression | 32 | Male | yes | Not MR |
| Munich 4 | DSMIV: 295 | no | 17 | Male | yes | Not MR |
| Munich 5 | DSMIV: 295 | no | 23 | Female | yes | Not MR |
| Bonn 1 | | | | Male | | Not MR |
| Iceland 1 | | no | 39 | Male | yes | Not MR |
| Iceland 2 | | no | 29 | Female | yes | Not MR |
| Iceland 3 | RDC: 126.3 | Yes, schizophrenia | 33 | Male | Yes | Not MR |
| Iceland 4 | RDC: 126 | Yes, schizophrenia | 16 | Female | Yes | Not MR |
| Scotland 1 | DSMIV: 295 | No | 37 | Female | Yes | Not MR, borderline leaming difficulties |
| Scotland 2 | DSMIV: 295 | | 23 | Female | Yes | Not MR |
| Scotland 3 | DSMIV: 295 | ? | 32 | Female | Yes | Not MR |

(continued)

| Case ID | Diagnosis | Family history | Age of onset | Gender | Response | Other |
|---|---|---|---|---|---|---|
| Scotland 4 | DSMIV: 295 | No | 22 | Male | Yes | Not MR |
| Scotland 5 | DSMIV: 295 | No | 31 | Female | Yes | Not MR, Nervous breakdown at 22 |
| Scotland 6 | DSMIV: 295 | No | 15 | Male | Yes | Not MR, heroin Not MR, addiction |
| Scotland 7 | | Yes, schizophrenia | 24 | Female | | |
| England 1 | | Yes, schizophrenia (co-twin) | 25 | Male | Yes | Not MR, No drug abuse, primarily |
| Denmark 1 | ICD10: F20 | No | 26 | Male | No | Not MR |
| Denmark 2 | ICD10: F20 | No | (27, afa) | Male | | Not MR |
| Denmark 3 | ICD10: F20 | No | 21 | Male | | Not MR, cannabis abuse |
| Denmark 4 | ICD10: F25 | Yes | 16 | Male | Yes | Not MR |
| Holland 1 | DSMIV: 295.30 | | 19 | Female | | Not MR |
| Holland 2 | DSMIV: 295.30 | No | 20 | Male | | Not MR |
| Holland 3 | DSMIV: 295.30 | No | 20 | Male | | Not MR |
| Holland 4 | DSMIV: 295.30 | | 22 | Male | | Not MR |
| MR=mentally retarded. | | | | | | |

**Table 10.** Diagnosis, family history, age of onset, response to neuroleptics based on available records and learning ability in cases carrying the 15q13.3 deletion associating with schizophrenia.

| Case ID | Diagnosis | Family history | Age of onset | Gender | Response | Other |
|---|---|---|---|---|---|---|
| Munich 1 | DSMIV: 295 | Yes | 24 | Male | yes | Not MR |
| Iceland 1 | | Yes | 30 | Male | Yes | Not MR |
| Scotland 1 | DSMIV: 295 | | 20 | Male | Yes | Not MR , IQ 83 |
| Norway 1 | DSMIV: 295.4 | | (31, afa) | Female | Yes | Not MR, No cannabis use or head injury |
| Holland 1 | DSMIV: 295.20 | | 23 | Male | | Not MR |
| Holland 2 | DSMIV: 295.30 | yes | 39 | Female | | Not MR |

(continued)

| Case ID | Diagnosis | Family history | Age of onset | Gender | Response | Other |
|---|---|---|---|---|---|---|
| Holland 3 | DSM IV: 295.30 | | | Male | | Not MR |
| MR=mentally retarded. | | | | | | |

**EXAMPLE 2.** Recurrent duplications of chromosome 16p13.1 associated with schizophrenia

A sub-microscopic duplication on chromosome 16p13.1 was recently found in two unrelated patients diagnosed with autism (Ullman et al., Human Mutation 28:674-682,(2007)). The duplication encompassed an interval of 1.5 Megabases (Mb), spanning positions 14.89 to 16.39 Mb (NCBI Build 36). A third duplication was identified by quantitative PCR in a second Australian cohort of 112 patients. Two of the duplications were familial, and in one family a severely autistic brother also carried the duplication. One of the brothers was continuously hyperactive, destructive and aggressive, whereas the younger brother was passive and easy to manage. Other carriers included a sister, who had learning difficulties (sister) and a mother who had learning difficulties coupled with obsessive compulsive disorder. The two deletion patients had severe mental retardation. The former was de novo; the latter had a mildly affected carrier mother.

The chromosome 16p13.1 duplication/deletion interval is located in a region previously reported linked to bipolar disorder (McInness et al., Proc. Natl. Sci. USA.493(23):13060-13065 (1996), Ewald et al., Mol Psychiatry 7(7): 734-744 (2002), Ekholm et al., Hum. Mol. Genet. 12(15):1907-1915 (2003), Kassem et al., Am. J. Psychiatry, 163(10):1099-104(2006) and to puerperal psychosis (Jones et al., Am. J. Psychiatry. 164(2):248-258 (2007)). Furthermore, in a genome wide scan of 458 Finnish schizophrenia families, linkage was reported to DISC1 locus (Ekelund et al., Mol. Psychiatry. 9(11): 1037-1041(2004)). When these families were later conditioned for a risk haplotype spanning intron 1 and exon 2 of the DISCI gene, linkage was found to 16p13.1 (lod 3.17)(Hennah et al., Hum. Mol. Genet. 16(5):453-462 (2007)). The duplicated/deleted region contains the gene coding the DISC1 binding protein NDE1. The authors found significant allelic association between NDE1 and schizophrenia. However this association was not confirmed in a recent Japanese study (Numata S et al., Schizophr. Res. 99(1-3): 367-369 (2008)). Finally, association was recently reported between NDE1 and schizophrenia, when schizophrenia cases and controls were conditioned by the presence of Cys residue at codon Ser 704Cys of DISC1 gene. (Burdick et al., Hum. Mol. Genet.(2008)).

In the present study, we assessed association of CNVs in the 16p13.1 region with schizophrenia as part of a genome-wide scan using the Illumina HumanHap300 and Human Hap550 and Affymetrix SNP 6.0 genotyping arrays in a sample of 3,843 schizophrenia patients and 34,602 controls from seven European populations (Iceland, Finland, Germany, Holland, Norway, Italy and the UK).

**RESULTS**

We limited our search on 16p13.1 to the region between Mb 14.66 and 18.70 Mb (Build36). For comparison, the duplications and deletions reported by Ullman et al. (Human Mutation 28:674-682- (2007)) span Mb 14.89-16.39 (NCBI Build36). We subdivided the region into three single copy sequence intervals which we called 1,2 and 3. Each was flanked by substantial low copy repeats (LCRs) extending approximately 15.23-15.38;16.38-16.53 and 18.19-18.34 respectively.

Figure 6 displays the region on USCC browser and gives examples of the duplications and deletions we observed. Duplicated intervals are identified by the numbers 1, 2 and 3. Interval 1 is a small island of single copy sequence embedded in a large cluster of LCRs. Table 11 lists the duplications and deletions found in our series plus country of origin. None were found in cases and controls from the UK samples from Institute of Psychiatry (n=108 and 92), Italy (n=86 and 92), Finland (n=191 and 200) and Norway (n=245 and 272). Accordingly, these samples were not included in Cochrane-Mantel-Haentzel analysis.

We found a three fold excess of duplications and deletions in cases compared to controls (Table 12). Duplications were present in 0.36% of schizophrenia cases versus 0.08% controls ($P < 0.0032$). Deletions were present in 0.12% cases and 0.04% controls ($p > 0.05$). Due to varying geographical origin of the samples we analysed the data for association using Cochrane Mantel Haentzel to correct for stratification. Total duplications were significantly associated with schizophrenia ($p < 0.0045$). When analysis was restricted to duplications containing intervals 1 and 2, the significance increased further ($p < 0.00018$). Duplications of intervals 1 plus 2 were present in 4 male and 2 female Scottish cases, 2 male Icelandic and 3 Dutch male cases; duplications were found in 12 female and 6 male Icelandic controls, and 2 Dutch male controls. Odds ratio was 8.50 (males) and 3.63 (females). The two Icelandic cases were independently ascertained and are included in the analysis as separate probands. However, when genealogical analysis was later performed unexpectedly we found that the two schizophrenia cases were second degree relatives. Other carriers in the family included single cases of alcoholism (under treatment), dyslexia and ADHD.

A 1 plus 2 deletion was present in 3 German schizophrenia cases, one German and ten Icelandic controls ($P>0.05$) and a 2 plus 3 deletion was present in one Scottish schizophrenia case, two German and two Icelandic controls ($P>0.05$).

We tested allelic association for all SNP markers on the Illumina microarrays that spanned the 16p13.1 region in 2,687 schizophrenia cases and 13,484 controls. One marker, rs228.3508 was significantly associated ($p<1.5E-05$) and remained significant after locus wide correction with a *P*-value of 0.0043. This marker is located within intron of ABCC6 gene.

In view of the report (Burdick et al *Hum. Mol. Genet*. 2008) of association between NDE1 and schizophrenia when schizophrenia cases were stratified by the presence of Cys residue at Ser 704Cys of DISC1 gene, we also conditioned our schizophrenia cases. The DISC1 Ser704Cys SNP, rs821616, is not on the Illumina 317K chip. However, a SNP that has $r2 = 1$ with rs821616 (i.e., a perfect surrogate) in the CEU, rs821596, was present. We therefore used rs821596 to divide the schizophrenia cases into

Cys-carrier and non carrier groups, and then looked for allelic association with SNPs at the NDE1 locus in the two groups. None were significantly associated. The data for 51 SNPs in, or within 200 kilobases of NDE1 for the Cys carrier and non carrier groups are in supplementary Table 12.

Since the majority of the duplicated cases were Scottish in origin, we examined the haplotype background of the duplicated regions. The CNV occurred on a different haplotype background in each individual. Indeed none of the non Icelandic individuals for which we had genotype data had a CNV on same haplotype background as any of the Icelandic cases. This suggests there was no founder, and each of the events is likely to have arisen independently. Within Iceland itself, for each of the CNV duplication and deletion subtypes found in more than one individual, there was no founder mutation. There were enough individuals in the Icelandic population with 1 plus 2 duplications to look at clustering patterns. Clustering occurred at a rate of 3 to 4 fold less than expected if the duplications were selectively neutral. However the maximum meiotic distance between individuals with the 1+2 duplication was longer than with the other 16p13.1 CNV categories we looked at here or among the deletions associated with schizophrenia on other chromosomal regions we have examined.

## DISCUSSION

We have examined chromosome 16p13.1 region for recurrent duplications and deletions in a large set of European schizophrenia cases and controls. We find a three to four fold over-representation of both duplications and deletions in schizophrenia cases compared to controls. The over-representation of duplications is statistically significant ($P<0.0045$). The great majority of duplications and deletions we found using Illumina micro-arrays are identical to those reported by Ulmann et al (Human Mutation 28:674-682(2007)) using BAC tiling pathway. They span the same 1.5 Mb region that includes intervals we call 1 and 2. However a minority of duplications and deletions in our cases and controls have breakpoints spanning intervals 2 and 3. These have not been previously reported.

The breakpoints for both types of deletion/duplication are located in areas with high LCR content. The region appears to be a region of genomic instability (Shaw and Lupski, Hum. Mol. Genet. 13: Spec No 1:R57-64 (2004)). There are several paralogous repeats in the region. The repeats are in the same orientation, and non allelic homologous recombination (NAHR) between these LCRs seems to be the most likely explanation for the recurrence of these rearrangements and for their identical size. Three inversion polymorphisms have previously been described in the 16p13.1 region (Tuzun et al., Nat. Genet.37(7):727-732 (2005) and Database of Human variants Zhang et al., Cytogen Genome Research (2006)). A large duplication in a patient with mental retardation has also been reported (Sharp et al., Nat Genet. Sep;38(9):1038-1042(2006)), and a smaller de novo duplication (Kriek et al., J. Med. Genet. 41(4):249-255 (2004)). However in the latter report, since the father also had learning difficulties interpretation is problematic. A much larger duplication (8Mb) of the region has also been reported in two unrelated patients with autistic features (Finelli et al., J. Med. Genet. 41(7):e90 (2004)).

Our most striking finding is the increased risk of schizophrenia associated with duplications at the 16p13.1 locus. Recurrent deletions at several loci have now been reported significantly associated with schizophrenia but to date duplications associated with schizophrenia have mostly been isolated case reports. This is the first locus to our knowledge where there is statistically significant evidence of association between a duplication and schizophrenia. The different sizes of the duplications and deletions we have identified at the 16p13.1 locus presents difficulties when it comes to assessing association with schizophrenia. Statistically, we have used the straightforward approach of counting all duplications as equivalent events, and only then tried to condition on those duplications that have the same breakpoints as the original ones reported by Ullman et al.(Human Mutation 28:674-682 (2007)). Although caution must be exercised when interpreting results from such a small number of cases, there are several grounds for thinking that our findings are genuine. First given the rarity of the duplications the association with schizophrenia is remarkably statistically significant, especially if the 1 plus 2 duplications are considered separately (P<0.0045 and P< 0.00018 respectively). Also identical duplications at the 16p13.1 locus have already been associated with autism. What is more, three of the schizophrenia duplication cases had an early onset of illness (12, 17 and 19 years) and in this respect resembled the 16p13.1 deletion cases where three of the five cases also had early onset of illness (15,17 and 18 years) see Table 13. This seems unlikely to be due to chance. The duplication co-segregates with schizophrenia in the Icelandic pedigree and also with other neuropsychiatric disorders including ADHD. This is not unexpected since an overlap of phenotypic features between autism and ADHD has been extensively reported, and individuals with ADHD are at increased risk of schizophrenia. (Amminger et al., Am. J. Psychiatry156(4):525-530(1999), Keshavan et al., Schizophr. Res.59(1):85-92 (2003), Oner et al., Schizophr. Res.76(2-3):293-299(2005)). It is also perhaps noteworthy that nine of the eleven 1 plus 2 schizophrenia cases were males. This cannot be accounted for by the excess of males in the schizophrenia series under investigation, and resembles the sex ratios observed in autism. The duplications at this locus appear to be under negative selection. Cluster analysis of the 1 plus 2 duplication events in the Icelandic population finds considerably fewer clusters than if the duplications were selectively neutral. This negative selection is not as pronounced as for the high penetrant recurrent deletions we have recently described at other loci but it is present nevertheless. It is consistent with the lower odds ratio we also observe. Finally the duplicated region contains two strong candidate genes over- or under-expression of one or both of which at key stages of neurodevelopment could predispose to autism and/or schizophrenia.

NTAN1 gene is located in the small island of single sequence called interval one. It encodes an N-terminal asparagines amidase that has been implicated in social behaviour and memory. Over-expression of NTAN1 leads to reduction in MAP2 protein expression through the ubiquitinproteasome pathway. Reduced expression of MAP2 may be a useful marker for diagnosis of schizophrenia and bipolar disorder *in vivo* (Whitaker-Azmitia et al., Neuropsychopharmacology12(3):269-272(1995); Mazer et al., Brain Res.760(1-2): 68-73(1997)) and *in vitro* (Marx et al.,Biol. Psychiatry50(10):743-749(2001); Bouras et al., ActaNeuropathol.102(4): 373-379(2001)). Mutations of UBE3 aubiquitinprotein ligasegene, cause Angelman syndrome, a neurodevelopmental disorder with associated autistic features. Recently, decreased expression of genes involved in ubiquitin metabolism has been reported in dorsal prefrontal cortex and laser sorted dentate granule neurons from schizophrenia patients (Middleton et al., J.Neurosci.22(7): 2718-2729 ( 2002); Vawter et al., Schizophr. Res.58(1):11-20(2002); Altar et al., Biol. Psychiatry58(2):85-96(2005)). The neuronal ubiquitinproteasome system controls the assembly, connectivity, function and signaling of the synapse, including the turnover of pre and postsynaptic proteins (Hedge and Antonio, Neuroscience3:854-861(2002); Collins CA and Di Antonio A, Current Opinion Neurobiology. 17:35-42 (2007)). Mice with disrupted NTAN1 gene show less locomotion in an open field and impairment of several spatial memory tasks (Kwon et al., Mol. Cell Biol.20(11):4135-4148 (2000); Balogh et al.,Learn Mem.7(5): 279-286 (2000)).

NDE1 and NDEL are highly homologous genes involved in brain development, neuronal proliferation, migration and synapse formation. They encode for proteins that biologically interact with DISC1 and LISI proteins, with NDE1 appearing to be interchangeable with its homolog NDEL, except that NDE1 is expressed earlier in development. The LIS1/NDEL pathway is involved in brain development and regulated by RELN, another candidate gene for schizophrenia. Mutations in RELN/ LIS1 pathway cause lissencephaly. NDE1 null mice are viable and display microcephaly with thinning cortical layering and reduced numbers of neurones. Interestingly two out of three reported autism cases with the duplication had increased head circumference. Mice display defects in neuronal proliferation and neuronal migration. NDE1 protein directly interacts with DISC1 protein at the C terminal end that is distal to the truncating mutation reported in the Scottish DISC1 translocation family. (Kamiya et al., Hum. Mol. Genet. 15(22):3313-3323 (2006)). Phenotypes in this family include schizophrenia, schizoaffective disorder, major depression and severe adolescent conduct disorder. (St Clair et al., Lancet336(8706):13-16 (1990); Blackwood et al., Am. J. Hum. Genet. 69(2):428-433 (2001)). Sachs et al. (Am. J. Hum. Genet. 69(2):428-433 (2005)) reported a frameshift mutation in DISC1 gene in an American pedigree. In addition to cases of schizophrenia and major depression the pedigree contains two cases of autistic spectrum disorder and two cases of mental retardation. The DISC1 gene has also recently been found associated with autism spectrum and Asperger's syndrome (Kilpinen et al., Mol. Psychiatry 13(2):187-196 (2008); Since DISCI is known to inhibit NDE1/NDEL activity,the duplications we report here might therefore be expected to have a similar biological effect as the truncating mutation associated with schizophrenia in the Scottish family, of increasing NDE1 activity.

All duplications and deletions in our study involve interval 2 that harbours the NDE1 gene and this makes dysregulation of NDE1 expression the most parsimonious explanation for the increased risk of the schizophrenia phenotypes we associate with the region. On the other hand the strongest association is with duplication cases that also involves interval 1. It is possible that combined changes in expression of NTAN1 and NDE1 increase susceptibility over changes in expression of NDE1 alone. We found evidence of allelic association with only one marker, located in an intron of ABCC6, spanning the region present on the Illumina micro-array. We were unable to replicate association with NDE1 when our samples were conditioned DISC1 ser704cys and cys704cys carrier status. Further examination of the region will be necessary to determine if it contains rare variants that increase risk of schizophrenia. It will also be necessary to analyse mRNA and protein levels using relative allelic expression to try to define which individuals may be able to compensate for dosage gain/ loss, for example through a high/low expressing residual copy of the gene or other modifying loci. These, along with as yet unidentified environmental influences, perhaps acting epigenetically eg on RELN gene, may help to determine the penetrance and expressivity of the phenotypes observed at the locus.

Further work is required before the clinical implications of our findings become clear. On the one hand the data strongly suggest that recurrent duplications at 16p13.1 locus increase risk of schizophrenia. They also strengthen the hypothesis that there are shared genetic risk factors between schizophrenia and autism. However the odds ratios, even for the 1 plus 2 duplications, are substantially less that the increased risks we have observed for recurrent deletions on chromosomes 1, 15 and 22. Whether the lesser odds ratio we observe for duplications is a feature of the 16p13.1 locus itself, or it is part of a broader rule than recurrent duplications are generally less penetrant than recurrent deletions remains to be determined. The 16p13.1 duplications we observe are rare, at a rate of about 3 or 4 per 1000 cases, and, from the control population In the present study, about 0.08 % of live births. This makes it difficult to obtain precise measurements of schizophrenia and/or autism risk. Analysis of CNV data from sets of cases and controls considerably larger than the sets we report in this paper, which itself to date is one of the largest assembled, will be required. These and many other questions will need to be answered before the exciting findings arising from CNV analysis can be used in clinical practice for diagnostics, disease classification or genetic testing.

## MATERIALS AND METHODS

*Samples*

A total of 3843 affected and 34602 controls from six European populations were successfully examined for CNVs at the two loci studied here; 1435 schizophrenia patients and 28554 control individuals from the Iceland, Scotland, Germany, England, Italy and Finland (The SGENE sample; http://www.SGENE.eu), additional 866 schizophrenics and 856 controls from Aberdeen, Scotland and Munich, Germany which have been collected with support from GSK and were genotyped at Duke University, 491 affected and 881 controls from Bonn, Germany, genotyped at Bonn University and 806 Dutch cases and 4039 controls. The Icelandic sample consists of 648 schizophrenics and 27747 controls. A further 5630 genotyped samples were examined but excluded from association analysis due to other psychiatric disorders (autism, bipolar disorder, ADHD, dyslexia and alcoholism) and/or first degree relationships to schizophrenic patients. Patients and controls were all Icelandic and diagnoses were assigned according to Research Diagnostic Criteria (RDC) (Spitzer et al., Arch. Gen. Psychiatry 35, 773-782 (1978) ) through the use of the lifetime version of the Schizophrenia and Affective Disorders Schedule (SADS-L) (Spitzer, New York State Psychiatric Institute, New York, (1977)). The Icelandic controls were chosen from persons who have participated in other genetic studies at deCODE Genetics. The Scottish sample is comprised of 661 schizophrenia cases and 665 controls. All participants self-identified as born in the British Isles (95% in Scotland) and met DSMIV and ICD-10 (American Psychiatric Association, 1994; WHO, 1994 48) criteria for schizophrenia. Diagnosis was made by OPCRIT (McGuffin et al., . Arch. Gen. Psychiatry764-770, (1991)). Controls were volunteers recruited through general practices in Scotland, and subjects with major mental illness were excluded. The Munich sample consisted of 611 Caucasian cases and 612 Caucasian controls. Cases diagnosed with DSMIV schizophrenia were ascertained from the Munich area in Germany. Diagnosis was made according to DSMIV criteria using the Structured Clinical Interview for Axis I DSM-IV Disorders (SCID) (First et al., Biometrics Research, New York, 1994). The controls were unrelated volunteers randomly selected from the general population of Munich. The Finnish sample consisted of 191 schizophrenics and 200 regionally selected controls that had no medical history of schizophrenia. Diagnosis was according to the criteria of Diagnostic and Statistical Manual of Mental Disorders, 4th edition (DSM-IV). The sample from the UK consisted of cases (n = 104) and controls (n = 95) who were unrelated white European Caucasians. All patients were interviewed with the Schedule for Affective Disorders and Schizophrenia Lifetime Version or the Item Group Checklist (IGC) of the Schedule for Clinical Assessment in Neuropsychiatry (SCAN) (WHO, Schedules for Clinical Assessment in Neuropsychiatry (SCAN) Manual, 1994) and diagnosed according to ICD-10 RDC. UK controls were unrelated individuals with no history of major mental Illness. Diagnosis of the 85 Italian cases from the local population of South Verona was also by IGC and ICD-10 RDC for schizophrenia, and the 91 controls were unrelated healthy volunteers randomly selected from the same population. The Bonn sample is comprised of 491 patients and 881 controls. Patients were recruited from consecutive hospital admissions and were all of German descent. In patients, lifetime best estimate diagnoses according to DSM-IV criteria were based on multiple sources of information including structured interview with the SCID (First *et al.,* 1994) or SADS-L (Endicott and Spitzer, 1978), the OPCRIT (McGuffin *et al.,* 1991), medical records, and the family history. Best estimate diagnoses were obtained from at least two experienced psychiatrists/psychologists. Controls were derived from two German population-based cohorts, PopGen (N=492) and Heinz Nixdorf Recall (N=383). The Norwegian sample included 245 patients who had been recruited to the TOP study from all the psychiatric hospitals in the Oslo area. The patients were diagnosed according to Structural Clinical Interview for DSM-IV (SCID. The healthy control subjects (N=272) were randomly selected from the same catchments area as the patient groups. Only subjects born in Norway, all of Caucasian origin, were contacted by letter and invited to participate.

Ethical approval was obtained from the local Ethics Committees. All participants gave written informed consent. One part of the Dutch sample consisted of 806 patients and 706 controls. Inpatients and outpatients were recruited from different psychiatric hospitals and institutions throughout the Netherlands, coordinated via academic hospitals in Amsterdam, Groningen, Maastricht and Utrecht. Detailed medical and psychiatric histories were collected, including the Comprehensive Assessment of Symptoms and History (CASH), an instrument for assessing diagnosis and psychopathology. To exclude related patients and controls, all subjects were fingerprinted (Illumina DNA panel, 400 SNPs). Only patients with a DSM-IV diagnosis of schizophrenia were finally included as cases (295.xx). All patients and controls were of Dutch descent, with at least three out of four grandparents of Dutch ancestry. The controls were volunteers and were free of any psychiatric history. Ethical approval was obtained from the local Ethics Committees. All participants gave written informed consent. The remaining Dutch control sample consisted of 3,333 individuals collected by the Radboud University Nijmegen Medical Centre (RUNMC) for genetic studies. All 3,333 participants used in the present study are of self-reported European descent. The study protocol was approved by the Institutional Review Board of Radboud University and all study subjects gave written informed consent.

The SGENE samples were typed on the HumanHap300 BeadArray™ (Illumina, San Diego, USA) at deCODE genetics. The additional samples from Aberdeen and Munich were typed at Duke University in collaboration with GlaxoSmithKline on HumanHap550v3 and HumanHap300 BeadArray™ (Illumina, San Diego, USA, respectively. The samples from Bonn were typed at Bonn University on HumanHap550v3 BeadArray™ (Illumina, San Diego, USA). The Dutch samples from Utrecht University were genotyped at the University of California, Los Angles, on HumanHap550v3 BeadArray™ (Illumina, San Diego, USA). Theremaining Dutch samples were genotyped at deCODE genetics on HumanHap300 BeadArray™ (Illumina, San Diego, USA). The Norwegian samples were genotyped on AffymetrixGeneChip(r) GenomeWide SNP 6.0 array and analyzed using the Affymetrix Power Tools 1.8.0. Samples with Contrast QC below 0.4 were excluded as recommended by the manufactory.

*CNV detection*

DosageMiner software developed at deCODE genetics and QuantiSNP software developed at Wellcome Trust Centre for Human Genetics and the University of Oxford (http://www.well.ox.ac.uk/QuantiSNP/) was used to identify deletions and duplications within the region reported by Ullman *et al.* (2007) in all samples except the Norwegian samples Dosage miner, described in detail elsewhere (Stefansson *et al.,* submitted), uses the intensities from SNP probes on the Illumina microarrays to estimate copy number of genomic regions, and models factors such as SNP effect, sample effect and GC-content the in neighbouring region to normalise the intensities. The software then automatically registers SNP loci where intensities fall above or below an empirical threshold.

The QuantiSNP program relies on an Objective Bayes Hidden-Markov Model to estimate copy number variations (Colella and Yau et al., Nucleic Acids Research 2007). In this model, the hidden states denote the unknown copy number at the inspected SNPs. Genotype data was used to compute different states. Based on the ratio of fluorescent dye ratios (logR) and stretches of, the algorithm computes a Bayes factor that is used to calibrate the model to a fixed type I (false-positive) error rate. A Bayes factor threshold of 10 is considered as a promising value for the possible presence of a CNV. Usually, such values occur when 5-10 consecutive SNPs are deleted/duplicated. Differences in GC base pairs may result in biased hybridization behaviour of SNP probes bearing the risk of miscalling genotypes. To normalize for this, QuantiSNP assigns a locus-specific GC value to each probe. All potential CNVs detected by both softwareswere subsequently visually inspected and confirmed.

*Association analysis*

A Cochrane-Mantel-Haenzsel analysis assuming common odds ratios was performed, stratifying samples by country of origin to take account of the possible effect of geographical variation on the results of the analysis.

Table 11. Duplications and deletions of 16p 13.1 in European populations

| | **Iceland** | | **Scotland** | | **Germany** | | **Holland** | |
|---|---|---|---|---|---|---|---|---|
| **Status No of cases** | **Scz (648)** | **Ctrl (27747)** | **Scz (661)** | **Ctrl (665)** | **Scz (1102)** | **Ctrl (1493)** | **Scz (806)** | **Ctrl (4039)** |
| % Male | 63 | 39 | 72 | 58 | 57 | 49 | 76 | 60 |
| All Dupl | 2 | 24 | 6 | 1 | 1 | 0 | 3 | 6 |
| All Del | 0 | 12 | 1 | 0 | 3 | 3 | 0 | 0 |
| Dup_1+2 | 2 | 18 | 6 | 0 | 0 | 0 | 3 | 2 |

(continued)

| | Iceland | | Scotland | | Germany | | Holland | |
|---|---|---|---|---|---|---|---|---|
| Status No of cases | Scz (648) | Ctrl (27747) | Scz (661) | Ctrl (665) | Scz (1102) | Ctrl (1493) | Scz (806) | Ctrl (4039) |
| Dup_2 | 0 | 3 | 0 | 1 | 0 | 0 | 0 | 1 |
| Dup_2+3 | 0 | 3 | 0 | 0 | 1 | 0 | 0 | 3 |
| Del_1+2 | 0 | 10 | 0 | 0 | 3 | 1 | 0 | 0 |
| Del_2 | 0 | 0 | 0 | 0 | 0 | 0 | 0 | 0 |
| Del_2+3 | 0 | 2 | 1 | 0 | 0 | 2 | 0 | 0 |

**Table 12.** P values, odds ratios and confidence intervals for recurrent duplications and deletions of chromsome 16p13.1

| | **All samples** | | | **Male only** | | | **Female only** | | |
|---|---|---|---|---|---|---|---|---|---|
| 16p13.11 CNV | P-value | common OR | 95% CI | P-value | common OR | 95% CI | P-value | common OR | 95% CI |
| All deletions & all duplications | 0.011 | 2.72 | 1.22-5.9 | 0.016 | 3.03 | 1.15-7.77 | 0.43 | 1.72 | 0.25-8.16 |
| All duplications | 0.0045 | 3.58 | 1.38-8.76 | 0.0078 | 4.12 | 1.32-12.39 | 0.59 | 2.05 | 0.17-12.89 |
| All deletions | 0.73 | 1.39 | 0.26-6.45 | 1 | 1.34 | 0.17-9.18 | 1 | 1.24 | 0.02-20.5 |
| Duplications regions 1 &2 | 0.00018 | 7.07 | 2.37-19.55 | 0.00054 | 8.50 | 2.24-31.81 | 0.24 | 3.63 | 0.22-28.74 |
| Deletions regions 1&2 | 0.38 | 2.26 | 0.28-14.36 | 0.28 | 3.66 | 0.18-45.46 | 1 | 1.28 | 0.02-22.78 |
| Duplications regions 2&3 | 1 | 1.01 | 0.02-11.47 | 1 | 1.86 | 0.02-60.28 | 1 | 0 | 0-29.09 |
| Deletions regions 2&3 | 1 | 0.59 | 0.01-9.48 | 1 | 0.50 | 0.01-8.59 | 1 | 0 | 0-2623.69 |

**Table 13.** Description of cases

| Case No | | Gender | | Dup/del | Diagnosis | Age of onset | Family history | Other |
|---|---|---|---|---|---|---|---|---|
| 584584/ AA02IK2/ GSK0253 | Scotland | M | 2+3 | del | Schizophrenia | 17 | Had a breakdown aged 16/17 when he saw a psychiatrist. Mother suffered a "nervous breakdown". 1959 contact with psychiatric services ?Anxiety and? Schizoid personality. | 14.05.96 grief reaction, following death of mother |
| 583786/ ABSZ1389/ Opcrit no 3885 | Scotland | F | 1+2 | dupl | Schizophrenia | 12 | Paternal mother is 'odd' | |
| 583447/ ABSZ1728/ Opcrit no 7020 | Scotland | M | 1+2 | dupl | Chronic schizophrenia/ paranoia | 19 | Father Bipolar illness. Paternal uncle schizophrenia. Mother died MI 1994. Oldest sister murdered 1995, known drug abuse. | 1993 Low mood {poor social circumstances}. Drug abuse. 1995 ;odd behaviour /laughing inappropriately/ talking to himself/{month after sister died) |
| ABSZ1323/ GSK0329 | Scotland | M | 1+2 | dupl | Schizophrenia | 30 | | Obsessional traits-specific routes and routines |
| 536751 GSK0183 | Scotland | M | 1+2 | dupl | Paranoid schizophrenia | 34 | Mother treated in Dundee Royal for depression. Brother drinks excessively and has abnormal personality. | |
| 536747 | Scotland | M | 1+2 | dupl | Paranoid schizophrenia | 23 | Father suffered from nervous breakdown 1956.impatient. | |
| 543523/ AA02C4T/ GSK3102 | Scotland | F | 1+2 | dupl | Paranoid schizophrenia | 29 | Father died alcoholic, cirrhosis of liver | |
| WG0012761-DNAC05 | Germany | F | 1+2 | del | Schizophrenia | 23 | No FH Behavioural disturbance since childhood | chronic |
| 586835 | Germany | M | 1+2 | del | Schizophrenia | 15 | mother depression, father alcohol abuse, brother heroin dependence: grandfather (father's side) possible schizophrenia | chronic |
| WG0012763-DNAD07 | Germany | M | 1+2 | del | Schizophrenia | 18 | Mother depression | chronic |
| WG0012761-DNAB11 | Germany | M | 2+3 | dupl | Schizophrenia | 17 | No FH behavioural disturbance since childhood | chronic |
| NE50218 | Holland | M | 1+2 | dupl. | Schizophrenia | x | x | x |

(continued)

| Case No | | Gender | | Dup/del | Diagnosis | Age of onset | Family history | Other |
|---|---|---|---|---|---|---|---|---|
| NE71493 | Holland | M | 1+2 | dupl | Schizophrenia | x | x | x |
| NE980503 | Holland | M | 1+2 | dupl | Schizophrenia | 34 | Psychosis and depression sisters | x |
| Ice014 | Iceland | M | | dupl | Schizophrenia | x | Yes, see pedigree | x |
| Ice032 | Iceland | M | | dupl | Schizophrenia | 23 | Yes, see pedigree | chronic |

**EXAMPLE 3.** Duplication on chr 5q35 associated with schizophrenia

By assessment of CNVs using SNP markers on the Illumina HumanHap300 chip in samples from Iceland, we have identified a region on chromosome 5q35.2 that is duplicated in individuals diagnosed with schizophrenia. The 5q35.2 duplication spans a region flanked by markers rs1545976 and rs2220368, between position 175,939,217 and 176,073,058, on chromosome 5 (Figure 7).

Several genes in the duplicated region may contribute to the development of schizophrenia in individuals carrying the 5q35.2 duplication.

The duplicated region contains the Protocadherin LKC precursor gene (PCLKC), a gene encoding G protein-regulated inducer of neurite outgrowth (GPRIN1), a beta-synuclein gene (SNCB) and a gene encoding transmembrane 4 super family member 17 isoform b (TSPAN17).

Protocadherin LKC precursor belongs to the protocadherin family. Members of the protocadherin family encode non-classical cadherins that function as calcium-dependent cell-cell adhesion molecules.

Northern blot analysis of human brain regions shows wide distribution in brain tissue and the central nervous system with highest expression in the spinal cord. Northern blot analysis of mouse tissues detected expression in brain only, and Western analysis detected the GPRIN1 protein in mouse neuroblastoma and rat pheochromocytoma cells. Using immunofluorescence studies and Western analysis of cell fractions, it has been found that GPRIN1 is a membrane-bound protein that is enriched in the growth cones of neurites, and as such is a possible schizophrenia candidate.

Beta synuclein is concentrated in presynaptic nerve terminals. It has been found that mice doubly transgenic for human alpha- and beta-synuclein have decreased accumulation of alpha-synuclein-immunoreactive neuronal inclusions and less severe neurodegenerative alterations compared to mice singly transgenic for human alpha-synuclein. In vitro cell culture studies showed that beta-synuclein coimmunoprecipitated with alpha-synuclein and that cells transfected with beta-synuclein were resistant to alpha-synuclein accumulation. The findings suggested that beta-synuclein may be a natural negative regulator of alpha-synuclein aggregation. Furhter, it has been found that cultured neurons overexpressing beta-synuclein had increased Akt signaling activity and were resistant to neurotoxic effects of the pesticide rotenone compared to cells overexpressing alpha-synuclein and control cells. Downregulation of Akt activity using Akt siRNA resulted in increased susceptibility to the neurotoxic effects of rotenone. Coimmunoprecipitation studies suggested a direct molecular interaction between beta-synuclein and Akt.

## Claims

1. A method of determining a susceptibility to a schizophrenia condition in a human individual, the method comprising:

   obtaining nucleic acid sequence information about a human individual obtained from a sample comprising nucleic acid material from the individual, identifying the chromosome 15q11.2 deletion that is flanked by and includes the markers rs8040193 and rs3883043,
   in the genome of the individual,
   determining by use of a computer the presence or absence of the chromosome 15q11.2 deletion in said individual, wherein the presence and absence of the 15q11.2 deletion are associated with different susceptibilities to the condition in humans, and
   determining by use of a computer a susceptibility to the condition for the individual from the nucleic acid sequence data,
   wherein determination of the presence of the 15q11.2 deletion in the genome of the individual is indicative of an increased risk of the schizophrenia condition in the individual.

2. The method claim 1, wherein determination of a susceptibility comprises comparing the nucleic acid sequence information to a database containing correlation data between copy number variation polymorphisms and susceptibility to the condition.

3. The method of claim 2, wherein the database comprises at least one risk measure of susceptibility to the condition for the chromosome 15q11.2 deletion.

4. The method of claim 2, wherein the database comprises a look-up table containing at least one risk measure of the condition for the chromosome 15q11.2 deletion.

5. The method of any of the preceding claims, wherein obtaining nucleic acid sequence information comprises analyzing at least one polymorphic marker within the chromosome 15q11.2 deletion in a nucleic acid in a biological sample from the human individual.

6. The method of claim 5, wherein analyzing the at least one polymorphic marker comprises obtaining dosage measurement data for the at least one polymorphic marker.

7. The method of any one of the preceding claims, wherein obtaining nucleic acid sequence information comprises identifying the chromosome 15q11.2 deletion in a nucleic acid sample from the individual using a nucleic acid probe selective for a nucleic acid segment that comprises the chromosome 15q11.2 deletion.

8. The method of claim 7, wherein the nucleic acid probe comprises a label, and wherein identifying the chromosome 15q11.2 deletion comprises allowing the nucleic acid probe to hybridize to the nucleic acid segment, such that when bound to the nucleic acid segment, the label is representative of the number of copies of the chromosome 15q11.2 deletion in the individual.

9. The method of any one of claims 1-4, wherein the obtaining nucleic acid sequence information comprises obtaining nucleic acid sequence information from a preexisting record obtained from a sample comprising nucleic acid material from the individual.

**10.** A computer-readable medium having computer executable instructions for determining susceptibility to a schizophrenia condition in a human individual, the computer readable medium comprising:

data indicative of the chromosome 15q11.2 deletion that is flanked by and includes the markers rs8040193 and rs3883043; and
a routine stored on the computer readable medium and adapted to be executed by a processor to determine based on said data, risk of developing a schizophrenia condition,
wherein determination of the presence of the 15q11.2 deletion in the genome of the individual is indicative of an increased risk of the schizophrenia condition in the individual,
and determination of the absence of the 15q11.2 deletion is indicative that the individual does not have the increased risk.

**11.** An apparatus for determining a genetic indicator for a schizophrenia condition in a human individual, comprising:

a processor
a computer readable memory having computer executable instructions adapted to be executed on the processor to analyze information about the chromosome 15q11.2 deletion that is flanked by and includes the markers rs8040193 and rs3883043 in the human individual, and
generate an output based on the information about the the chromosome 15q11.2 deletion, wherein the output comprises a risk measure of the chromosome 15q11.2 deletion as a genetic indicator of the schizophrenia condition for the human individual,
wherein determination of the presence of the 15q11.2 deletion in the genome of the individual is indicative of an increased risk of the schizophrenia condition in the individual,
and determination of the absence of the 15q11.2 deletion is indicative that the individual does not have the increased risk.

**12.** The apparatus of claim 11, wherein the data for the chromosome 15q11.2 deletion is dosage data for at least one marker within the chromosome 15q11.2 deletion.

## Patentansprüche

**1.** Verfahren zur Ermittlung einer Anfälligkeit für ein Schizophrenieleiden bei einem menschlichen Individuum, wobei man bei dem Verfahren Nukleinsäuresequenzinformationen über ein menschliches Individuum erhält, die von einer Nukleinsäurematerial von dem Individuum umfassenden Probe erhalten wurden, wodurch die chromosomale 15q11.2-Deletion, die von den Markern rs8040193 und rs3883043 flankiert wird und diese einschließt, im Genom des Individuums identifiziert wird,
mit einem Computer das Vorliegen oder Fehlen der chromosomalen 15q11.2-Deletion bei dem Individuum ermittelt, wobei das Vorliegen und das Fehlen der 15q11.2-Deletion mit unterschiedlichen Anfälligkeiten für das Leiden beim Menschen assoziiert sind, und
mit einem Computer für das Individuum eine Anfälligkeit für das Leiden anhand der Nukleinsäuresequenzdaten ermittelt,
wobei eine Ermittlung des Vorliegens der 15q11.2-Deletion im Genom des Individuums ein erhöhtes Risiko des Schizophrenieleidens bei dem Individuum anzeigt.

**2.** Verfahren nach Anspruch 1, wobei die Ermittlung einer Anfälligkeit das Vergleichen der Nukleinsäuresequenzinformationen mit einer Datenbank, die Korrelationsdaten zwischen Kopienzahlvariationspolymorphismen und Anfälligkeit für das Leiden enthält, umfasst.

**3.** Verfahren nach Anspruch 2, wobei die Datenbank wenigstens ein Maß für das Risiko der Anfälligkeit für das Leiden für die chromosomale 15q11.2-Deletion umfasst.

**4.** Verfahren nach Anspruch 2, wobei die Datenbank eine Lookup-Tabelle umfasst, die wenigstens ein Maß für das Risiko der Anfälligkeit für das Leiden für die chromosomale 15q11.2-Deletion enthält.

**5.** Verfahren nach einem der vorhergehenden Ansprüche, wobei das Erhalten von Nukleinsäuresequenzinformationen das Analysieren wenigstens eines polymorphen Markers innerhalb der chromosomalen 15q11.2-Deletion in einer Nukleinsäure in einer biologischen Probe von dem menschlichen Individuum umfasst.

6. Verfahren nach Anspruch 5, wobei das Analysieren des wenigstens einen polymorphen Markers das Gewinnen von Dosierungsmessdaten für den wenigstens einen polymorphen Marker umfasst.

7. Verfahren nach einem der vorhergehenden Ansprüche, wobei das Erhalten von Nukleinsäuresequenzinformationen das Identifizieren der chromosomalen 15q11.2-Deletion in einer Nukleinsäureprobe von dem Individuum umfasst, wobei eine für einen Nukleinsäureabschnitt, der die chromosomale 15q11.2-Deletion umfasst, selektive Nukleinsäuresonde verwendet wird.

8. Verfahren nach Anspruch 7, wobei die Nukleinsäuresonde eine Markierung umfasst und wobei das Identifizieren der chromosomalen 15q11.2-Deletion umfasst, dass der Nukleinsäuresonde das Hybridisieren an den Nukleinsäureabschnitt gestattet wird, so dass bei ihrer Bindung an den Nukleinsäureabschnitt die Markierung die Anzahl der Kopien der chromosomalen 15q11.2-Deletion bei dem Individuum repräsentiert.

9. Verfahren nach einem der Ansprüche 1-4, wobei das Erhalten von Nukleinsäuresequenzinformationen das Erhalten von Nukleinsäuresequenzinformationen aus einem bereits bestehenden Dokument, das von einer Nukleinsäurematerial von dem Individuum umfassenden Probe erhalten wurde, umfasst.

10. Computerlesbarer Datenträger mit von einem Computer ausführbaren Anweisungen zur Ermittlung der Anfälligkeit für ein Schizophrenieleiden bei einem menschlichen Individuum, wobei der computerlesbare Datenträger Folgendes umfasst:

Daten, die die chromosomale 15q11.2-Deletion, die von den Markern rs8040193 und rs3883043 flankiert wird und diese einschließt, anzeigen; und
eine auf dem computerlesbaren Datenträger gespeicherte Routine, die adaptiert ist, so dass sie von einem Prozessor ausgeführt wird, um das Risiko der Entwicklung eines Schizophrenieleidens auf der Grundlage der Daten zu ermitteln,
wobei eine Ermittlung des Vorliegens der 15q11.2-Deletion im Genom des Individuums ein erhöhtes Risiko des Schizophrenieleidens bei dem Individuum anzeigt und eine Ermittlung des Fehlens der 15q11.2-Deletion anzeigt, dass das Individuum das erhöhte Risiko nicht aufweist.

11. Vorrichtung zur Ermittlung eines genetischen Indikators für ein Schizophrenieleiden bei einem menschlichen Individuum, umfassend:

einen Prozessor,
einen computerlesbaren Speicher mit von einem Computer ausführbaren Anweisungen, die adaptiert sind, so dass sie auf dem Prozessor ausgeführt werden, um Informationen über die chromosomale 15q11.2-Deletion, die von den Markern rs8040193 und rs3883043 flankiert wird und diese einschließt, in dem menschlichen Individuum zu analysieren und
eine Ausgabe bezogen auf die Informationen über die chromosomale 15q11.2-Deletion zu erzeugen,
wobei die Ausgabe ein Maß für das Risiko der chromosomalen 15q11.2-Deletion als genetischer Indikator des Schizophrenieleidens für das menschliche Individuum umfasst, wobei eine Ermittlung des Vorliegens der 15q11.2-Deletion im Genom des Individuums ein erhöhtes Risiko des Schizophrenieleidens bei dem Individuum anzeigt und eine Ermittlung des Fehlens der 15q11.2-Deletion anzeigt, dass das Individuum das erhöhte Risiko nicht aufweist.

12. Vorrichtung nach Anspruch 11, wobei es sich bei den Daten für die chromosomale 15q11.2-Deletion um Dosierungsdaten für wenigstens einen Marker innerhalb der chromosomalen 15q11.2-Deletion handelt.

## Revendications

1. Procédé de détermination d'une susceptibilité à un trouble schizophrénique chez un individu humain, le procédé comprenant :

l'obtention d'informations de séquence d'acide nucléique concernant un individu humain obtenues à partir d'un échantillon comprenant un matériau d'acide nucléique de l'individu, l'identification de la délétion du chromosome 15q11.2 qui est flanquée par et
comprend les marqueurs rs8040193 et rs3883043, dans le génome de l'individu,
la détermination par utilisation d'un ordinateur de la présence ou l'absence de la délétion du chromosome 15q11.2 chez ledit individu, la présence et l'absence de la délétion de 15q11.2 étant associées à différentes susceptibilités de l'affection chez des humains, et
la détermination par utilisation d'un ordinateur d'une susceptibilité de l'affection pour l'individu à partir des données de séquence d'acide nucléique,
la détermination de la présence de la délétion de 15q11.2 dans le génome de l'individu étant indicative d'un risque accru du trouble schizophrénique chez l'individu.

**2.** Procédé de la revendication 1, dans lequel la détermination d'une susceptibilité comprend la comparaison des informations de séquence d'acide nucléique à une base de données contenant des données de corrélation entre des polymorphismes de variation de nombre de copies et la susceptibilité de l'affection.

**3.** Procédé de la revendication 2, dans lequel la base de données comprend au moins une mesure de risque de susceptibilité de l'affection pour la délétion du chromosome 15q11.2.

**4.** Procédé de la revendication 2, dans lequel la base de données comprend une table de consultation contenant au moins une mesure de risque de susceptibilité de l'affection pour la délétion du chromosome 15q11.2.

**5.** Procédé de l'une quelconque des revendications précédentes, dans lequel l'obtention d'informations de séquence d'acide nucléique comprend l'analyse d'au moins un marqueur polymorphe dans la délétion du chromosome 15q11.2 dans un acide nucléique dans un échantillon biologique de l'individu humain.

**6.** Procédé de la revendication 5, dans lequel l'analyse d'au moins un marqueur polymorphe comprend l'obtention de données de mesure de dose pour l'au moins un marqueur polymorphe.

**7.** Procédé de l'une quelconque des revendications précédentes, dans lequel l'obtention d'informations de séquence d'acide nucléique comprend l'identification de la délétion du chromosome 15q11.2 dans un échantillon d'acide nucléique de l'individu en utilisant une sonde d'acide nucléique sélective pour un segment d'acide nucléique qui comprend la délétion du chromosome 15q11.2.

**8.** Procédé de la revendication 7, dans lequel la sonde d'acide nucléique comprend un marqueur, et dans lequel l'identiication de la délétion du chromosome 15q11.2 comprend l'étape consistant à laisser la sonde d'acide nucléique s'hybrider avec le segment d'acide nucléique, de sorte que lorsqu'il est lié au segment d'acide nucléique, le marqueur est représentatif du nombre de copies de la délétion du chromosome 15q11.2 chez l'individu.

**9.** Procédé de l'une quelconque des revendications 1 à 4, dans lequel l'obtention d'informations de séquence d'acide nucléique comprend l'obtention d'informations de séquence d'acide nucléique à partir d'un enregistrement préexistant obtenu à partir d'un échantillon comprenant du matériau d'acide nucléique provenant de l'individu.

**10.** Support lisible par ordinateur ayant des instructions exécutables par ordinateur pour déterminer la susceptibilité à un trouble schizophrénique chez un individu humain, le support lisible par ordinateur comprenant :

des données indicatrices de la délétion du chromosome 15q11.2 qui est flanquée par et comprend les marqueurs rs8040193 et rs3883043 ; et
un sous-programme stocké sur le support lisible par ordinateur et adapté pour être exécuté par un processeur pour déterminer, sur la base desdites données, le risque de développer un trouble schizophrénique,
la détermination de la présence de la délétion de 15q11.2 dans le génome de l'individu étant indicative d'un risque augmenté du trouble schizophrénique chez l'individu, et la détermination de l'absence de la délétion de 15q11.2 est indicative que l'individu n'a pas le risque accru.

**11.** Appareil pour déterminer un indicateur génétique pour un trouble schizophrénique chez un individu humain, comprenant :

un processeur
une mémoire lisible par ordinateur ayant des instructions exécutables par ordinateur adaptées pour être exécutées sur le processeur pour analyser des informations sur la délétion du chromosome 15q11.2 qui est flanquée par et comprend les marqueurs rs8040193 et
rs3883043 chez l'individu humain, et
générer une sortie sur la base des informations sur la délétion du chromosome 15q11.2, la sortie comprenant une mesure du risque de délétion du chromosome 15q11.2 en tant qu'indicateur génétique du trouble schizophrénique pour l'individu humain, la détermination de la présence de la délétion de 15q11.2 dans le génome de l'individu étant indicative d'un risque accru du trouble schizophrénique chez l'individu, et la détermination de l'absence de la délétion de 15q11.2 étant indicative que l'individu n'a pas le risque accru.

**12.** Appareil de la revendication 11, les données pour la délétion du chromosome 15q11.2 étant des données de dosage pour au moins un marqueur dans la délétion du chromosome 15q11.2.

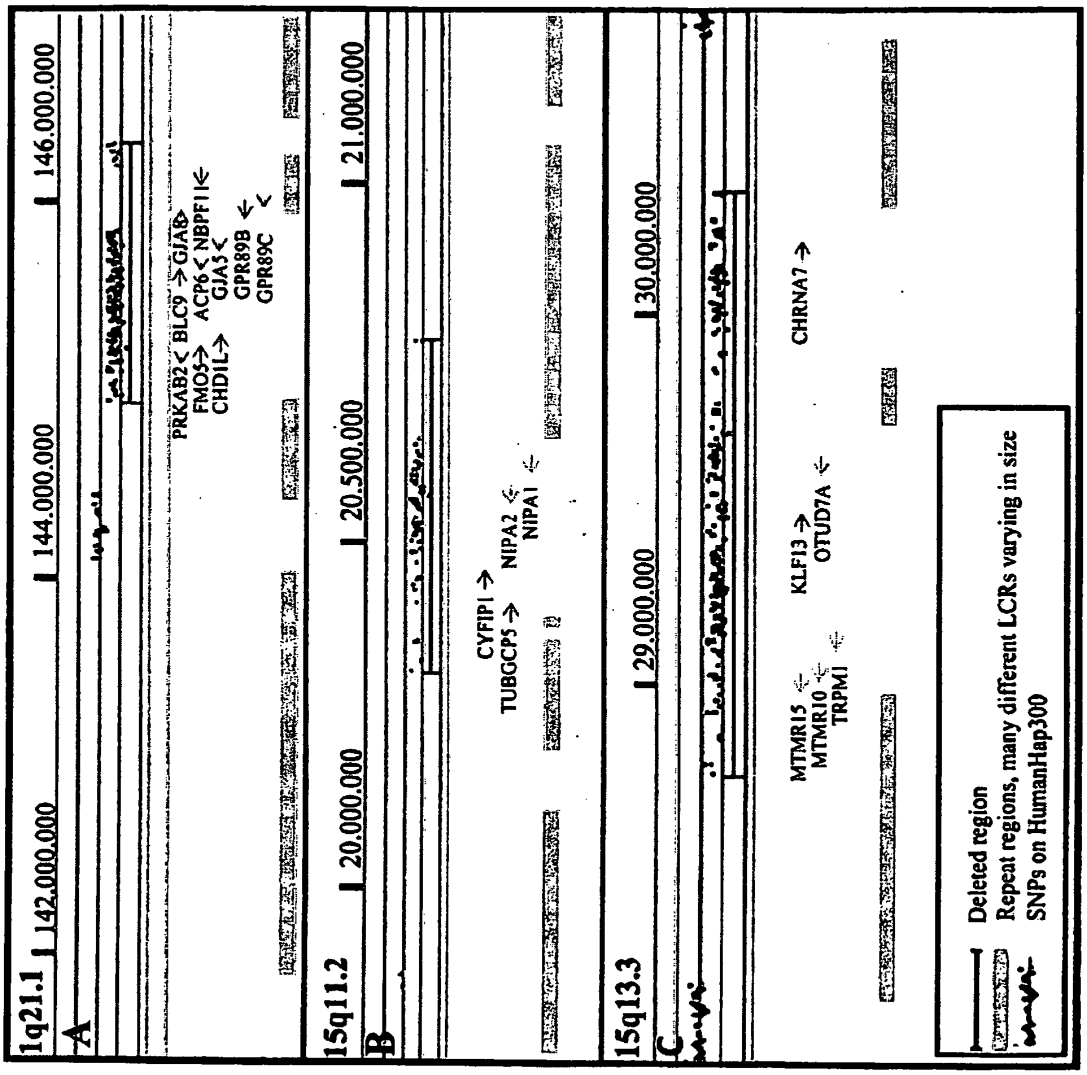
1q21.1
A
142.000.000
144.000.000
146.000.000
PRKAB2
FMO5
CHD1L
BLC9
ACP6
GJA5
GJA8
NBPF11
GPR89B
GPR89C
15q11.2
B
20.000.000
20.500.000
21.000.000
TUBGCP5
CYFIP1
NIPA2
NIPA1
15q13.3
C
29.000.000
30.000.000
MTMR15
MTMR10
TRPM1
KLF13
OTUD7A
CHRNA7
Deleted region
Repeat regions, many different LCRs varying in size
SNPs on HumanHap300

FIG. 1

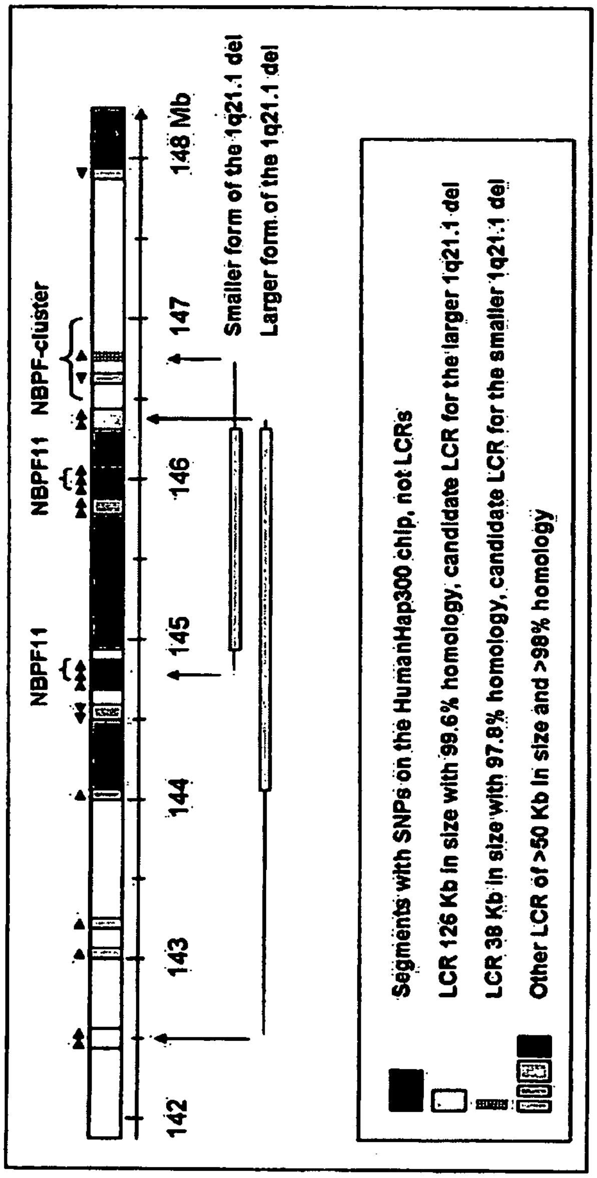
NBPF11
NBPF11
NBPF-cluster
142
143
144
145
146
147
148 Mb
Smaller form of the 1q21.1 del
Larger form of the 1q21.1 del
Segments with SNPs on the HumanHap300 chip, not LCRs
LCR 126 Kb in size with 99.6% homology, candidate LCR for the larger 1q21.1 del
LCR 38 Kb in size with 97.8% homology, candidate LCR for the smaller 1q21.1 del
Other LCR of >50 Kb in size and >98% homology

FIG. 2

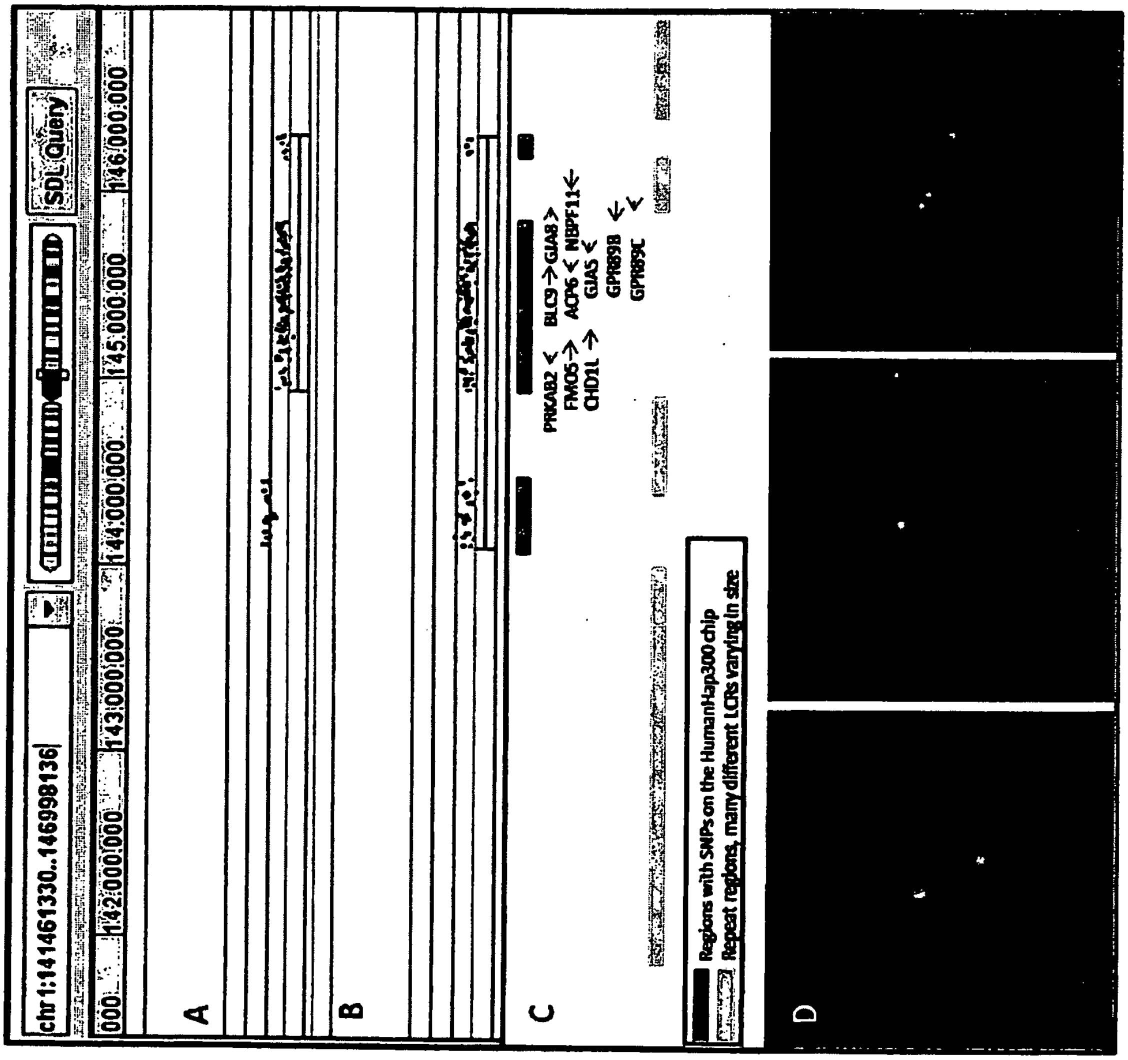
chr1:141461330..146998136
SDL Query
142,000,000
143,000,000
144,000,000
145,000,000
146,000,000
A
B
C
D
PRKAB2
FMO5
CHD1L
BCL9
GJA8
ACP6
NBPF11
GJA5
GPR89B
GPR89C
Regions with SNPs on the HumanHap300 chip
Repeat regions, many different LCRs varying in size

FIG. 3

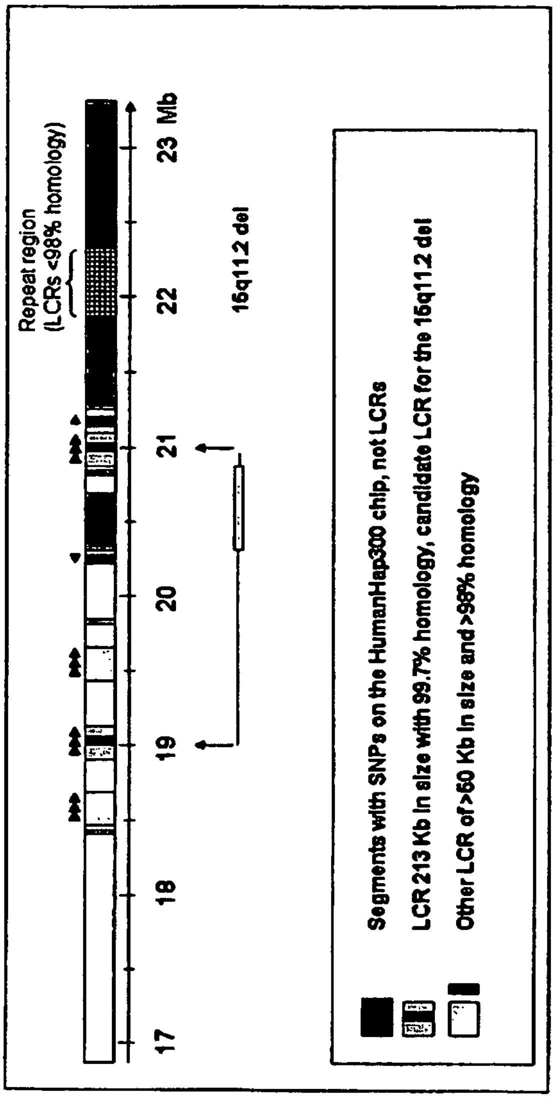
Repeat region
(LCRs <98% homology)
17
18
19
20
21
22
23 Mb
15q11.2 del
Segments with SNPs on the HumanHap300 chip, not LCRs
LCR 213 Kb in size with 99.7% homology, candidate LCR for the 15q11.2 del
Other LCR of >50 Kb in size and >98% homology

FIG. 4

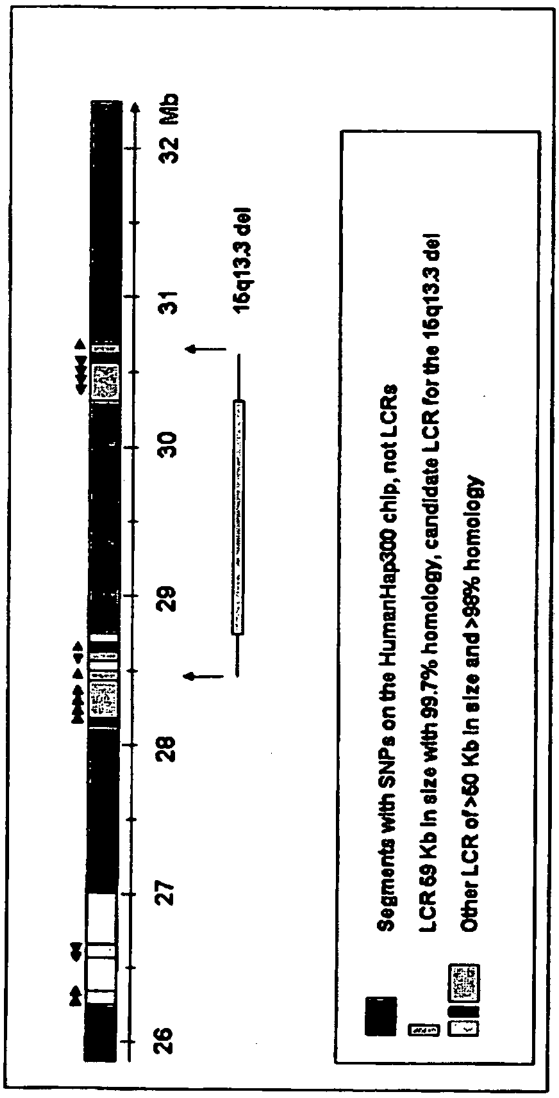
26
27
28
29
30
31
32 Mb
16q13.3 del
Segments with SNPs on the HumanHap300 chip, not LCRs
LCR 59 Kb in size with 99.7% homology, candidate LCR for the 16q13.3 del
Other LCR of >50 Kb in size and >98% homology

FIG. 5

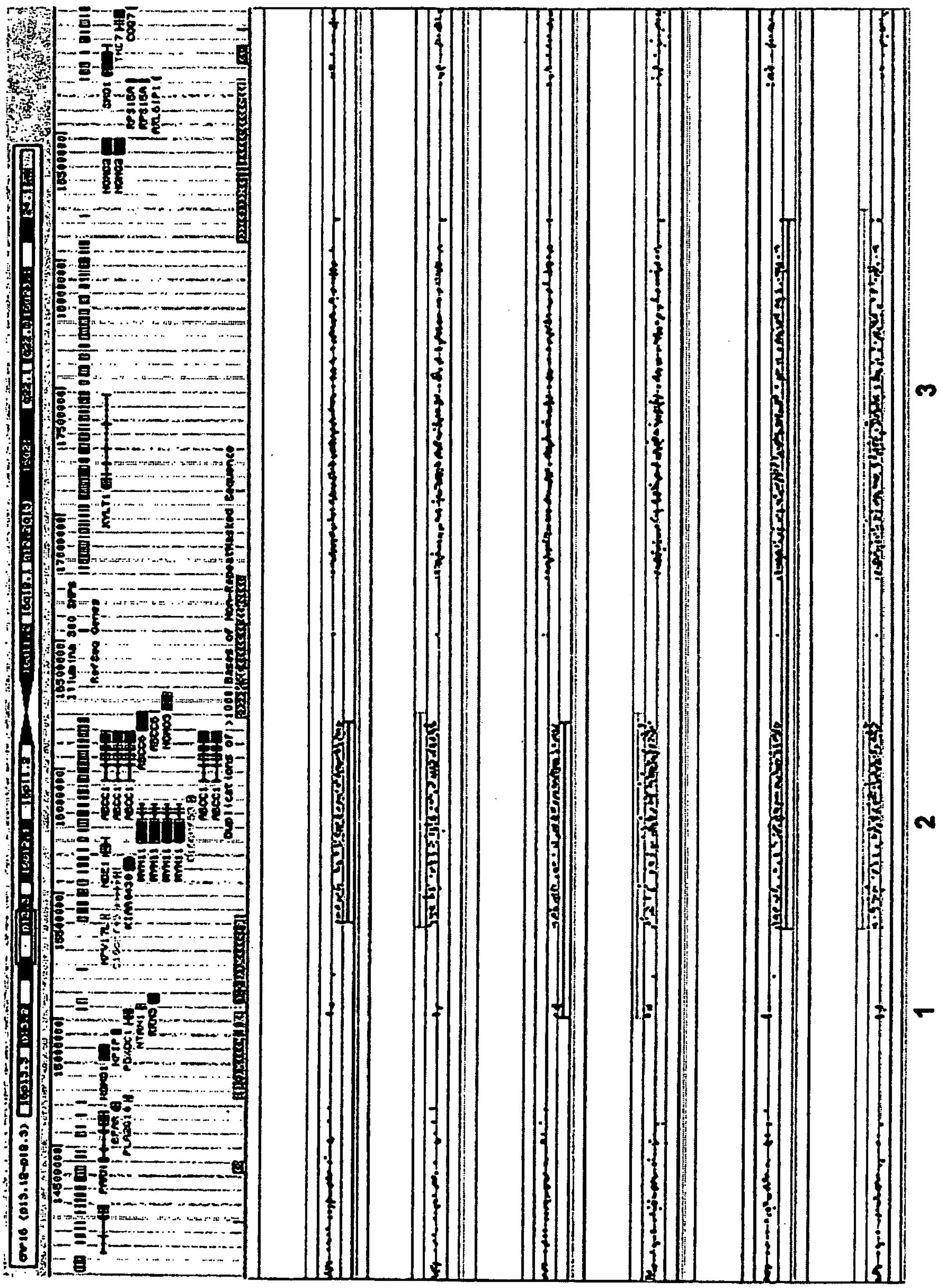


FIG. 6

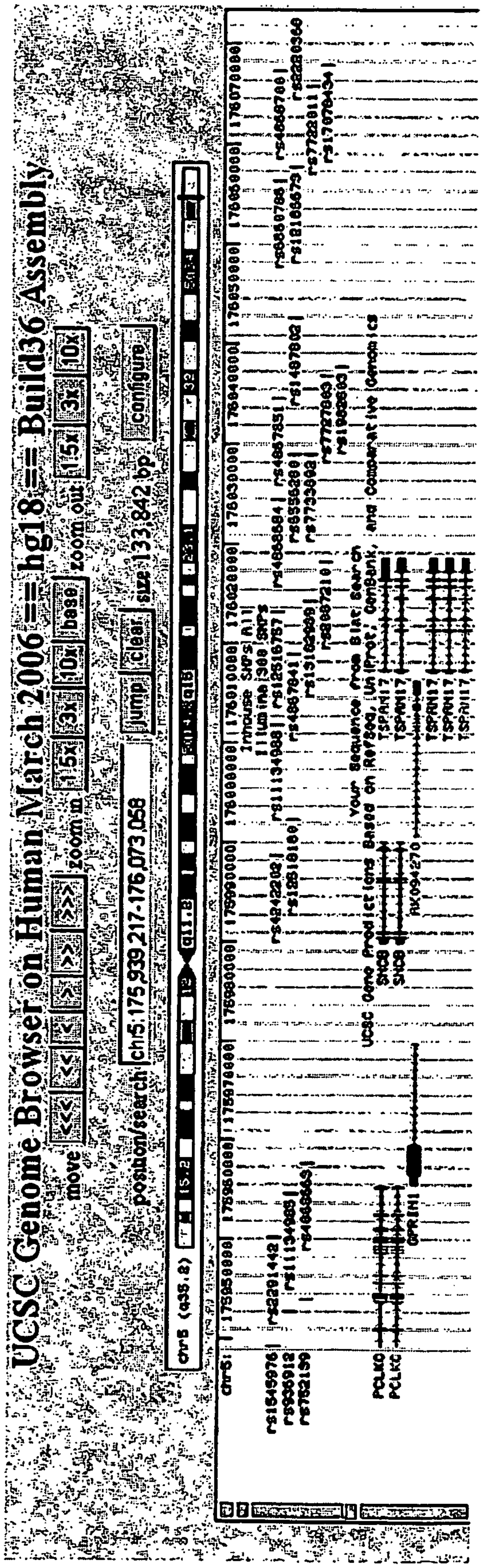
UCSC Genome Browser on Human March 2006 == hg18 == Build36 Assembly
move
zoom in
zoom out
position/search chr5:175,939,217-176,073,058
jump
clear
size 133,842 bp.
configure

FIG. 7

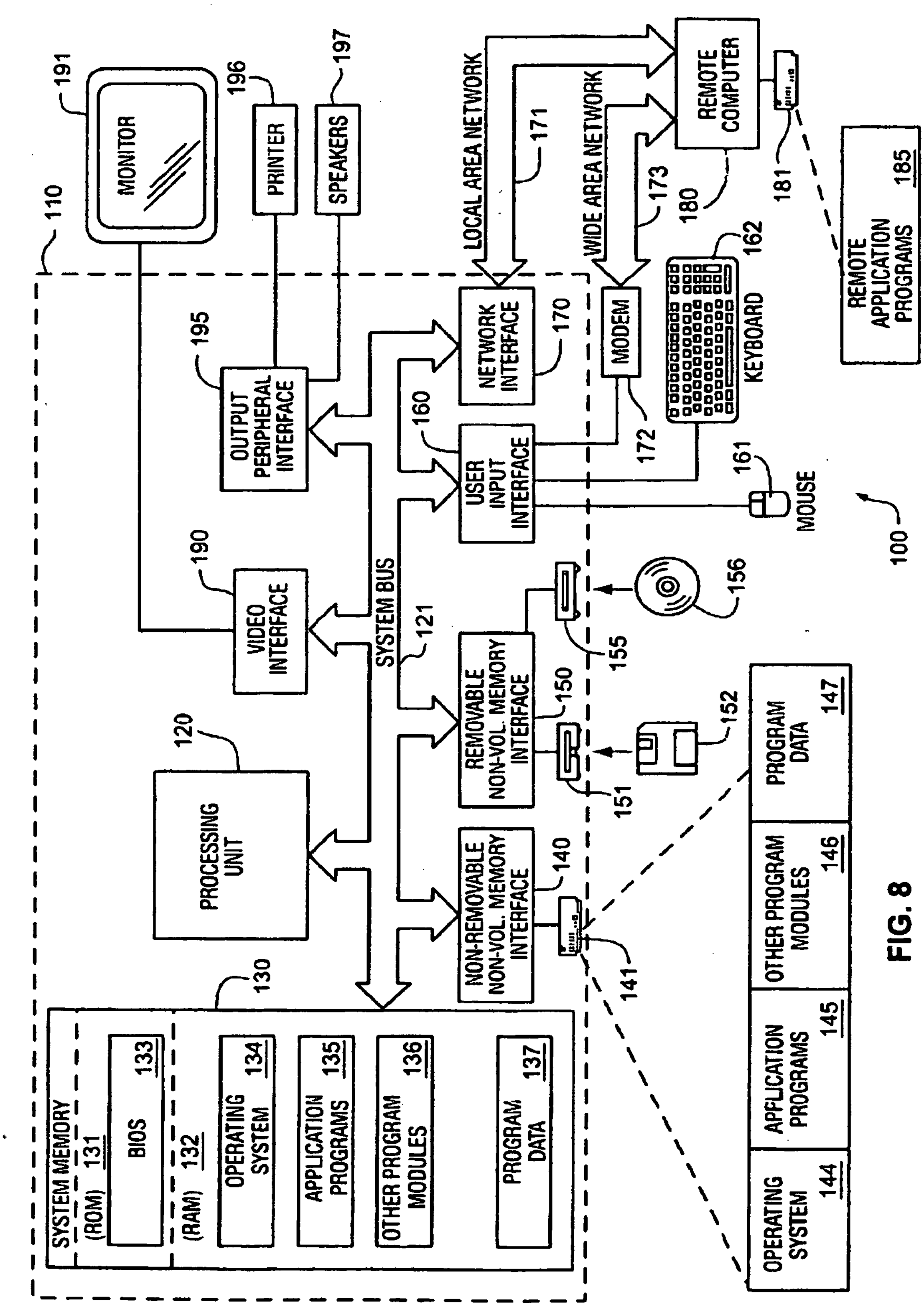
SYSTEM MEMORY
(ROM) 131
BIOS 133
(RAM) 132
OPERATING SYSTEM 134
APPLICATION PROGRAMS 135
OTHER PROGRAM MODULES 136
PROGRAM DATA 137
130
PROCESSING UNIT
120
VIDEO INTERFACE
190
OUTPUT PERIPHERAL INTERFACE
195
MONITOR
191
PRINTER
196
SPEAKERS
197
110
SYSTEM BUS
121
NON-REMOVABLE NON-VOL. MEMORY INTERFACE
140
141
REMOVABLE NON-VOL. MEMORY INTERFACE
150
151
152
155
156
USER INPUT INTERFACE
160
NETWORK INTERFACE
170
MODEM
172
LOCAL AREA NETWORK
171
WIDE AREA NETWORK
173
REMOTE COMPUTER
180
181
REMOTE APPLICATION PROGRAMS 185
KEYBOARD
162
MOUSE
161
100
OPERATING SYSTEM 144
APPLICATION PROGRAMS 145
OTHER PROGRAM MODULES 146
PROGRAM DATA 147
FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6858394 B
- US 6429027 B
- US 5445934 A
- US 5700637 A
- US 5744305 A
- US 5945334 A
- US 6054270 A
- US 6300063 B
- US 6733977 B
- US 7364858 B
- EP 619321 A
- EP 373203 A
- US 5288644 A, Beavis
- US 4376110 A, David
- US 5223409 A
- WO 9218619 A
- WO 9117271 A
- WO 9220791 A
- WO 9215679 A
- WO 9301288 A
- WO 9201047 A
- WO 9209690 A
- WO 9002809 A


### Non-patent literature cited in the description


- **REDON, R. et al.** *Nature,* 2006, vol. 444, 444-54
- **ESTIVILL, X. ; ARMENGOL, L.** *PloS Genetics,* 2007, vol. 3, e190
- **COYLE et al.** *Ann. NY Acad. Sci.,* 2003, vol. 1003, 318-27
- **TSUANG, M.T. et al.** *Schizophr. Res.,* 1991, vol. 4 (2), 157-71
- **TIENARI, P.J. ; WYNNE, L.C.** *Ann. Med.,* 1994, vol. 26 (4), 233-7
- **FRANZEK, E. ; BECKMANN, H.** *Am. J. Psychiatry,* 1998, vol. 155 (1), 76-83
- **TSUANG, M. T.** *J. Biomed. Sci.,* 1998, vol. 5 (1), 28-30
- **MCGUE, M. ; GOTTESMANN, I.I.** *Eur. Arch. Psychiatry Clin. Neurosci,* 1991, vol. 240, 174-181
- **LIM, L.C. ; SIM, L.P.** *Singapore Med. J.,* 1992, vol. 33 (6), 645-7
- **PULVER, A.E. et al.** *Am J Med Genet,* 1995, vol. 60 (4), 252-60
- **KENDLER, K.S. et al.** *Am J Med Genet,* 1999, vol. 88 (1), 29-33
- **HOVATTA, I. et al.** *Psychiatry,* 1998, vol. 3 (5), 452-7
- **SCHWAB, S.G. et al.** *Nat Genet,* 1995, vol. 11 (3), 325-7
- **BRZUSTOWICZ, L.M. et al.** *Am J Hum Genet,* 1997, vol. 61 (6), 1388-96
- **CAO, Q. et al.** *Genomics,* 1997, vol. 43 (1), 1-8
- **STRAUB, R.E. et al.** *Cold Spring Harbor Symp Quant Biol,* 1996, vol. 61I, 823-33
- **KENDLER, KS. et al.** *Am J Psychiatry,* 1996, vol. 153 (12), 1534-40
- **STRAUB, R.E. et al.** *Am J Med Genet,* 1998, vol. 81 (4), 296-301
- **SCHWAB, S.G. et al.** *Am J Med Genet,* 1998, vol. 81 (4), 302-307
- **LIN, M.W. et al.** *Psyciatr Genet,* 1995, vol. 5 (3), 117-26
- **LIN, M.W. et al.** *Hum Genet,* 1997, vol. 99 (3), 417-420
- **BLOUIN, J.L. et al.** *Nat Genet,* 1993, vol. 20 (1), 70-73
- **GILL, M. et al.** *Am J Med Genet,* 1996, vol. 67 (1), 40-45
- **BASSETT, A.S. et al.** *Am J Med Genet,* 1998, vol. 81 (4), 328-37
- **MILUNSKY, J. et al.** *Clin Genet,* 1999, vol. 55 (6), 455-60
- **HARRISON ; OWEN.** *Lancet,* 2003, vol. 361, 417-9
- **STEFANSSON et al.** *Am. J. Hum. Genet.,* 2003, vol. 72, 83-7
- **STEFANSON et al.** *Am. J. Hum. Genet.,* 2003, vol. 72, 83-7
- **WILLIAMS et al.** *Mol. Psychiatry,* 2003, vol. 8, 485-7
- **YANG et al.** *Mol. Psychiatry,* 2003, vol. 8, 706-9
- **JESSELL et al.** *Proc. Natl. Acad. Sci.,* 1979, vol. 76, 5397-5401
- **FALLS et al.** *J. Neurocytol.,* 2003, vol. 32, 619-647
- **FREEDMAN, N.** *Engl. J. Med.,* 2003, vol. 349, 1738-1749

- **REVIEWED.** *Konradi & Heckers Pharmacology & Therapeutics,* 2003, vol. 97, 153-197
- **YU et al.** *Science,* 1997, vol. 275, 674-678
- **WANG et al.** *Nature,* 1994, vol. 369, 233-235
- **SLATER et al.** *Nat. Rev. Neurosci.,* 2004, vol. 5, 317-328
- **MOGHADDAM.** *Neuron,* 2003, vol. 40, 881-884
- Diagnostic and Statistical Manual of Mental Disorder
- **ESTIVILL, X ; ARMENGOL; L.** *Plos Genetics,* 2007, vol. 3, 1787-99
- **REDON, R. et al.** *Nature,* 2006, vol. 23, 444-454
- **CARTER.** *Nature Genetics,* 2007, vol. 39, S16-S21
- **CHEN, X. et al.** *Genome Res.,* 1999, vol. 9 (5), 492-98
- **KUTYAVIN et al.** *Nucleic Acid Res.,* 2006, vol. 34, e128
- **GUDMUNDSSON, J. et al.** *Nat Genet,* 2007, vol. 39, 631-7
- **GUDMUNDSSON, J. et al.** *Nat Genet,* 2007, vol. 39, 977-83
- **YEAGER, M. et al.** *Nat Genet,* 2007, vol. 39, 645-49
- **AMUNDADOTTIR, L. et al.** *Nat Genet,* 2006, vol. 38, 652-8
- **HAIMAN, C.A. et al.** *Nat Genet,* 2007, vol. 39, 638-44
- **BIECHE, I. et al.** *Int J Cancer,* 1998, vol. 78, 661-6
- **PERKEL. J.** *Nature Methods,* 2008, vol. 5, 447-453
- **GRETARSDOTTIR S. et al.** *Nat. Genet.,* 2003, vol. 35, 131-38
- **NICOLAE ; KONG.** Technical Report 537, Department of Statistics, University of Statistics, University of Chicago. *Biometrics,* 2004, vol. 60 (2), 368-75
- **RISCH, N. ; TENG, J.** *Genome Res.,* 1998, vol. 8, 1273-1288
- **DEVLIN, B. ; ROEDER, K.** *Biometrics,* 1999, vol. 55, 997
- **TERWILLIGER, J.D. ; OTT, J.** *Hum. Hered.,* 1992, vol. 42, 337-46
- **FALK, C.T. ; RUBINSTEIN, P.** *Ann. Hum. Genet.,* 1987, vol. 51, 227-33
- **MANTEL ; HAENSZEL.** *J Natl Cancer Inst,* 1959, vol. 22, 719-48
- **SULEM, P. et al.** *Nat Genet,* 17 May 2009
- **RAFNAR, T. et al.** *Nat Genet,* 2009, vol. 41, 221-7
- **GRETARSDOTTIR, S. et al.** *Ann Neurol,* 2008, vol. 64, 402-9
- **STACEY, S.N. et al.** *Nat Genet,* 2008, vol. 40, 1313-18
- **GUDBJARTSSON, D.F. et al.** *Nat Genet,* 2008, vol. 40, 886-91
- **STYRKARSDOTTIR, U. et al.** *N Engl J Med,* 2008, vol. 358, 2355-65
- **THORGEIRSSON, T. et al.** *Nature,* 2008, vol. 452, 638-42
- **GUDMUNDSSON, J. et al.** *Nat Genet.,* 2008, vol. 40, 281-3
- **STACEY, S.N. et al.** *Nat Genet.,* 2007, vol. 39, 865-69
- **HELGADOTTIR, A. et al.** *Science,* 2007, vol. 316, 1491-93
- **STEINTHORSDOTTIR, V. et al.** *Nat Genet.,* 2007, vol. 39, 770-75
- **GUDMUNDSSON, J. et al.** *Nat Genet.,* 2007, vol. 39, 631-37
- **FRAYLING, TM.** *Nature Reviews Genet,* 2007, vol. 8, 657-662
- **AMUNDADOTTIR, L.T. et al.** *Nat Genet.,* 2006, vol. 38, 652-58
- **GRANT, S.F. et al.** *Nat Genet.,* 2006, vol. 38, 320-23
- **SMITH et al.** *Am J Hum Genet,* 2004, vol. 74, 1001-13
- Current Protocols in Molecular Biology. John Wiley & Sons
- **NIELSEN, P. et al.** *Bioconjug. Chem.,* 1994, vol. 5, 3-7
- **BIER, F.F. et al.** *Adv Biochem Eng Biotechnol,* 2008, vol. 109, 433-53
- **HOHEISEL, J.D.** *Nat Rev Genet,* 2006, vol. 7, 200-10
- **FAN, J.B. et al.** *Methods Enzymol,* 2006, vol. 410, 57-73
- **RAQOUSSIS, J. ; ELVIDGE, G.** *Expert Rev Mol Diagn,* 2006, vol. 6, 145-52
- **MOCKLER, T.C. et al.** *Genomics,* 2005, vol. 85, 1-15
- **CHURCH ; GILBERT.** *Proc. Natl. Acad. Sci. USA,* 1998, vol. 81, 1991-1995
- **SANGER, F. et al.** *Proc. Natl. Acad. Sci. USA,* 1977, vol. 74, 5463-5467
- **SHEFFIELD, V. et al.** *Proc. Natl. Acad. Sci. USA,* 1989, vol. 86, 232-236
- **ORITA, M. et al.** *Proc. Natl. Acad. Sci. USA,* 1989, vol. 86, 2766-2770
- **FLAVELL, R. et al.** *Cell,* 1978, vol. 15, 25-41
- **GEEVER, R. et al.** *Proc. Natl. Acad. Sci. USA,* 1981, vol. 78, 5081-5085
- **COTTON, R. et al.** *Proc. Natl. Acad. Sci. USA,* 1985, vol. 85, 4397-4401
- **MYERS, R.** *Science,* 1985, vol. 230, 1242-1246
- Current Protocols in Molecular Biology
- AntisenseDrug Technology: Principles, Strategies, and Applications. Marcel Dekker Inc, 2001
- **THOMPSON.** *Drug Discovery Today,* 2002, vol. 7, 912-917

- **LAVERY et al.** *Curr. Opin. Drug Discov. Devel.,* 2003, vol. 6, 561-569
- **STEPHENS et al.** *Curr. Opin. Mol. Ther.,* 2003, vol. 5, 118-122
- **KURRECK.** *Eur. J. Biochem.,* 2003, vol. 270, 1628-44
- **DIAS et al.** *Mol. Cancer Ter.,* 2002, vol. 1, 347-55
- **CHEN.** *Methods Mol. Med.,* 2003, vol. 75, 621-636
- **WANG et al.** *Curr. Cancer Drug Targets,* 2001, vol. 1, 177-96
- **BENNETT.** *Antisense Nucleic Acid Drug.Dev.,* 2002, vol. 12, 215-24
- **FIRE et al.** *Nature,* 1998, vol. 391, 806-11
- **KIM ; ROSSI.** *Nature Rev. Genet.,* 2007, vol. 8, 173-204
- **AMARZGUIOUI et al.** *FEBS Lett.,* 2005, vol. 579, 5974-81
- **KIM et al.** *Nature Biotechnol.,* 2005, vol. 23, 222-226
- **SIOLAS et al.** *Nature Biotechnol.,* 2005, vol. 23, 227-231
- **MARQUES et al.** *Nature Biotechnol.,* 2006, vol. 23, 559-565
- **BRUMMELKAMP et al.** *Science,* 2002, vol. 296, 550-553
- **KIM ; ROSSI.** *Nat. Rev. Genet.,* 2007, vol. 8, 173-184
- **CHEN ; RAJEWSKY.** *Nat. Rev. Genet.,* 2007, vol. 8, 93-103
- **REYNOLDS et al.** *Nat. Biotechnol.,* 2004, vol. 22, 326-330
- **CHI et al.** *Proc. Natl. Acad. Sci. USA,* 2003, vol. 100, 6343-6346
- **VICKERS et al.** *J. Biol. Chem.,* 2003, vol. 278, 7108-7118
- **AGAMI.** *Curr. Opin. Chem. Biol.,* 2002, vol. 6, 829-834
- **SHI.** *Trends Genet.,* 2003, vol. 19, 9-12
- **SHUEY et al.** *Drug Discov. Today,* 2002, vol. 7, 1040-46
- **MCMANUS et al.** *Nat. Rev. Genet.,* 2002, vol. 3, 737-747
- **XIA et al.** *Nat. Biotechnol.,* 2002, vol. 20, 1006-10
- **PLASTERK et al.** *curr. Opin. Genet. Dev.,* 2000, vol. 10, 562-7
- **BOSHER et al.** *Nat. Cell Biol.,* 2000, vol. 2, E31-6
- **HUNTER.** *Curr. Biol.,* 1999, vol. 9, R440-442
- **AUSUBEL, F. et al.** Current Protocols in Molecular Biology. John Wiley & Sons, 1998
- **KRAUS, M. ; AARONSON, S.** *Methods Enzymol.,* 1991, vol. 200, 546-556
- **KARLIN, S. ; ALTSCHUL, S.** *Proc. Natl. Acad. Sci. USA,* 1993, vol. 90, 5873-5877
- **ALTSCHUL, S. et al.** *Nucleic Acids Res.,* 1997, vol. 25, 3389-3402
- **KENT, W.J.** *Genome Res.,* 2002, vol. 12, 656-64
- **MYERS ; MILLER.** *CABIOS,* 1989
- **TORELLIS, A. ; ROBOTTI, C.** *Comput. Appl. Biosci.,* 1994, vol. 10, 3-5
- **PEARSON, W. ; LIPMAN, D.** *Proc. Natl. Acad. Sci. USA,* 1988, vol. 85, 2444-48
- **NIELSEN, P. et al.** *Science,* 1991, vol. 254, 1497-1500
- **KOHLER ; MILSTEIN.** *Nature,* vol. 256, 495-497
- **KOZBOR et al.** *Immunol. Today,* 1983, vol. 4, 72
- **COLE et al.** Monoclonal Antibodies and Cancer Therapy. Alan R. Liss, 1985, 77-96
- Current Protocols in Immunology. John Wiley & Sons, Inc, 1994
- **GALFRE et al.** *Nature,* 1977, vol. 266, 55052
- **R.H. KENNETH.** Monoclonal Antibodies: A New Dimension In Biological Analyses. Plenum Publishing Corp, 1980
- **LERNER.** *Yale J. Biol. Med.,* 1980, vol. 54, 387-402
- **FUCHS et al.** *Bio/Technology,* 1981, vol. 9, 1370-1372
- **HAY et al.** *Hum. Antibod. Hybridomas,* 1992, vol. 3, 81-85, Hum. Antibod. Hybridomas
- **HUSE et al.** *Science,* 1989, vol. 246, 1275-1281
- **GRIFFITHS et al.** *EMBO J.,* 1993, vol. 12, 725-734
- **WEISS, L. A. et al.** Association between Microdeletion and Microduplication at 16p11.2 and Autism. *N Engl J Med,* 2008
- **MILLAR, J. K. et al.** Disruption of two novel genes by a translocation co-segregating with schizophrenia. *Hum Mol Genet,* 2000, vol. 9, 1415-23
- **INOUE, K. ; LUPSKI, J. R.** Molecular mechanisms for genomic disorders. *Annu Rev Genomics Hum Genet,* 2002, vol. 3, 199-242
- **LEE, J. A. ; CARVALHO, C. M. ; LUPSKI, J. R. A.** DNA replication mechanism for generating nonrecurrent rearrangements associated with genomic disorders. *Cell,* 2007, vol. 131, 1235-47
- **NI, X. et al.** Connexin 50 gene on human chromosome 1q21 is associated with schizophrenia in matched case control and family-based studies. *J Med Genet,* 2007, vol. 44, 532-6
- **WALSH, T. et al.** Rare structural variants disrupt multiple genes in neurodevelopmental pathways in schizophrenia. *Science,* 2008, vol. 320, 539-43
- **BRZUSTOWICZ, L. et al.** *Science,* 2000, vol. 288, 678-82
- **GURLING, H. M. et al.** *Am J Hum Genet,* 2001, vol. 68, 661-73

- **HWU, H. G. et al.** *Mol Psychiatry,* 2003, vol. 8, 445-52
- **ZHENG, Y. et al.** *Biochem Biophys Res Commun,* 2006, vol. 342, 1049-57
- **MURTHY, S. K. et al.** *Cytogenet Genome Res,* 2007, vol. 116, 135-40
- **ROGERS, S. J. et al.** The behavioral phenotype in fragile X: symptoms of autism in very young children with fragile X syndrome, idiopathic autism, and other developmental disorders. *J Dev Behav Pediatr,* 2001, vol. 22, 409-17
- **NOWICKI, S. T. et al.** The Prader-Willi phenotype of fragile X syndrome. *J Dev Behav Pediatr,* 2007, vol. 28, 133-8
- **BORGHGRAEF, M. et al.** The female and the fragile X syndrome: data on clinical and psychological findings in 7 fra(X) carriers. *Clin Genet,* 1990, vol. 37, 341-6
- **THOMPSON, N. M. et al.** Neurobehavioral characteristics of CGG amplification status in fragile X females. *Am J Med Genet,* 1994, vol. 54, 378-83
- **HANCOCK, M. L. et al.** Presynaptic type III neuregulin1-ErbB signaling targets {alpha}7 nicotinic acetylcholine receptors to axons. *J Cell Biol,* 2008, vol. 181, 511-21
- **FREEDMAN, R. et al.** Linkage of a neurophysiological deficit in schizophrenia to a chromosome 15 locus. *Proc Natl Acad Sci U S A,* 1997, vol. 94, 587-92
- **SHARP, A. J. et al.** Discovery of previously unidentified genomic disorders from the duplication architecture of the human genome. *Nat Genet,* 2006, vol. 38, 1038-42
- **FERNANDES, C. et al.** Performance deficit of alpha7 nicotinic receptor knockout mice in a delayed matching-to-place task suggests a mild impairment of working/episodic-like memory. *Genes Brain Behav,* 2006, vol. 5, 433-40
- **MURPHY, K. C.** Schizophrenia and velo-cardio-facial syndrome. *Lancet,* 2002, vol. 359, 426-30
- **OUSLEY, O. et al.** A review of neurocognitive and behavioral profiles associated with 22q11 deletion syndrome: implications for clinical evaluation and treatment. *Curr Psychiatry Rep,* 2007, vol. 9, 148-58
- **LEE, J. A. et al.** A DNA replication mechanism for generating nonrecurrent rearrangements associated with genomic disorders. *Cell,* 2007, vol. 131, 1235-47
- **BIECHE, I. et al.** Novel approach to quantitative polymerase chain reaction using real-time detection: application to the detection of gene amplification in breast cancer. *Int J Cancer,* 1998, vol. 78, 661-6
- **COLELLA et al.** QuantiSNP: an Objective Bayes Hidden-Markov Model to detect and accurately map copy number variation using SNP genotyping data. *Nucleic Acids Res,* 2007, vol. 35, 2013-25
- **CONRAD et al.** A high-resolution survey of deletion polymorphism in the human genome. *Nat Genet,* 2006, vol. 38, 75-81
- **HASTIE, T. A. T.** R. Generalized additive models (with discussion). *Statist Sci,* 1986, vol. 1, 297-318
- **SPITZER, RL. et al.** Research diagnostic criteria: rationale and reliability. *Arch Gen Psychiatry,* 1978, vol. 35, 773-82
- **SPITZER RL. et al.** The schedule for affective disorders and schizophrenia, lifetime version. New York State Psychiatric Institute, 1977
- Diagnostic and Statistical Manual of Mental Disorders. Diagnostic and statistical manual of mental disorders. American Psychiatric Press, Inc, 1994
- **ROSA, A. et al.** Further evidence that congenital dermatoglyphic abnormalities are associated with psychosis: a twin study. *Schizophr Bull,* 2002, vol. 28, 697-701
- **TOULOPOULOU, T. et al.** Episodic memory in schizophrenic patients and their relatives. *Schizophr Res,* 2003, vol. 63, 261-71
- **SCAN.** Manual. World Health Organization, 1994
- **FIRST, MB. et al.** Structured Clinical Interview for Axis I DSM-IV Disorders. Biometrics Research, 1994
- **MCGUFFIN, P. et al.** A polydiagnostic application of operational criteria in studies of psychotic illness. Development and reliability of the OPCRIT system. *Arch Gen Psychiatry,* 1991, vol. 48, 764-70
- **ULLMAN et al.** *Human Mutation,* 2007, vol. 28, 674-682
- **MCINNESS et al.** *Proc. Natl. Sci. USA,* 1996, vol. 493 (23), 13060-13065
- **EWALD et al.** *Mol Psychiatry,* 2002, vol. 7 (7), 734-744
- **EKHOLM et al.** *Hum. Mol. Genet.,* 2003, vol. 12 (15), 1907-1915
- **KASSEM et al.** *Am. J. Psychiatry,* 2006, vol. 163 (10), 1099-104
- **JONES et al.** *Am. J. Psychiatry.,* 2007, vol. 164 (2), 248-258
- **EKELUND et al.** *Mol. Psychiatry,* 2004, vol. 9 (11), 1037-1041
- **HENNAH et al.** *Hum. Mol. Genet.,* 2007, vol. 16 (5), 453-462
- **NUMATA S et al.** *Schizophr. Res.,* 2008, vol. 99 (1-3), 367-369
- **BURDICK et al.** *Hum. Mol. Genet.,* 2008
- **ULMANN et al.** *Human Mutation,* 2007, vol. 28, 674-682
- **SHAW ; LUPSKI.** *Hum. Mol. Genet.,* 2004, vol. 13 (1), R57-64
- **TUZUN et al.** *Nat. Genet.,* 2005, vol. 37 (7), 727-732
- **ZHANG et al.** *Cytogen Genome Research,* 2006

- **SHARP et al.** *Nat Genet.,* September 2006, vol. 38 (9), 1038-1042
- **KRIEK et al.** *J. Med. Genet.,* 2004, vol. 41 (4), 249-255
- **FINELLI et al.** *J. Med. Genet.,* 2004, vol. 41 (7), e90
- **ULLMAN.** *Human Mutation,* 2007, vol. 28, 674-682
- **AMMINGER et al.** *Am. J. Psychiatry,* 1999, vol. 156 (4), 525-530
- **KESHAVAN et al.** *Schizophr. Res.,* 2003, vol. 59 (1), 85-92
- **ONER et al.** *Schizophr. Res.,* 2005, vol. 76 (2-3), 293-299
- **WHITAKER-AZMITIA et al.** *Neuropsychopharmacology,* 1995, vol. 12 (3), 269-272
- **MAZER et al.** *Brain Res.,* 1997, vol. 760 (1-2), 68-73
- **MARX et al.** *Biol. Psychiatry,* 2001, vol. 50 (10), 743-749
- **BOURAS et al.** *ActaNeuropathol.,* 2001, vol. 102 (4), 373-379
- **MIDDLETON et al.** *J.Neurosci.,* vol. 22 (7), 2718-2729
- **VAWTER et al.** *Schizophr. Res.,* 2002, vol. 58 (1), 11-20
- **ALTAR et al.** *Biol. Psychiatry,* 2005, vol. 58 (2), 85-96
- **HEDGE ; AND ANTONIO.** *Neuroscience,* 2002, vol. 3, 854-861
- **COLLINS CA ; DI ANTONIO A.** *Current Opinion Neurobiology,* 2007, vol. 17, 35-42
- **KWON et al.** *Mol. Cell Biol.,* 2000, vol. 20 (11), 4135-4148
- **BALOGH et al.** *Learn Mem.,* 2000, vol. 7 (5), 279-286
- **KAMIYA et al.** *Hum. Mol. Genet.,* 2006, vol. 15 (22), 3313-3323
- **ST CLAIR et al.** *Lancet,* 1990, vol. 336 (8706), 13-16
- **BLACKWOOD et al.** *Am. J. Hum. Genet.,* 2001, vol. 69 (2), 428-433
- **SACHS et al.** *Am. J. Hum. Genet.,* 2005, vol. 69 (2), 428-433
- **KILPINEN et al.** *Mol. Psychiatry,* 2008, vol. 13 (2), 187-196
- **SPITZER et al.** *Arch. Gen. Psychiatry,* 1978, vol. 35, 773-782
- **SPITZER.** *New York State Psychiatric Institute, New York,* 1977
- **MCGUFFIN et al.** *Arch. Gen. Psychiatry,* 1991, 764-770
- **FIRST et al.** *Biometrics Research, New York,* 1994
- Schedules for Clinical Assessment in Neuropsychiatry (SCAN) Manual. 1994
- **COLELLA ; YAU et al.** *Nucleic Acids Research,* 2007